# EUROPÄISCHE PATENTSCHRIFT

(11) **EP 2 227 463 B1**
(45) Veröffentlichungstag und Bekanntmachung des Hinweises auf die Patenterteilung: **17.08.2016**
(21) Anmeldenummer: 08867378.5
(22) Anmeldetag: 23.12.2008
(51) Int. Cl.: C07D 401/14, A61K 31/4439, A61K 31/496, A61P 25/00

(54) **SUBSTITUIERTE OXINDOL-DERIVATE UND IHRE VERWENDUNG ZUR BEHANDLUNG VON VASOPRESSIN-ABHÄNGIGEN ERKRANKUNGEN**
SUBSTITUTED OXINDOLE-DERIVATIVES AND THE USE THEREOF FOR THE TREATMENT OF VASOPRESSIN-DEPENDENT ILLNESSES
DÉRIVÉS SUBSTITUÉS D'OXINDOLE ET LEUR UTILISATION POUR LE TRAITEMENT DE MALADIES DÉPENDANT DE LA VASOPRESSINE

(30) Priorität: 27.12.2007 US 9276
(43) Veröffentlichungstag der Anmeldung: 15.09.2010
(73) Patentinhaber: AbbVie Deutschland GmbH & Co KG, 65205 Wiesbaden (DE)
(72) Erfinder: BRAJE, Wilfried, 67061 Ludwigshafen (DE); NETZ, Astrid, 67061 Ludwigshafen (DE); OOST, Thorsten, 88400 Biberach an der Riss (DE); WERNET, Wolfgang, "deceased" (DE); UNGER, Liliane, 67061 Ludwigshafen (DE); HORNBERGER, Wilfried, 67061 Ludwigshafen (DE); LUBISCH, Wilfried, 69118 Heidelberg (DE)
(74) Vertreter: Reitstötter Kinzebach
(86) Internationale Anmeldenummer: PCT/EP2008/068254
(87) Internationale Veröffentlichungsnummer: WO 2009/083559

(56) Entgegenhaltungen:
- WO-A-03/008407
- WO-A-2005/030755
- WO-A-2007/063123

## Beschreibung

Die vorliegende Erfindung betrifft neue substituierte Oxindol-Derivate, diese enthaltende pharmazeutische Mittel und ihre Verwendung zur Behandlung von Vasopressin-abhängigen Erkrankungen.

Vasopressin ist ein endogenes Hormon, das verschiedenste Wirkungen an Organen und Gewebe ausübt. Bei verschiedenen Krankheitszuständen, wie zum Beispiel Herzinsuffizienz und Bluthochdruck, vermutet man, dass das Vasopressin-System eine Rolle spielt. Derzeit sind drei Rezeptoren (V1a, V1b bzw. V3 und V2) bekannt, über die Vasopressin seine zahlreichen Wirkungen vermittelt. Daher werden Antagonisten dieser Rezeptoren als mögliche neue therapeutische Ansätze zur Behandlung von Krankheiten untersucht (M. Thibonnier, Exp.Opin. Invest. Drugs 1998, 7(5), 729-740).

Vorliegend werden neue substituierte Oxindole beschrieben, die in der 1-Position eine Phenylsulfonyl-Gruppe tragen. 1-Phenylsulfonyl-1,3-dihydro-2H-indol-2-one wurden bereits als Liganden der Vasopressin-Rezeptoren beschrieben. Auch die WO 93/15051, WO 95/18105, WO 98/25901, WO 01/55130, WO 01/55134, WO 01/164668 und WO 01/98295 beschreiben Derivate, die in der 1-Position des Oxindolgerüsts Arylsulfonlygruppen tragen. Diese Verbindungen unterscheiden sich von den erfindungsgemäßen Verbindungen wesentlich durch die Substituenten in der 3-Position.

So werden in der WO 93/15051 und WO 98/25901 1-Phenylsulfonyl-1,3-dihydro-2H-indol-2-one als Liganden der Vasopressinrezeptoren beschrieben, bei denen das Oxindol-Gerüst in der 3-Position durch zwei Alkylreste substituiert ist, die gemeinsam auch einen Cycloalkylrest (Spiroverknüpfung) bilden können. Als Alternative kann der Spiroring Heteroatome, wie Sauerstoff und Stickstoff (wahlweise mit Substituenten), enthalten.

Die WO 95/18105 beschreibt 1-Phenylsulfonyl-1,3-dihydro-2*H*-indol-2-one als Liganden der Vasopressinrezeptoren, die ein Stickstoffatom in der 3-Position besitzen. Zusätzlich sind in der 3-Position Reste gebunden, die unter gegebenenfalls substituierten Alkyl-, Cycloalkyl-, Phenyl- oder Benzylresten ausgewählt sind.

In der WO 03/008407 werden 1-Phenylsulfonyloxindole beschrieben, in denen in der 3-Position Pyridylpiperazine über eine Harnstoff-, Carbamat- oder 2-Oxoethyl-Gruppe am Oxindol gebunden sind.

Neben der Bindungsaffinität zum Vasopressin V1 b Rezeptor können weitere Eigenschaften vorteilhaft für die Behandlung und/oder Prophylaxe von Vasopressin-abhängigen Erkrankungen sein, wie beispielsweise:
1.) eine Selektivität zum Vasopressin V1b Rezeptor im Vergleich zum Vasopressin V1a Rezeptor, d.h. der Quotient aus der Bindungsaffinität zum V1a Rezeptor (Ki(V1a) (bestimmt in der Einheit "Nanomolar (nM)") und der Bindungsaffinität zum V1 b Rezeptor (Ki(V1 b)) (bestimmt in der Einheit "Nanomolar (nM)"). Je größer der Quotient Ki(V1a)/Ki(V1 b) ist, desto größer ist die V1 b-Selektivität;
2.) eine Selektivität zum Vasopressin V1 b Rezeptor im Vergleich zum Vasopressin V2 Rezeptor, d.h. der Quotient aus der Bindungsaffinität zum V2 Rezeptor (Ki(V2) (bestimmt in der Einheit "Nanomolar (nM)") und der Bindungsaffinität zum V1 b Rezeptor (Ki(V1 b)) (bestimmt in der Einheit "Nanomolar (nM)"). Je größer der Quotient Ki(V2)/Ki(V1 b) ist, desto größer ist die V1b-Selektivität;
3.) eine Selektivität zum Vasopressin V1b Rezeptor im Vergleich zum Oxytozin OT Rezeptor, d.h. der Quotient aus der Bindungsaffinität zum OT Rezeptor (Ki(OT) (bestimmt in der Einheit "Nanomolar (nM)") und der Bindungsaffinität zum V1 b Rezeptor (Ki(V1 b)) (bestimmt in der Einheit "Nanomolar (nM)"). Je größer der Quotient Ki(OT)/Ki(V1b) ist, desto größer ist die V1b-Selektivität.
4.) die metabolische Stabilität, beispielsweise bestimmt anhand der in-vitro ermittelten Halbwertszeiten in Lebermikrosomen von verschiedenen Spezies (z.B. Ratte oder Mensch);
5.) keine oder nur geringe Inhibierung von Cytochrom P450 (CYP) Enzymen: Cytochrom P450 (CYP) ist die Bezeichnung für eine Superfamilie von Hämproteinen mit enzymatischer Aktivität (Oxidase). Besondere Bedeutung besitzen sie auch für den Abbau (Metabolismus) von Fremdstoffen wie Pharmaka oder Xenobiotika in Säugetierorganismen. Die wichtigsten Vertreter der Typen und Untertypen von CYP im menschlichen Organismus sind: CYP 1A2, CYP 2C9, CYP 2D6 und CYP 3A4. Bei gleichzeitiger Anwendung von CYP 3A4-Hemmstoffen (z.B. Grapefruitsaft, Cimetidin, Erythromycin) und Arzneistoffen, die über dieses Enzymsystem abgebaut werden und somit um die gleiche Bindungsstelle am Enzym konkurrieren, kann deren Abbau verlangsamt und dadurch Wirkungen und Nebenwirkungen des verabreichten Arzneistoffs unerwünscht verstärkt werden;
6.) eine geeignete Wasserlöslichkeit (in mg/ml);
7.) eine geeignete Pharmakokinetik (zeitlicher Verlauf der Konzentration der erfindungsgemässen Verbindung im Plasma bzw. in Geweben, zum Beispiel Gehirn). Die Pharmakokinetik kann durch die folgenden Parameter beschrieben werden: Halbwertszeit (in h), Verteilungsvolumen (in I·kg⁻¹), Plasma-Clearance (in I·h⁻¹·kg⁻¹), AUC ("area under the curve", Fläche unter der Konzentrations-Zeit-Kurve, in ng·h·I⁻¹), orale Bioverfügbarkeit (das Dosis-normalisierte Verhältnis von AUC nach oraler Gabe und AUC nach intravenöser Gabe), das sog. Brain-Plasma-Ratio (das Verhältnis von AUC im Hirngewebe und AUC im Plasma);
8.) keine oder nur geringe Blockade des hERG-Kanals: Verbindungen, die den hERG-Kanal blockieren, können eine Verlängerung des QT-Intervalls verursachen und dadurch zu ernsten Herz-Rhythmus-Störungen führen (zum Beispiel sogenannte "torsade de pointes"). Mittels eines in der Literatur beschriebenen Verdrängungsassays mit radioaktiv markiertem Dofetilid (G. J. Diaz et al., Journal of Pharmacological and Toxicological Methods, 50 (2004), 187-199) kann das Potential von Verbindungen, den hERG-Kanal zu blockieren, bestimmt werden. Je geringer der IC50 in diesem "dofetilide assay", desto wahrscheinlicher ist eine potente hERG-Blockade. Darüber hinaus kann die Blockade des hERG-Kanals durch elektrophysiologische Experimente an Zellen, die mit dem hERG-Kanal transfiziert wurden, durch sogenanntes "whole-cell patch clamping" gemessen werden (G. J. Diaz et al., Journal of Pharmacological and Toxicological Methods, 50 (2004), 187-199).

Aufgabe der vorliegenden Erfindung war es, Verbindungen zur Behandlung oder Prophylaxe von verschiedenen Vasopressin-abhängigen Krankheiten zur Verfügung zu stellen. Die Verbindungen sollten eine hohe Aktivität und Selektivität aufweisen, vor allem eine hohe Affinität und Selektivität gegebenüber dem Vasopressin V1 b Rezeptor. Zusätzlich sollte die erfindungsgemäße Substanz einen oder mehrere der vorstehend genannten Vorteile 1.) bis 8.) besitzen.

Die Aufgabe wird durch Verbindungen der Formel I gelöst 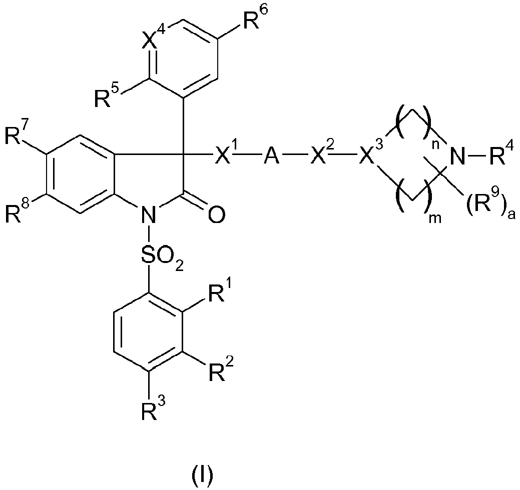 worin
- X¹: für -O-, -O-CH₂-, -O-C(=O)-, -NR¹¹-, -NR¹¹-CH₂- oder -NR¹¹-C(=O)- steht;
- X²: für eine Einfachbindung, CO oder CH₂ steht;
- X³: für N oder CH steht;
- X⁴: für N oder CH steht;
- A: für Phenylen oder 6-gliedriges Hetarylen mit 1 oder 2 Stickstoffatomen als Ringgliedern steht, wobei Phenylen oder Hetarylen durch 1 oder 2 Reste R¹⁰ substituiert sein kann;
- R¹ und R³: unabhängig voneinander für Wasserstoff, C₁-C₃-Alkyl, C₁-C₃-Fluoralkyl, C₁-C₃-Alkoxy, C₁-C₃-Fluoralkoxy, Halogen oder CN stehen;
- R²: für Wasserstoff oder Methoxy steht; wobei wenigstens einer der Reste R¹, R² und R³ für Wasserstoff steht;
- R⁴: für Wasserstoff oder C₁-C₄-Alkyl steht;
- R⁵: für Ethoxy, fluoriertes Ethoxy, Fluormethoxy, Difluormethoxy, Trifluormethoxy oder Isopropoxy steht;
- R⁶: für Wasserstoff oder Methyl steht;
- R⁷: für Wasserstoff, I, Br, Cl, F oder CN steht;
- R⁸: für Wasserstoff, I, Br, Cl, F oder CN steht;
- R⁹: für C₁-C₃-Alkyl oder C₁-C₃-Fluoralkyl steht;
- R¹⁰: für C₁-C₃-Alkyl, C₁-C₃-Fluoralkyl, C₁-C₃-Alkoxy oder C₁-C₃-Fluoralkoxy steht;
- R¹¹: für Wasserstoff, C₁-C₄-Alkyl, C₁-C₄-Fluoralkyl, C₁-C₄-Alkoxy oder C₁-C₄-Fluoralkoxy steht;
- a: für 0, 1 oder 2 steht; und
- m und n: unabhängig voneinander für 1 oder 2 stehen;

sowie deren pharmazeutisch verträglichen Salze.

Dementsprechend betrifft die vorliegende Erfindung Verbindungen der Formel I (im Folgenden auch "Verbindungen I") sowie die pharmazeutisch verträgliche Salze der Verbindungen I.

Die pharmazeutisch verträglichen Salze von Verbindungen der Formel I, die auch als physiologisch verträgliche Salze bezeichnet werden, sind in der Regel durch Umsetzung der freien Base der erfindungsgemäßen Verbindungen I (d.h. der Verbindungen I gemäß Strukturformel I) mit geeigneten Säuren erhältlich. Geeignete Säuren sind beispielsweise aufgeführt in "Fortschritte der Arzneimittelforschung", 1966, Birkhäuser Verlag, Bd.10, S.224-285. Darunterfallen beispielsweise Salzsäure, Zitronensäure, Weinsäure, Milchsäure, Phosphorsäure, Methansulfonsäure, Essigsäure, Ameisensäure, Maleinsäure und Fumarsäure.

C₁-C₃-Alkyl steht im Rahmen der vorliegenden Erfindung für einen linearen oder verzweigten Alkylrest mit 1 bis 3 Kohlenstoffatomen, wie Methyl, Ethyl, n-Propyl oder Isopropyl.

C₁-C₄-Alkyl steht im Rahmen der vorliegenden Erfindung für einen linearen oder verzweigten Alkylrest mit 1 bis 4 Kohlenstoffatomen, wie Methyl, Ethyl, n-Propyl, Isopropyl, n-Butyl, sek-Butyl, Isobutyl oder tert-Butyl.

C₁-C₃-Fluoralkyl steht im Rahmen der vorliegenden Erfindung für einen linearen oder verzweigten Alkylrest mit 1 bis 3 Kohlenstoffatomen, wie oben definiert, in welchem wenigstens ein Wasserstoffatom, z.B. 1, 2, 3, 4 oder 5 Wasserstoffatome, durch Fluoratome ersetzt sind. Beispiele hierfür sind Fluormethyl, Difluormethyl, Trifluormethyl, 1- und 2-Fluorethyl, 1,1-, 1,2- und 2,2-Difluorethyl, 1,1,2-, 1,2,2 und 2,2,2-Trifluorethyl, 1,1,2,2-Tetrafluorethyl, 1,2,2,2-Tetrafluorethyl, Pentafluorethyl, 1-, 2- und 3-Fluorprop-1-yl, 1,1-, 1,2-, 1,3-, 2,2-, 2,3- und 3,3-Difluorprop-1-yl, 1,1,2-, 1,2,2-, 1,1,3-, 2,2,3-, 1,2,3- und 3,3,3-Trifluorprop-1-yl, 1- und 2-Fluorprop-2-yl, 1,1- und 1,3-Difluorprop-2-yl, 1,1,1-Trifluorprop-2-yl und dergleichen.

C₁-C₄-Fluoralkyl steht im Rahmen der vorliegenden Erfindung für einen linearen oder verzweigten Alkylrest mit 1 bis 4 Kohlenstoffatomen, wie oben definiert, in welchem wenigstens ein Wasserstoffatom, z.B. 1, 2, 3, 4 oder 5 Wasserstoffatome, durch Fluoratome ersetzt sind. Beispiele hierfür sind neben den zuvor bei C₁-C₃-Fluoralkyl genannten Resten 1-, 2-, 3- und 4-Fluor-n-butyl, 1,1-, 2,2-, 3,3- und 4,4-Difluor-n-butyl, 4,4,4-Trifluor-n-butyl, 3-Fluor-2-(fluormethyl)-1-propyl, 3,3-Difluor-2-(difluormethyl)-1-propyl, 3,3,3-Trifluor-2-(trifluormethyl)-1-propyl und dergleichen.

C₁-C₃-Alkoxy steht im Rahmen der vorliegenden Erfindung für einen über ein Sauerstoffatom gebundenen linearen oder verzweigten Alkylrest mit 1 bis 3 Kohlenstoffatomen. Beispiele sind Methoxy, Ethoxy, n-Propoxy und Isopropoxy.

C₁-C₄-Alkoxy steht im Rahmen der vorliegenden Erfindung für einen über ein Sauerstoffatom gebundenen linearen oder verzweigten Alkylrest mit 1 bis 4 Kohlenstoffatomen. Beispiele sind neben den zuvor bei C₁-C₃-Alkoxy genannten Resten n-Butoxy, sec-Butoxy, Isobutoxy und tert-Butoxy.

C₁-C₃-Fluoralkoxy steht im Rahmen der vorliegenden Erfindung für einen über ein Sauerstoffatom gebundenen linearen oder verzweigten Alkylrest mit 1 bis 3 Kohlenstoffatomen, wie oben definiert, in welchem wenigstens ein Wasserstoffatom, z.B. 1, 2, 3, 4 oder 5 Wasserstoffatome, durch Fluoratome ersetzt sind. Beispiele hierfür sind Fluormethoxy, Difluormethoxy, Trifluormethoxy, 1- und 2-Fluorethoxy, 1,1-, 1,2- und 2,2-Difluorethoxy, 1,1,2-, 1,2,2 und 2,2,2-Trifluorethoxy, 1,1,2,2-Tetrafluorethoxy, 1,2,2,2-Tetrafluorethoxy, Pentafluorethoxy, 1-, 2- und 3-Fluorprop-1-oxy, 1,1-, 1,2-, 1,3-, 2,2-, 2,3- und 3,3-Difluorprop-1-oxy, 1,1,2-, 1,2,2-, 1,1,3-, 2,2,3-, 1,2,3- und 3,3,3-Trifluorprop-1-oxy, 1- und 2-Fluorprop-2-oxy, 1,1- und 1,3-Difluorprop-2-oxy, 1,1,1-Trifluorprop-2-oxy und dergleichen.

C₁-C₄-Fluoralkoxy steht im Rahmen der vorliegenden Erfindung für einen über ein Sauerstoffatom gebundenen linearen oder verzweigten Alkylrest mit 1 bis 4 Kohlenstoffatomen, wie oben definiert, in welchem wenigstens ein Wasserstoffatom, z.B. 1, 2, 3, 4 oder 5 Wasserstoffatome, durch Fluoratome ersetzt sind. Beispiele hierfür sind neben den zuvor bei C₁-C₃-Fluoralkoxy genannten Resten 1-, 2-, 3- und 4-Fluor-n-butoxy, 1,1-, 2,2-, 3,3-und 4,4-Difluor-n-butoxy, 4,4,4-Trifluor-n-butoxy, 3-Fluor-2-(fluormethyl)-1-propoxy, 3,3-Difluor-2-(difluormethyl)-1-propoxy, 3,3,3-Trifluor-2-(trifluormethyl)-1-propoxy und dergleichen.

Fluoriertes Ethoxy steht im Rahmen der vorliegenden Erfindung für Ethoxy, in welchem 1, 2, 3, 4 oder 5 der Wasserstoffatome durch Fluoratome substituiert sind. Beispiele sind 1-Fluorethoxy, 2-Fluorethoxy, 1,1-Difluorethoxy, 1,2-Difluorethoxy, 2,2-Difluorethoxy, 1,1,2-Trifluorethoxy, 1,2,2-Trifluorethoxy, 2,2,2-Trifluorethoxy, 1,1,2,2-Tetrafluorethoxy und 1,1,2,2,2-Pentafluorethoxy.

Halogen steht im Rahmen der vorliegenden Erfindung für Fluor, Chlor, Brom oder Iod. Phenylen steht für einen zweiwertigen Phenylrest, wie 1,2-Phenylen, 1,3-Phenylen und 1,4-Phenylen.

6-gliedriges Hetarylen mit ein oder 2 Stickstoffatomen als Ringgliedern steht für einen zweiwertigen Pyridylrest (Pyridylen), wie 2,3-Pyridylen, 2,4-Pyridylen, 2,5-Pyridylen, 2,6-Pyridylen, 3,4-Pyridylen, 3,5-Pyridylen, 3,6-Pyridylen, 4,5-Pyridylen, 4,6-Pyridylen oder 5,6-Pyridylen; für einen zweiwertigen Pyrimidylrest (Pyrimidylen), wie 2,3-Pyrimidylen, 2,4-Pyrimidylen, 4,2-Pyrimidylen, 4,5-Pyrimidylen, 4,6-Pyrimidylen oder 5,2-Pyrimidylen; für einen zweiwertigen Pyrazinrest (Pyrazinylen), wie 2,3-Pyrazinylen, 2,5-Pyrazinylen oder 2,6-Pyrazinylen; oder für einen zweiwertigen Pyridazinrest, (Pyridazinylen), wie 3,4-Pyridazinylen, 3,5-Pyridazinylen, 3,6-Pyridazinylen oder 4,5-Pyridazinylen.

Die erfindungsgemäßen Verbindungen der Formel I und ihre pharmakologisch verträglichen Salze können auch in Form von Solvaten oder Hydraten vorliegen. Unter Solvaten versteht man im Rahmen der vorliegenden Erfindung kristalline Formen der Verbindungen I bzw. ihrer pharmazeutisch verträglichen Salze, die im Kristallgitter Lösungsmittelmoleküle eingebaut enthalten. Vorzugsweise sind die Lösungsmittelmoleküle in stöchiometrischen Verhältnissen eingebaut. Hydrate sind eine Spezialform der Solvate; das Lösungsmittel ist hier Wasser.

Die nachstehend gemachten Angaben zu geeigneten und bevorzugten Merkmalen der Erfindung, insbesondere zu den Variablen R¹, R², R³, R⁴, R⁵, R⁶, R⁷, R⁸, R⁹, R¹⁰, R¹¹, X¹, X², X³, X⁴, A, a, m und n in der Verbindung I, zu den Variablen R¹, R², R³, R⁵, R⁶, R⁷, R⁸, R¹², X¹, X⁴ und A in der Verbindung XVIII, aber auch zu den Merkmalen des erfindungsgemäßen Verfahrens und der erfindungsgemäßen Verwendung gelten sowohl für sich allein genommen als auch vorzugsweise in jeder möglichen Kombination miteinander.

Die Verbindungen I werden bevorzugt in Form der freien Base (d.h. gemäß der Strukturformel I) oder in Form ihrer Säureadditionssalze bereitgestellt.

In Verbindungen I steht wenigstens einer der Reste R¹, R², R³ für Wasserstoff. Vorzugsweise steht wenigstens R² für Wasserstoff. Besonders bevorzugt sind dabei R¹ oder R³ oder R¹ und R³ von Wasserstoff verschieden.

In der Definition von R¹ und R³ steht C₁-C₃-Alkoxy vorzugsweise für Ethoxy oder Methoxy und speziell für Methoxy. C₁-C₃-Fluoralkoxy steht vorzugsweise für C₁-C₂-Fluoralkoxy, d.h. für Fluormethoxy, Difluormethoxy, Trifluormethoxy, 1- und 2-Fluorethoxy, 1,1-, 1,2- und 2,2-Difluorethoxy, 1,1,2-, 1,2,2 und 2,2,2-Trifluorethoxy, 1,1,2,2-Tetrafluorethoxy, 1,2,2,2-Tetrafluorethoxy oder Pentafluorethoxy, bevorzugt für Fluormethoxy, Difluormethoxy, Trifluormethoxy, 2,2-Difluorethoxy oder 2,2,2-Trifluorethoxy, und speziell für Trifluormethoxy, 2,2-Difluorethoxy oder 2,2,2-Trifluorethoxy.

In einer bevorzugten Ausführungsform stehen R¹ und R³ unabhängig voneinander für Wasserstoff, CN, C₁-C₃-Alkoxy oder C₁-C₃-Fluoralkoxy. Dabei steht in der Definition der Reste R¹ und R³ C₁-C₃-Alkoxy vorzugsweise für Ethoxy oder Methoxy und speziell für Methoxy. C₁-C₃-Fluoralkoxy steht vorzugsweise für C₁-C₂-Fluoralkoxy, d.h. für Fluormethoxy, Difluormethoxy, Trifluormethoxy, 1- und 2-Fluorethoxy, 1,1-, 1,2- und 2,2-Difluorethoxy, 1,1,2-, 1,2,2 und 2,2,2-Trifluorethoxy, 1,1,2,2-Tetrafluorethoxy, 1,2,2,2-Tetrafluorethoxy oder Pentafluorethoxy, bevorzugt für Fluormethoxy, Difluormethoxy, Trifluormethoxy, 2,2-Difluorethoxy oder 2,2,2-Trifluorethoxy, und speziell für Trifluormethoxy, 2,2-Difluorethoxy oder 2,2,2-Trifluorethoxy.

In einer besonders bevorzugten Ausführungsform steht R¹ für Wasserstoff, Methoxy, Ethoxy, Fluormethoxy, Difluormethoxy oder Trifluormethoxy, stärker bevorzugt für Wasserstoff, Methoxy oder Trifluormethoxy und insbesondere für Wasserstoff oder Methoxy.

In einer besonders bevorzugten Ausführungsform steht R³ für Wasserstoff, C₁-C₃-Alkoxy oder C₁-C₃-Fluoralkoxy, stärker bevorzugt für Wasserstoff, Methoxy, Ethoxy, Fluormethoxy, Difluormethoxy oder Trifluormethoxy, noch stärker bevorzugt für Wasserstoff, Methoxy oder Trifluormethoxy, insbesondere für Wasserstoff oder Methoxy und speziell für Methoxy.

In einer besonders bevorzugten Ausführungsform steht wenigstens einer der Reste R¹ und R³ für Methoxy.

In einer bevorzugten Ausführungsform steht R² für Wasserstoff. Alternativ steht R² vorzugsweise für Methoxy und R¹ und R³ stehen gleichzeitig für Wasserstoff. Besonders bevorzugt steht R² jedoch für Wasserstoff.

In einer speziellen Ausführungsform stehen R¹ und R³ für Methoxy und R² steht für Wasserstoff; oder einer der Reste R¹ und R³ steht für Methoxy, der andere steht für Wasserstoff und R² steht für Wasserstoff.

In einer bevorzugten Ausführungsform steht R⁴ für Wasserstoff, Methyl, Ethyl, n-Propyl oder Isopropyl, besonders bevorzugt für Wasserstoff, Methyl oder Ethyl, insbesondere für Wasserstoff oder Methyl.

In einer bevorzugten Ausführungsform steht R⁵ für Ethoxy oder fluoriertes Ethoxy, besonders bevorzugt für Ethoxy, 2,2-Difluorethoxy oder 2,2,2-Trifluorethoxy und insbesondere für Ethoxy.

In einer bevorzugten Ausführungsform steht R⁶ für H.

In einer besonders bevorzugten Ausführungsform steht R⁵ für Ethoxy und R⁶ steht für H. Dabei steht X⁴ für N oder CH und vorzugsweise für N.

In einer alternativ besonders bevorzugten Ausführungsform steht R⁵ für Ethoxy und R⁶ steht für Methyl. Dabei steht X⁴ vorzugsweise für N.

In einer alternativ besonders bevorzugten Ausführungsform steht R⁵ für Isopropoxy und R⁶ steht für H. Dabei steht X⁴ vorzugsweise für N.

In einer alternativ besonders bevorzugten Ausführungsform steht R⁵ für fluoriertes Ethoxy, vorzugsweise für 2,2-Difluorethoxy oder 2,2,2-Trifluorethoxy und besonders bevorzugt für 2,2-Difluorethoxy, und R⁶ steht für H. Gleichzeitig steht X⁴ dabei vorzugsweise für CH.

Besonders bevorzugt steht X⁴ für N.

Insbesondere steht R⁵ für Ethoxy und R⁶ steht für H. Dabei steht X⁴ für N oder CH und vorzugsweise für N.

In einer bevorzugten Ausführungsform stehen R⁷ und R⁸ nicht gleichzeitig für CN.

In einer bevorzugten Ausführungsform stehen R⁷ und R⁸ nicht gleichzeitig für H.

Vorzugsweise steht R⁷ für F, Cl, I oder CN, besonders bevorzugt für I, Cl oder CN und insbesondere für CN.

Vorzugsweise steht R⁸ für H oder F.

Insbesondere steht R⁷ für CN und R⁸ steht gleichzeitig für H oder F, oder R⁷ steht für I und R⁸ steht gleichzeitig für H oder F, speziell für H, oder R⁷ steht für Cl und R⁸ steht gleichzeitig für H oder F, speziell für H.

In einer bevorzugten Ausführungsform steht R⁹ für Methyl oder Ethyl.
a steht vorzugsweise für 0 oder 1 und insbesondere für 0.

In einer bevorzugten Ausführungsform steht R¹⁰ für Methyl oder Methoxy.

In einer bevorzugten Ausführungsform steht R¹¹ für Wasserstoff, C₁-C₄-Alkyl oder C₁-C₄-Alkoxy, besonders bevorzugt für Wasserstoff, Methyl oder Methoxy und insbesondere für Wasserstoff.

In einer bevorzugten Ausführungsform steht X¹ für -O-CH₂-, -O-C(=O)-, -NR¹¹-, -NR¹¹-CH₂- oder -NR¹¹-C(=O)-. Besonders bevorzugt steht X¹ für -O-C(=O)-, -NR¹¹-, -NR¹¹-CH₂- oder -NR¹¹-C(=O)-. Stärker bevorzugt steht X¹ für -NR¹¹- oder -NR¹¹-CH₂- und insbesondere für -NR¹¹-CH₂-. Bevorzugt hat dabei in diesen bevorzugten Bedeutungen von X¹ R¹¹ eine der oben angegebenen bevorzugten Bedeutungen und steht insbesondere für H.

In einer bevorzugten Ausführungsform steht X² für eine Einfachbindung.

In einer bevorzugten Ausführungsform steht X³ für N. Dabei ist es besonders bevorzugt, wenn m und n beide für 2 stehen. Dementsprechend steht der Ring, der X³ als Ringglied enthält, besonders bevorzugt für einen Piperazin-1-yl-Ring.

In einer alternativ bevorzugten Ausführungsform steht X³ für CH. Dabei ist es besonders bevorzugt, wenn m und n beide für 2 stehen. Dementsprechend steht der Ring, der X³ als Ringglied enthält, besonders bevorzugt für einen Piperidin-4-yl-Ring.

In einer alternativ bevorzugten Ausführungsform steht X³ für CH. Dabei ist es besonders bevorzugt, wenn m und n beide für 1 stehen. Dementsprechend steht der Ring, der X³ als Ringglied enthält, besonders bevorzugt für einen Azetidin-3-yl-Ring.

In einer alternativ bevorzugten Ausführungsform steht X³ für CH. Dabei ist es besonders bevorzugt, wenn m für 1 steht und n für 2 steht. Dementsprechend steht der Ring, der X³ als Ringglied enthält, besonders bevorzugt für einen Pyrrolidin-3-yl-Ring.

Besonders bevorzugt steht jedoch X³ für N und m und n stehen beide für 2 oder X³ für CH und m und n stehen beide für 1. Insbesondere steht X³ für N und m und n stehen beide für 2.

In einer bevorzugten Ausführungsform steht A für Phenylen, z.B. für 1,2-, 1,3- oder 1,4-Phenylen. Besonders bevorzugt steht A dabei für 1,3- oder 1,4-Phenylen und insbesondere für 1,4-Phenylen.

In einer alternativ bevorzugten Ausführungsform steht A für Pyridylen oder Pyrimidylen und besonders bevorzugt für Pyridylen. Besonders bevorzugt steht A in diesem Fall für 3,5- oder 3,6-Pyridylen und insbesondere für 3,6-Pyridylen.

Besonders bevorzugt steht A jedoch für Phenylen, z.B. für 1,2-, 1,3- oder 1,4-Phenylen, stärker bevorzugt für 1,3- oder 1,4-Phenylen und insbesondere für 1,4-Phenylen.

A ist vorzugsweise nicht durch R¹⁰ substituiert.

Ein bevorzugter Gegenstand der Erfindung sind Verbindungen der Formel I, worin
- R¹: für H oder Methoxy steht;
- R²: für H steht;
- R³: für Methoxy steht;
- R⁴: für H, Methyl oder Ethyl steht;
- R⁵: für Ethoxy steht;
- R⁶: für H steht;
- R⁷: für CN steht;

- R⁸: für H oder F steht;
- X¹: für -NH- oder -NHCH₂- steht;
- X²: für eine Einfachbindung steht;
- X³: für N steht;
- X⁴: für N steht;
- A: für 1,4-Phenylen steht;
- a: für 0 steht; und
- m und n: für 2 stehen;
sowie die pharmazeutisch verträglichen Salze davon.

Ein besonders bevorzugter Gegenstand der Erfindung sind dabei Verbindungen der Formel I, worin
- R¹: für H oder Methoxy steht;
- R²: für H steht;
- R³: für Methoxy steht;
- R⁴: für H oder Methyl steht;
- R⁵: für Ethoxy steht;
- R⁶: für H steht;
- R⁷: für CN steht;
- R⁸: für H oder F steht;
- X¹: für -NHCH₂- steht;
- X²: für eine Einfachbindung steht;
- X³: für N steht;
- X⁴: für N steht;
- A: für 1,4-Phenylen steht;
- a: für 0 steht; und
- m und n: für 2 stehen;
sowie die pharmazeutisch verträglichen Salze davon.

Ein bevorzugter Gegenstand der Erfindung sind Verbindungen der Formel I, worin
- R¹: für H oder Methoxy steht;
- R²: für H steht;
- R³: für Methoxy steht;
- R⁴: für H, Methyl oder Ethyl steht;
- R⁵: für Ethoxy steht;

- R⁶: für H steht;
- R⁷: für I steht;
- R⁸: für H steht;
- X¹: für -NH- oder -NHCH₂- steht;
- X²: für eine Einfachbindung steht;
- X³: für N steht;
- X⁴: für N steht;
- A: für 1,4-Phenylen steht;
- a: für 0 steht; und
- m und n: für 2 stehen;
sowie die pharmazeutisch verträglichen Salze davon.

Ein besonders bevorzugter Gegenstand der Erfindung sind dabei Verbindungen der Formel I, worin
- R¹: für H oder Methoxy steht;
- R²: für H steht;
- R³: für Methoxy steht;
- R⁴: für H oder Methyl steht;
- R⁵: für Ethoxy steht;
- R⁶: für H steht;
- R⁷: für I steht;
- R⁸: für H steht;
- X¹: für -NHCH₂- steht;
- X²: für eine Einfachbindung steht;
- X³: für N steht;
- X⁴: für N steht;
- A: für 1,4-Phenylen steht;
- a: für 0 steht; und
- m und n: für 2 stehen;
sowie die pharmazeutisch verträglichen Salze davon.

Beispiele für bevorzugte Ausführungsformen der vorliegenden Erfindung sind Verbindungen der Formeln I.1 bis I.56 sowie die pharmazeutisch verträglichen Salze davon, worin die Reste X¹, R¹, R², R³, R⁴, R⁷ und R⁸ die oben angegebenen allgemeinen oder insbesondere bevorzugten Bedeutungen haben. Besonders bevorzugt haben die Reste R¹, R², R³, R⁴, R⁷ und R⁸ die in Tabelle A angegebenen Bedeutungen.

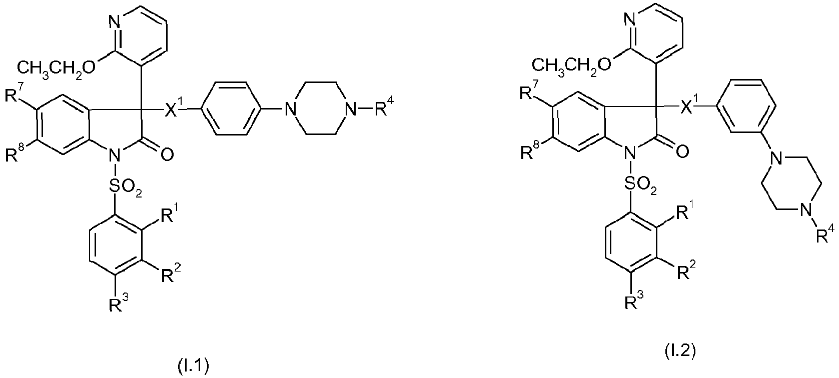

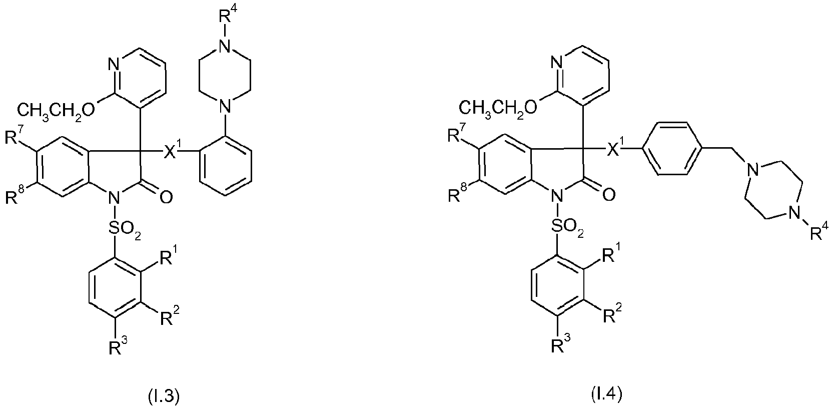

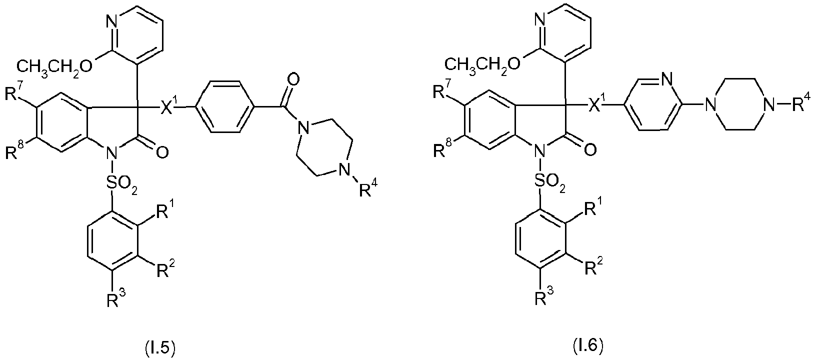

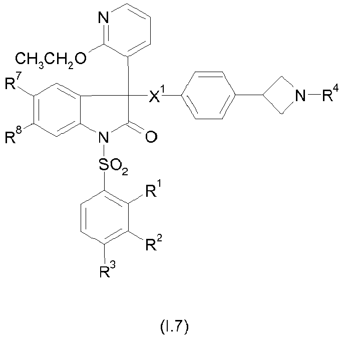

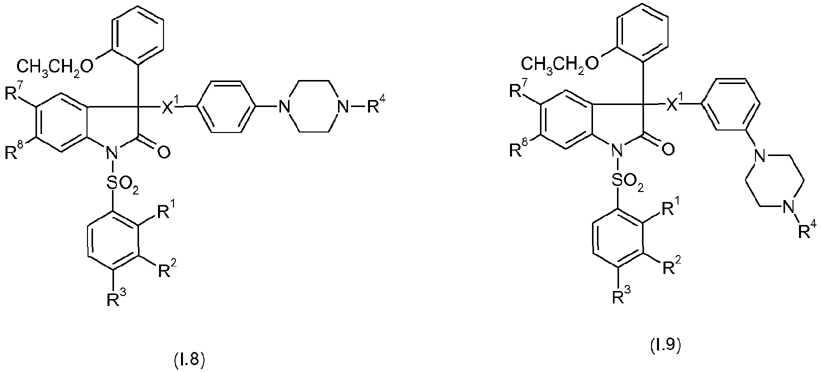

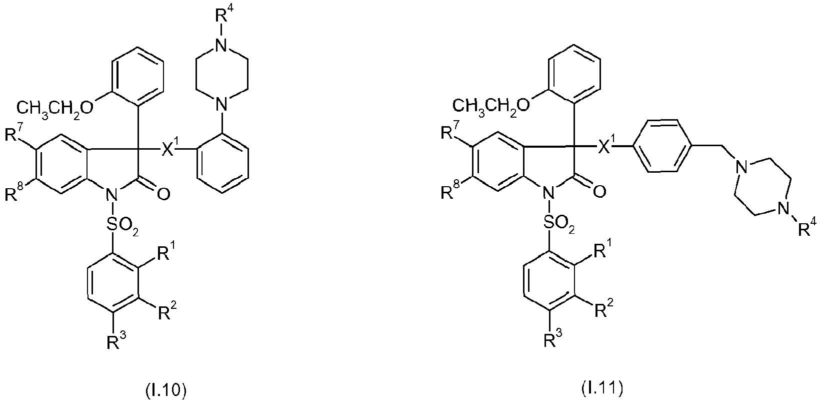

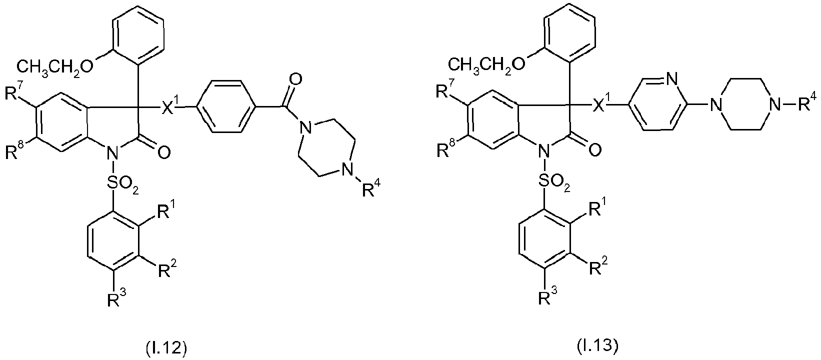

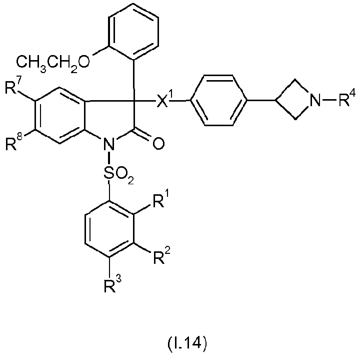

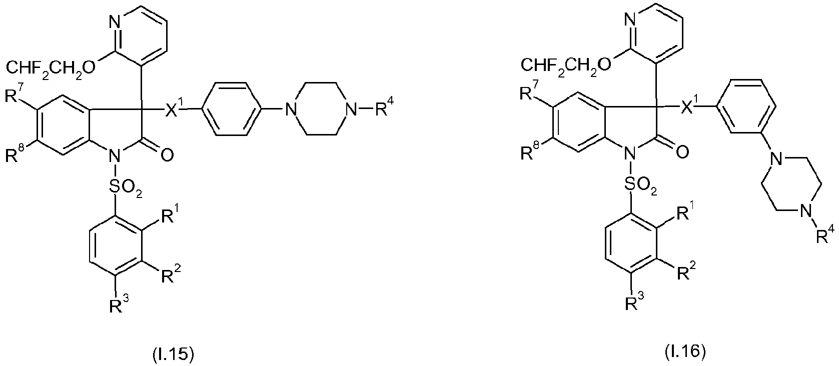

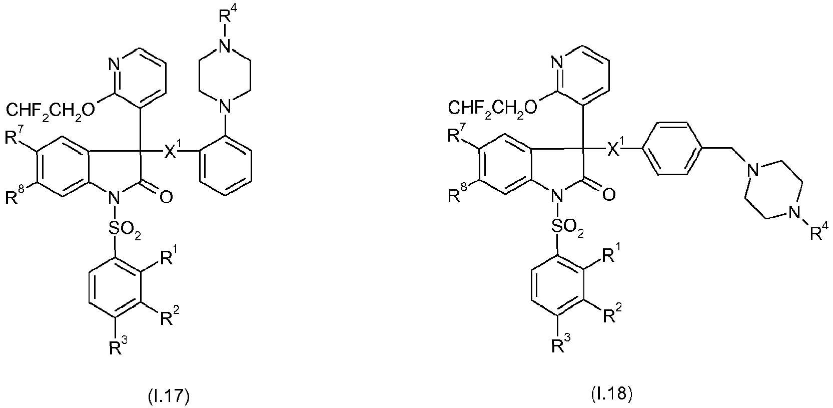

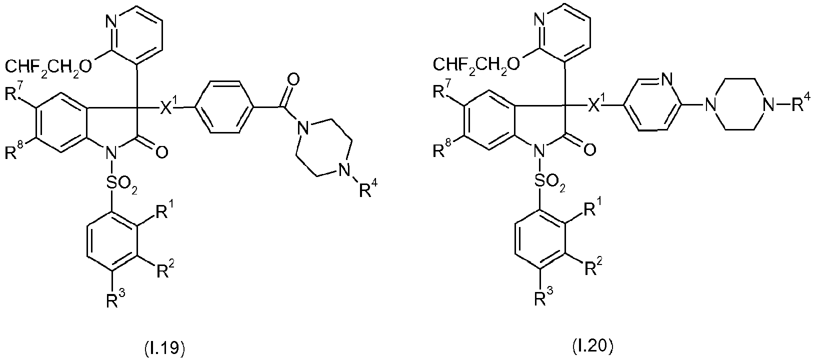

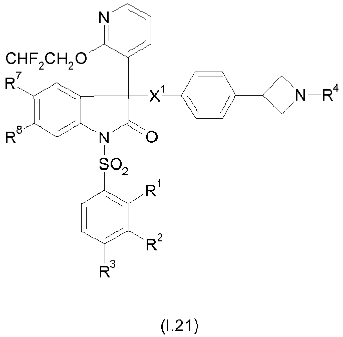

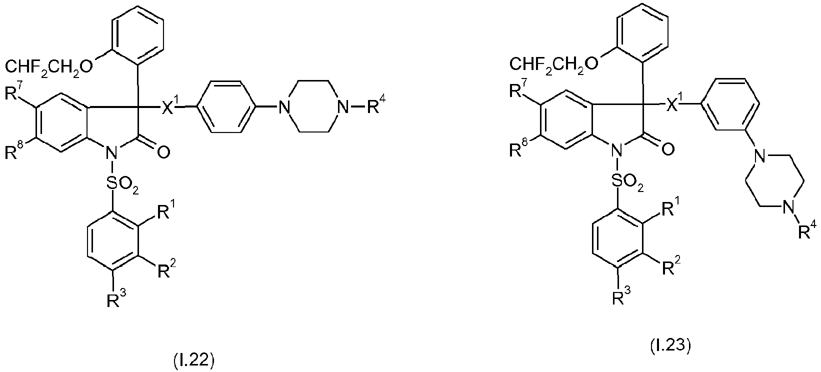

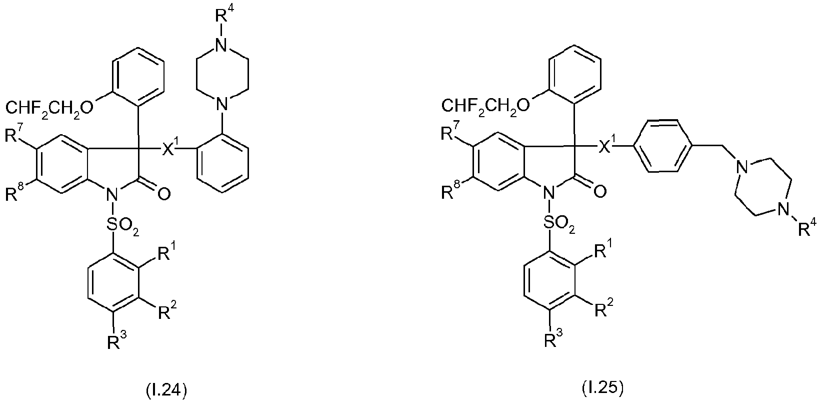

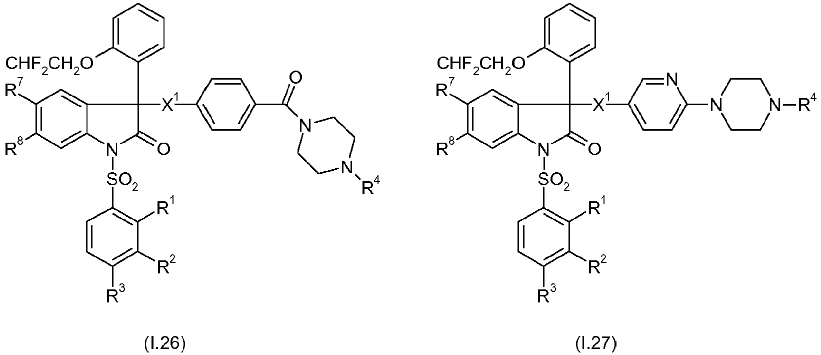

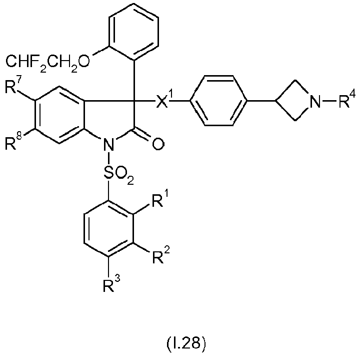

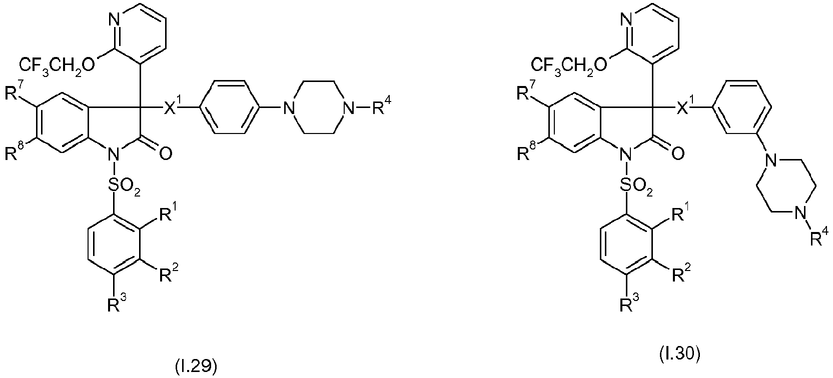

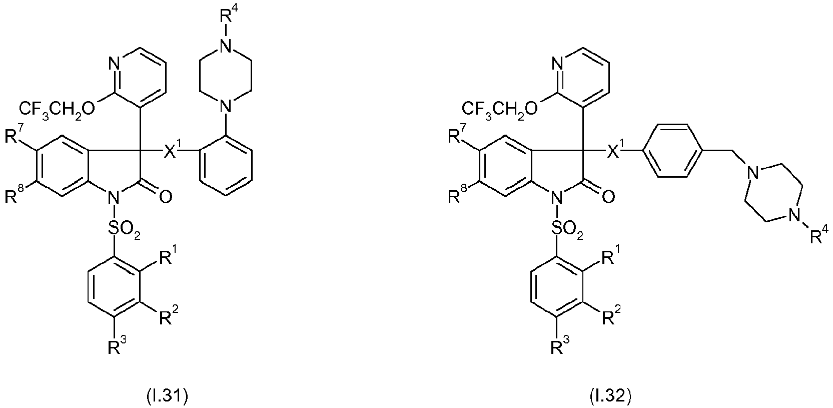

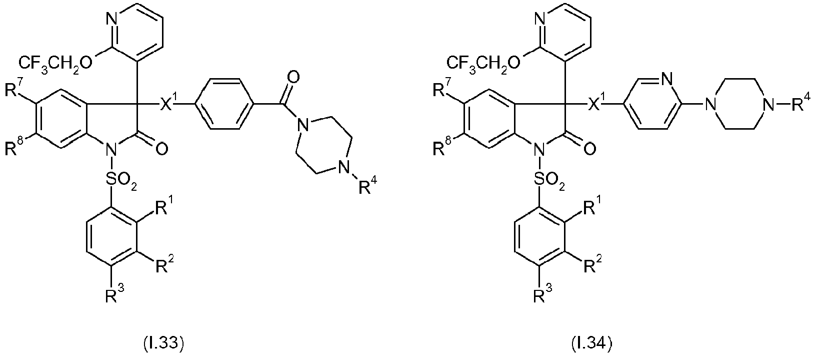

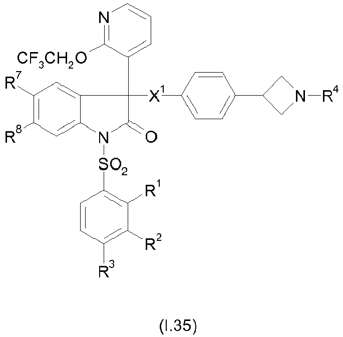

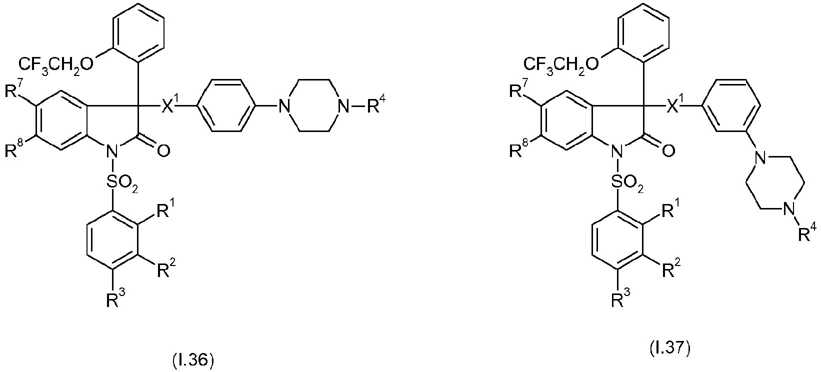

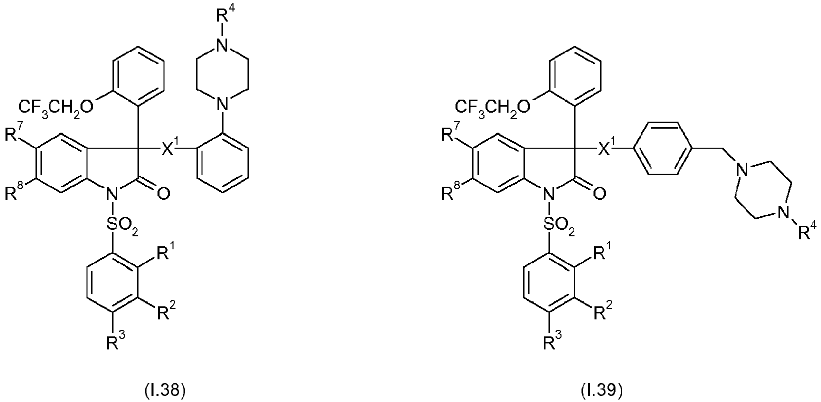

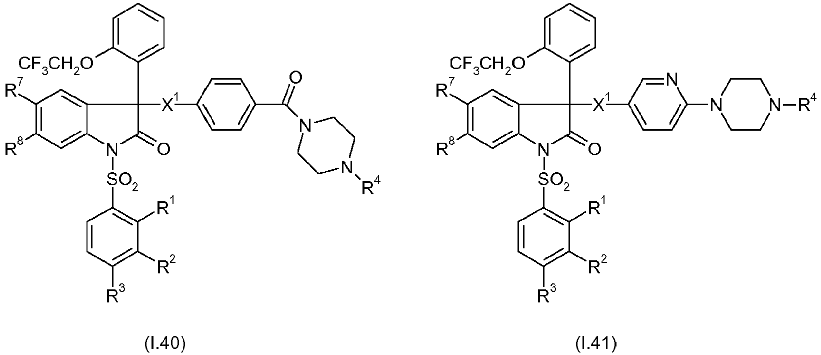

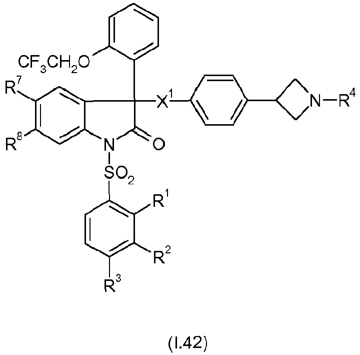

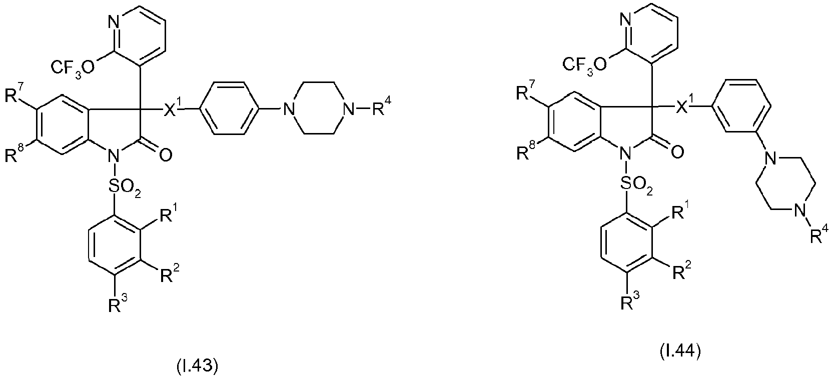

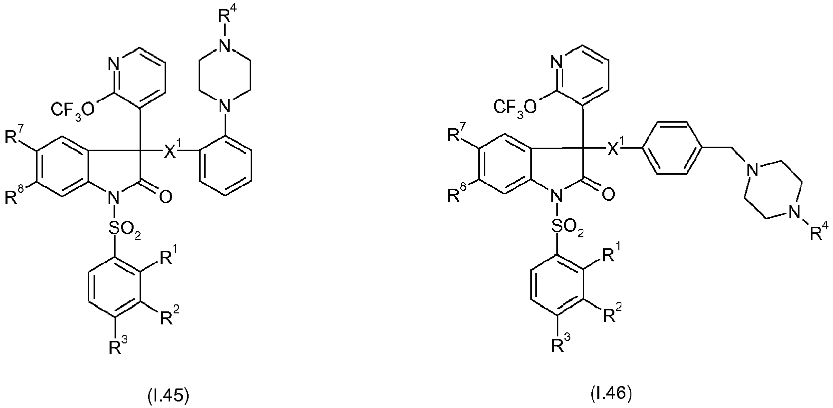

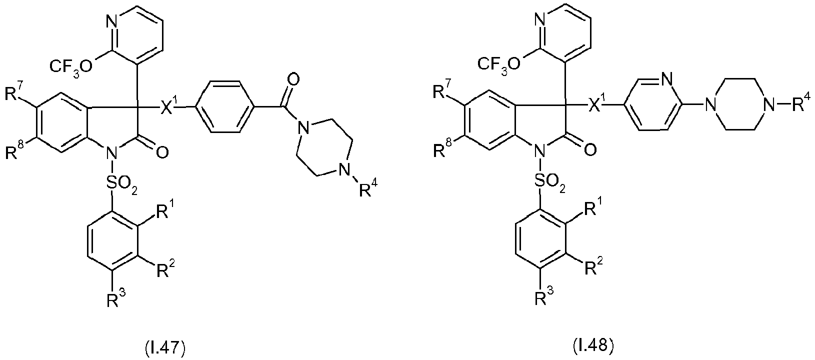

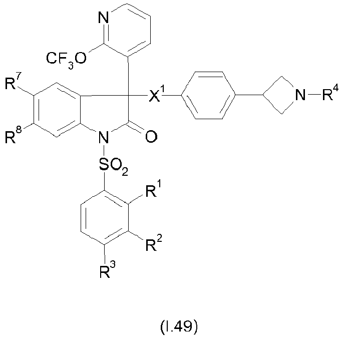

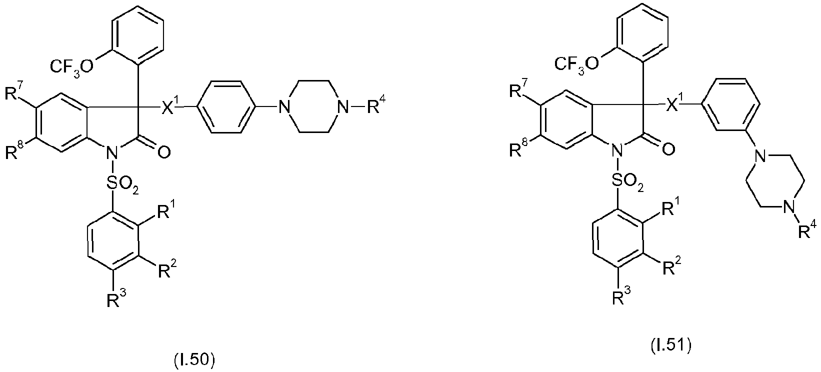

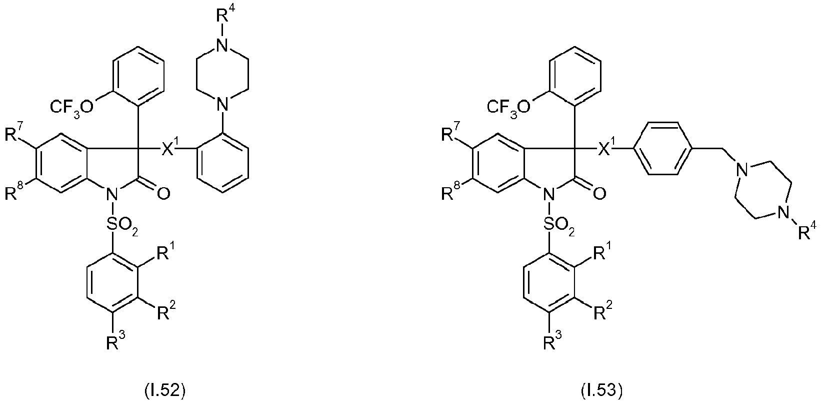

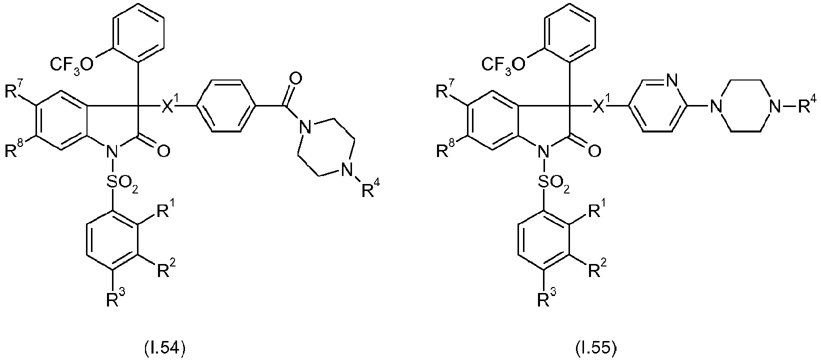

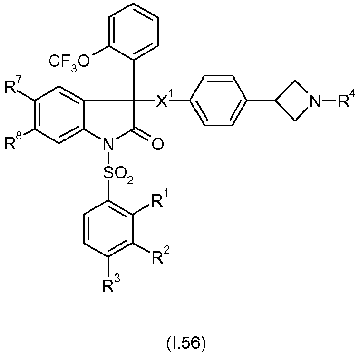

Bevorzugte Verbindungen der Formeln I.1 bis I.56 sind in folgenden Tabellen 1 bis 336 aufgeführt:

### Tabelle 1

Verbindungen der Formel I.1, worin X¹ für -NH₂-CH₂- steht und die Kombination aus R¹, R², R³, R⁴, R⁷ und R⁸ jeweils einer Zeile in Tabelle A entspricht

### Tabelle 2

Verbindungen der Formel I.1, worin X¹ für -NH- steht und die Kombination aus R¹, R², R³, R⁴, R⁷ und R⁸ jeweils einer Zeile in Tabelle A entspricht

### Tabelle 3

Verbindungen der Formel I.1, worin X¹ für -NH-C(=O)- steht und die Kombination aus R¹, R², R³, R⁴, R⁷ und R⁸ jeweils einer Zeile in Tabelle A entspricht

### Tabelle 4

Verbindungen der Formel I.1, worin X¹ für -O-CH₂- steht und die Kombination aus R¹, R², R³, R⁴, R⁷ und R⁸ jeweils einer Zeile in Tabelle A entspricht

### Tabelle 5

Verbindungen der Formel I.1, worin X¹ für -O- steht und die Kombination aus R¹, R², R³, R⁴, R⁷ und R⁸ jeweils einer Zeile in Tabelle A entspricht

### Tabelle 6

Verbindungen der Formel I.1, worin X¹ für -O-C(=O)- steht und die Kombination aus R¹, R², R³, R⁴, R⁷ und R⁸ jeweils einer Zeile in Tabelle A entspricht

### Tabellen 7 bis 12

Verbindungen der Formel I.2, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 13 bis 18

Verbindungen der Formel I.3, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 19 bis 24

Verbindungen der Formel I.4, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 25 bis 30

Verbindungen der Formel I.5, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 31 bis 36

Verbindungen der Formel I.6, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 37 bis 42

Verbindungen der Formel I.7, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 43 bis 48

Verbindungen der Formel I.8, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 49 bis 54

Verbindungen der Formel I.9, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 55 bis 60

Verbindungen der Formel I.10, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 61 bis 66

Verbindungen der Formel I.11, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 67 bis 72

Verbindungen der Formel I.12, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 73 bis 78

Verbindungen der Formel I.13, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 79 bis 84

Verbindungen der Formel I.14, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 85 bis 90

Verbindungen der Formel 1.15, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 91 bis 96

Verbindungen der Formel 1.16, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 97 bis 102

Verbindungen der Formel 1.17, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 103 bis 108

Verbindungen der Formel 1.18, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 109 bis 114

Verbindungen der Formel I.19, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 115 bis 120

Verbindungen der Formel I.20, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 121 bis 126

Verbindungen der Formel I.21, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 127 bis 132

Verbindungen der Formel I.22, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 133 bis 138

Verbindungen der Formel I.23, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 139 bis 144

Verbindungen der Formel I.24, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 145 bis 150

Verbindungen der Formel I.25, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 151 bis 156

Verbindungen der Formel I.26, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 157 bis 162

Verbindungen der Formel I.27, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 163 bis 168

Verbindungen der Formel I.28, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 169 bis 174

Verbindungen der Formel I.29, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 175 bis 180

Verbindungen der Formel I.30, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 181 bis 186

Verbindungen der Formel I.31, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 187 bis 192

Verbindungen der Formel I.32, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 193 bis 198

Verbindungen der Formel I.33, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 199 bis 204

Verbindungen der Formel I.34, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 205 bis 210

Verbindungen der Formel I.35, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 211 bis 216

Verbindungen der Formel I.36, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 217 bis 222

Verbindungen der Formel I.37, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 223 bis 228

Verbindungen der Formel I.38, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 229 bis 234

Verbindungen der Formel 1.39, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 235 bis 240

Verbindungen der Formel I.40, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 241 bis 246

Verbindungen der Formel I.41, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 247 bis 252

Verbindungen der Formel I.42, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 253 bis 258

Verbindungen der Formel I.43, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 259 bis 264

Verbindungen der Formel I.44, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 265 bis 270

Verbindungen der Formel I.45, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 271 bis 276

Verbindungen der Formel I.46, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 277 bis 282

Verbindungen der Formel I.47, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 283 bis 288

Verbindungen der Formel I.48, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 289 bis 294

Verbindungen der Formel 1.49, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 295 bis 300

Verbindungen der Formel I.50, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 301 bis 306

Verbindungen der Formel I.51, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 307 bis 312

Verbindungen der Formel I.52, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 313 bis 318

Verbindungen der Formel I.53, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 319 bis 324

Verbindungen der Formel I.54, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 325 bis 330

Verbindungen der Formel I.55, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

### Tabellen 331 bis 336

Verbindungen der Formel I.56, worin X¹ jeweils eine der in den Tabellen 1, 2, 3, 4, 5 oder 6 angegebenen Bedeutungen hat und die Kombination aus R¹, R², R³, R⁴, R⁷ und R⁸ jeweils einer Zeile in Tabelle A entspricht

**Tabelle A:**

| **Beispiel Nr.** | **R¹** | **R²** | **R³** | **R⁴** | **R⁷** | **R⁸** |
|---|---|---|---|---|---|---|
| A-1. | H | H | H | H | CN | H |
| A-2. | Methoxy | H | H | H | CN | H |
| A-3. | Ethoxy | H | H | H | CN | H |
| A-4. | OCF₃ | H | H | H | CN | H |
| A-5. | H | H | Methoxy | H | CN | H |
| A-6. | Methoxy | H | Methoxy | H | CN | H |
| A-7. | Ethoxy | H | Methoxy | H | CN | H |
| A-8. | OCF₃ | H | Methoxy | H | CN | H |
| A-9. | H | H | Ethoxy | H | CN | H |
| A-10. | Methoxy | H | Ethoxy | H | CN | H |
| A-11. | Ethoxy | H | Ethoxy | H | CN | H |
| A-12. | OCF₃ | H | Ethoxy | H | CN | H |
| A-13. | H | H | OCH₂F | H | CN | H |
| A-14. | H | H | OCHF₂ | H | CN | H |
| A-15. | H | H | OCF₃ | H | CN | H |
| A-16. | H | H | OCH₂CH₂F | H | CN | H |
| A-17. | H | H | OCH₂CHF₂ | H | CN | H |
| A-18. | H | H | OCH₂CF₃ | H | CN | H |
| A-19. | H | H | Cl | H | CN | H |
| A-20. | H | H | CN | H | CN | H |
| A-21. | H | Methoxy | H | H | CN | H |
| A-22. | H | H | H | Methyl | CN | H |
| A-23. | Methoxy | H | H | Methyl | CN | H |
| A-24. | Ethoxy | H | H | Methyl | CN | H |
| A-25. | OCF₃ | H | H | Methyl | CN | H |
| A-26. | H | H | Methoxy | Methyl | CN | H |
| A-27. | Methoxy | H | Methoxy | Methyl | CN | H |
| A-28. | Ethoxy | H | Methoxy | Methyl | CN | H |
| A-29. | OCF₃ | H | Methoxy | Methyl | CN | H |
| A-30. | H | H | Ethoxy | Methyl | CN | H |
| A-31. | Methoxy | H | Ethoxy | Methyl | CN | H |
| A-32. | Ethoxy | H | Ethoxy | Methyl | CN | H |
| A-33. | OCF₃ | H | Ethoxy | Methyl | CN | H |
| A-34. | H | H | OCH₂F | Methyl | CN | H |
| A-35. | H | H | OCHF₂ | Methyl | CN | H |
| A-36. | H | H | OCF₃ | Methyl | CN | H |
| A-37. | H | H | OCH₂CH₂F | Methyl | CN | H |
| A-38. | H | H | OCH₂CHF₂ | Methyl | CN | H |
| A-39. | H | H | OCH₂CF₃ | Methyl | CN | H |
| A-40. | H | H | Cl | Methyl | CN | H |
| A-41. | H | H | CN | Methyl | CN | H |
| A-42. | H | Methoxy | H | Methyl | CN | H |
| A-43. | H | H | H | Ethyl | CN | H |
| A-44. | Methoxy | H | H | Ethyl | CN | H |
| A-45. | Ethoxy | H | H | Ethyl | CN | H |
| A-46. | OCF₃ | H | H | Ethyl | CN | H |
| A-47. | H | H | Methoxy | Ethyl | CN | H |
| A-48. | Methoxy | H | Methoxy | Ethyl | CN | H |
| A-49. | Ethoxy | H | Methoxy | Ethyl | CN | H |
| A-50. | OCF₃ | H | Methoxy | Ethyl | CN | H |
| A-51. | H | H | Ethoxy | Ethyl | CN | H |
| A-52. | Methoxy | H | Ethoxy | Ethyl | CN | H |
| A-53. | Ethoxy | H | Ethoxy | Ethyl | CN | H |
| A-54. | OCF₃ | H | Ethoxy | Ethyl | CN | H |
| A-55. | H | H | OCH₂F | Ethyl | CN | H |
| A-56. | H | H | OCHF₂ | Ethyl | CN | H |
| A-57. | H | H | OCF₃ | Ethyl | CN | H |
| A-58. | H | H | OCH₂CH₂F | Ethyl | CN | H |
| A-59. | H | H | OCH₂CHF₂ | Ethyl | CN | H |
| A-60. | H | H | OCH₂CF₃ | Ethyl | CN | H |
| A-61. | H | H | Cl | Ethyl | CN | H |
| A-62. | H | H | CN | Ethyl | CN | H |
| A-63. | H | Methoxy | H | Ethyl | CN | H |
| A-64. | H | H | H | n-Propyl | CN | H |
| A-65. | Methoxy | H | H | n-Propyl | CN | H |
| A-66. | Ethoxy | H | H | n-Propyl | CN | H |
| A-67. | OCF₃ | H | H | n-Propyl | CN | H |
| A-68. | H | H | Methoxy | n-Propyl | CN | H |
| A-69. | Methoxy | H | Methoxy | n-Propyl | CN | H |
| A-70. | Ethoxy | H | Methoxy | n-Propyl | CN | H |
| A-71. | OCF₃ | H | Methoxy | n-Propyl | CN | H |
| A-72. | H | H | Ethoxy | n-Propyl | CN | H |
| A-73. | Methoxy | H | Ethoxy | n-Propyl | CN | H |
| A-74. | Ethoxy | H | Ethoxy | n-Propyl | CN | H |
| A-75. | OCF₃ | H | Ethoxy | n-Propyl | CN | H |
| A-76. | H | H | OCH₂F | n-Propyl | CN | H |
| A-77. | H | H | OCHF₂ | n-Propyl | CN | H |
| A-78. | H | H | OCF₃ | n-Propyl | CN | H |
| A-79. | H | H | OCH₂CH₂F | n-Propyl | CN | H |
| A-80. | H | H | OCH₂CHF₂ | n-Propyl | CN | H |
| A-81. | H | H | OCH₂CF₃ | n-Propyl | CN | H |
| A-82. | H | H | CI | n-Propyl | CN | H |
| A-83. | H | H | CN | n-Propyl | CN | H |
| A-84. | H | Methoxy | H | n-Propyl | CN | H |
| A-85. | H | H | H | Isopropyl | CN | H |
| A-86. | Methoxy | H | H | Isopropyl | CN | H |
| A-87. | Ethoxy | H | H | Isopropyl | CN | H |
| A-88. | OCF₃ | H | H | Isopropyl | CN | H |
| A-89. | H | H | Methoxy | Isopropyl | CN | H |
| A-90. | Methoxy | H | Methoxy | Isopropyl | CN | H |
| A-91. | Ethoxy | H | Methoxy | Isopropyl | CN | H |
| A-92. | OCF₃ | H | Methoxy | Isopropyl | CN | H |
| A-93. | H | H | Ethoxy | Isopropyl | CN | H |
| A-94. | Methoxy | H | Ethoxy | Isopropyl | CN | H |
| A-95. | Ethoxy | H | Ethoxy | Isopropyl | CN | H |
| A-96. | OCF₃ | H | Ethoxy | Isopropyl | CN | H |
| A-97. | H | H | OCH₂F | Isopropyl | CN | H |
| A-98. | H | H | OCHF₂ | Isopropyl | CN | H |
| A-99. | H | H | OCF₃ | Isopropyl | CN | H |
| A-100. | H | H | OCH₂CH₂F | Isopropyl | CN | H |
| A-101. | H | H | OCH₂CHF₂ | Isopropyl | CN | H |
| A-102. | H | H | OCH₂CF₃ | Isopropyl | CN | H |
| A-103. | H | H | Cl | Isopropyl | CN | H |
| A-104. | H | H | CN | Isopropyl | CN | H |
| A-105. | H | Methoxy | H | Isopropyl | CN | H |
| A-106. | H | H | H | H | I | H |
| A-107. | Methoxy | H | H | H | I | H |
| A-108. | Ethoxy | H | H | H | I | H |
| A-109. | OCF₃ | H | H | H | I | H |
| A-110. | H | H | Methoxy | H | I | H |
| A-111. | Methoxy | H | Methoxy | H | I | H |
| A-112. | Ethoxy | H | Methoxy | H | I | H |
| A-113. | OCF₃ | H | Methoxy | H | I | H |
| A-114. | H | H | Ethoxy | H | I | H |
| A-115. | Methoxy | H | Ethoxy | H | I | H |
| A-116. | Ethoxy | H | Ethoxy | H | I | H |
| A-117. | OCF₃ | H | Ethoxy | H | I | H |
| A-118. | H | H | OCH₂F | H | I | H |
| A-119. | H | H | OCHF₂ | H | I | H |
| A-120. | H | H | OCF₃ | H | I | H |
| A-121. | H | H | OCH₂CH₂F | H | I | H |
| A-122. | H | H | OCH₂CHF₂ | H | I | H |
| A-123. | H | H | OCH₂CF₃ | H | I | H |
| A-124. | H | H | Cl | H | I | H |
| A-125. | H | H | CN | H | I | H |
| A-126. | H | Methoxy | H | H | I | H |
| A-127. | H | H | H | Methyl | I | H |
| A-128. | Methoxy | H | H | Methyl | I | H |
| A-129. | Ethoxy | H | H | Methyl | I | H |
| A-130. | OCF₃ | H | H | Methyl | I | H |
| A-131. | H | H | Methoxy | Methyl | I | H |
| A-132. | Methoxy | H | Methoxy | Methyl | I | H |
| A-133. | Ethoxy | H | Methoxy | Methyl | I | H |
| A-134. | OCF₃ | H | Methoxy | Methyl | I | H |
| A-135. | H | H | Ethoxy | Methyl | I | H |
| A-136. | Methoxy | H | Ethoxy | Methyl | I | H |
| A-137. | Ethoxy | H | Ethoxy | Methyl | I | H |
| A-138. | OCF₃ | H | Ethoxy | Methyl | I | H |
| A-139. | H | H | OCH₂F | Methyl | I | H |
| A-140. | H | H | OCHF₂ | Methyl | I | H |
| A-141. | H | H | OCF₃ | Methyl | I | H |
| A-142. | H | H | OCH₂CH₂F | Methyl | I | H |
| A-143. | H | H | OCH₂CHF₂ | Methyl | I | H |
| A-144. | H | H | OCH₂CF₃ | Methyl | I | H |
| A-145. | H | H | Cl | Methyl | I | H |
| A-146. | H | H | CN | Methyl | I | H |
| A-147. | H | Methoxy | H | Methyl | I | H |
| A-148. | H | H | H | Ethyl | I | H |
| A-149. | Methoxy | H | H | Ethyl | I | H |
| A-150. | Ethoxy | H | H | Ethyl | I | H |
| A-151. | OCF₃ | H | H | Ethyl | I | H |
| A-152. | H | H | Methoxy | Ethyl | I | H |
| A-153. | Methoxy | H | Methoxy | Ethyl | I | H |
| A-154. | Ethoxy | H | Methoxy | Ethyl | I | H |
| A-155. | OCF₃ | H | Methoxy | Ethyl | I | H |
| A-156. | H | H | Ethoxy | Ethyl | I | H |
| A-157. | Methoxy | H | Ethoxy | Ethyl | I | H |
| A-158. | Ethoxy | H | Ethoxy | Ethyl | I | H |
| A-159. | OCF₃ | H | Ethoxy | Ethyl | I | H |
| A-160. | H | H | OCH₂F | Ethyl | I | H |
| A-161. | H | H | OCHF₂ | Ethyl | I | H |
| A-162. | H | H | OCF₃ | Ethyl | I | H |
| A-163. | H | H | OCH₂CH₂F | Ethyl | I | H |
| A-164. | H | H | OCH₂CHF₂ | Ethyl | I | H |
| A-165. | H | H | OCH₂CF₃ | Ethyl | I | H |
| A-166. | H | H | Cl | Ethyl | I | H |
| A-167. | H | H | CN | Ethyl | I | H |
| A-168. | H | Methoxy | H | Ethyl | I | H |
| A-169. | H | H | H | n-Propyl | I | H |
| A-170. | Methoxy | H | H | n-Propyl | I | H |
| A-171. | Ethoxy | H | H | n-Propyl | I | H |
| A-172. | OCF₃ | H | H | n-Propyl | I | H |
| A-173. | H | H | Methoxy | n-Propyl | I | H |
| A-174. | Methoxy | H | Methoxy | n-Propyl | I | H |
| A-175. | Ethoxy | H | Methoxy | n-Propyl | I | H |
| A-176. | OCF₃ | H | Methoxy | n-Propyl | I | H |
| A-177. | H | H | Ethoxy | n-Propyl | I | H |
| A-178. | Methoxy | H | Ethoxy | n-Propyl | I | H |
| A-179. | Ethoxy | H | Ethoxy | n-Propyl | I | H |
| A-180. | OCF₃ | H | Ethoxy | n-Propyl | I | H |
| A-181. | H | H | OCH₂F | n-Propyl | I | H |
| A-182. | H | H | OCHF₂ | n-Propyl | I | H |
| A-183. | H | H | OCF₃ | n-Propyl | I | H |
| A-184. | H | H | OCH₂CH₂F | n-Propyl | I | H |
| A-185. | H | H | OCH₂CHF₂ | n-Propyl | I | H |
| A-186. | H | H | OCH₂CF₃ | n-Propyl | I | H |
| A-187. | H | H | Cl | n-Propyl | I | H |
| A-188. | H | H | CN | n-Propyl | I | H |
| A-189. | H | Methoxy | H | n-Propyl | I | H |
| A-190. | H | H | H | Isopropyl | I | H |
| A-191. | Methoxy | H | H | Isopropyl | I | H |
| A-192. | Ethoxy | H | H | Isopropyl | I | H |
| A-193. | OCF₃ | H | H | Isopropyl | I | H |
| A-194. | H | H | Methoxy | Isopropyl | I | H |
| A-195. | Methoxy | H | Methoxy | Isopropyl | I | H |
| A-196. | Ethoxy | H | Methoxy | Isopropyl | I | H |
| A-197. | OCF₃ | H | Methoxy | Isopropyl | I | H |
| A-198. | H | H | Ethoxy | Isopropyl | I | H |
| A-199. | Methoxy | H | Ethoxy | Isopropyl | I | H |
| A-200. | Ethoxy | H | Ethoxy | Isopropyl | I | H |
| A-201. | OCF₃ | H | Ethoxy | Isopropyl | I | H |
| A-202. | H | H | OCH₂F | Isopropyl | I | H |
| A-203. | H | H | OCHF₂ | Isopropyl | I | H |
| A-204. | H | H | OCF₃ | Isopropyl | I | H |
| A-205. | H | H | OCH₂CH₂F | Isopropyl | I | H |
| A-206. | H | H | OCH₂CHF₂ | Isopropyl | I | H |
| A-207. | H | H | OCH₂CF₃ | Isopropyl | I | H |
| A-208. | H | H | Cl | Isopropyl | I | H |
| A-209. | H | H | CN | Isopropyl | I | H |
| A-210. | H | Methoxy | H | Isopropyl | I | H |
| A-211. | H | H | H | H | Br | H |
| A-212. | Methoxy | H | H | H | Br | H |
| A-213. | Ethoxy | H | H | H | Br | H |
| A-214. | OCF₃ | H | H | H | Br | H |
| A-215. | H | H | Methoxy | H | Br | H |
| A-216. | Methoxy | H | Methoxy | H | Br | H |
| A-217. | Ethoxy | H | Methoxy | H | Br | H |
| A-218. | OCF₃ | H | Methoxy | H | Br | H |
| A-219. | H | H | Ethoxy | H | Br | H |
| A-220. | Methoxy | H | Ethoxy | H | Br | H |
| A-221. | Ethoxy | H | Ethoxy | H | Br | H |
| A-222. | OCF₃ | H | Ethoxy | H | Br | H |
| A-223. | H | H | OCH₂F | H | Br | H |
| A-224. | H | H | OCHF₂ | H | Br | H |
| A-225. | H | H | OCF₃ | H | Br | H |
| A-226. | H | H | OCH₂CH₂F | H | Br | H |
| A-227. | H | H | OCH₂CHF₂ | H | Br | H |
| A-228. | H | H | OCH₂CF₃ | H | Br | H |
| A-229. | H | H | Cl | H | Br | H |
| A-230. | H | H | CN | H | Br | H |
| A-231. | H | Methoxy | H | H | Br | H |
| A-232. | H | H | H | Methyl | Br | H |
| A-233. | Methoxy | H | H | Methyl | Br | H |
| A-234. | Ethoxy | H | H | Methyl | Br | H |
| A-235. | OCF₃ | H | H | Methyl | Br | H |
| A-236. | H | H | Methoxy | Methyl | Br | H |
| A-237. | Methoxy | H | Methoxy | Methyl | Br | H |
| A-238. | Ethoxy | H | Methoxy | Methyl | Br | H |
| A-239. | OCF₃ | H | Methoxy | Methyl | Br | H |
| A-240. | H | H | Ethoxy | Methyl | Br | H |
| A-241. | Methoxy | H | Ethoxy | Methyl | Br | H |
| A-242. | Ethoxy | H | Ethoxy | Methyl | Br | H |
| A-243. | OCF₃ | H | Ethoxy | Methyl | Br | H |
| A-244. | H | H | OCH₂F | Methyl | Br | H |
| A-245. | H | H | OCHF₂ | Methyl | Br | H |
| A-246. | H | H | OCF₃ | Methyl | Br | H |
| A-247. | H | H | OCH₂CH₂F | Methyl | Br | H |
| A-248. | H | H | OCH₂CHF₂ | Methyl | Br | H |
| A-249. | H | H | OCH₂CF₃ | Methyl | Br | H |
| A-250. | H | H | Cl | Methyl | Br | H |
| A-251. | H | H | CN | Methyl | Br | H |
| A-252. | H | Methoxy | H | Methyl | Br | H |
| A-253. | H | H | H | Ethyl | Br | H |
| A-254. | Methoxy | H | H | Ethyl | Br | H |
| A-255. | Ethoxy | H | H | Ethyl | Br | H |
| A-256. | OCF₃ | H | H | Ethyl | Br | H |
| A-257. | H | H | Methoxy | Ethyl | Br | H |
| A-258. | Methoxy | H | Methoxy | Ethyl | Br | H |
| A-259. | Ethoxy | H | Methoxy | Ethyl | Br | H |
| A-260. | OCF₃ | H | Methoxy | Ethyl | Br | H |
| A-261. | H | H | Ethoxy | Ethyl | Br | H |
| A-262. | Methoxy | H | Ethoxy | Ethyl | Br | H |
| A-263. | Ethoxy | H | Ethoxy | Ethyl | Br | H |
| A-264. | OCF₃ | H | Ethoxy | Ethyl | Br | H |
| A-265. | H | H | OCH₂F | Ethyl | Br | H |
| A-266. | H | H | OCHF₂ | Ethyl | Br | H |
| A-267. | H | H | OCF₃ | Ethyl | Br | H |
| A-268. | H | H | OCH₂CH₂F | Ethyl | Br | H |
| A-269. | H | H | OCH₂CHF₂ | Ethyl | Br | H |
| A-270. | H | H | OCH₂CF₃ | Ethyl | Br | H |
| A-271. | H | H | Cl | Ethyl | Br | H |
| A-272. | H | H | CN | Ethyl | Br | H |
| A-273. | H | Methoxy | H | Ethyl | Br | H |
| A-274. | H | H | H | n-Propyl | Br | H |
| A-275. | Methoxy | H | H | n-Propyl | Br | H |
| A-276. | Ethoxy | H | H | n-Propyl | Br | H |
| A-277. | OCF₃ | H | H | n-Propyl | Br | H |
| A-278. | H | H | Methoxy | n-Propyl | Br | H |
| A-279. | Methoxy | H | Methoxy | n-Propyl | Br | H |
| A-280. | Ethoxy | H | Methoxy | n-Propyl | Br | H |
| A-281. | OCF₃ | H | Methoxy | n-Propyl | Br | H |
| A-282. | H | H | Ethoxy | n-Propyl | Br | H |
| A-283. | Methoxy | H | Ethoxy | n-Propyl | Br | H |
| A-284. | Ethoxy | H | Ethoxy | n-Propyl | Br | H |
| A-285. | OCF₃ | H | Ethoxy | n-Propyl | Br | H |
| A-286. | H | H | OCH₂F | n-Propyl | Br | H |
| A-287. | H | H | OCHF₂ | n-Propyl | Br | H |
| A-288. | H | H | OCF₃ | n-Propyl | Br | H |
| A-289. | H | H | OCH₂CH₂F | n-Propyl | Br | H |
| A-290. | H | H | OCH₂CHF₂ | n-Propyl | Br | H |
| A-291. | H | H | OCH₂CF₃ | n-Propyl | Br | H |
| A-292. | H | H | Cl | n-Propyl | Br | H |
| A-293. | H | H | CN | n-Propyl | Br | H |
| A-294. | H | Methoxy | H | n-Propyl | Br | H |
| A-295. | H | H | H | Isopropyl | Br | H |
| A-296. | Methoxy | H | H | Isopropyl | Br | H |
| A-297. | Ethoxy | H | H | Isopropyl | Br | H |
| A-298. | OCF₃ | H | H | Isopropyl | Br | H |
| A-299. | H | H | Methoxy | Isopropyl | Br | H |
| A-300. | Methoxy | H | Methoxy | Isopropyl | Br | H |
| A-301. | Ethoxy | H | Methoxy | Isopropyl | Br | H |
| A-302. | OCF₃ | H | Methoxy | Isopropyl | Br | H |
| A-303. | H | H | Ethoxy | Isopropyl | Br | H |
| A-304. | Methoxy | H | Ethoxy | Isopropyl | Br | H |
| A-305. | Ethoxy | H | Ethoxy | Isopropyl | Br | H |
| A-306. | OCF₃ | H | Ethoxy | Isopropyl | Br | H |
| A-307. | H | H | OCH₂F | Isopropyl | Br | H |
| A-308. | H | H | OCHF₂ | Isopropyl | Br | H |
| A-309. | H | H | OCF₃ | Isopropyl | Br | H |
| A-310. | H | H | OCH₂CH₂F | Isopropyl | Br | H |
| A-311. | H | H | OCH₂CHF₂ | Isopropyl | Br | H |
| A-312. | H | H | OCH₂CF₃ | Isopropyl | Br | H |
| A-313. | H | H | Cl | Isopropyl | Br | H |
| A-314. | H | H | CN | Isopropyl | Br | H |
| A-315. | H | Methoxy | H | Isopropyl | Br | H |
| A-316. | H | H | H | H | Cl | H |
| A-317. | Methoxy | H | H | H | Cl | H |
| A-318. | Ethoxy | H | H | H | Cl | H |
| A-319. | OCF₃ | H | H | H | Cl | H |
| A-320. | H | H | Methoxy | H | Cl | H |
| A-321. | Methoxy | H | Methoxy | H | Cl | H |
| A-322. | Ethoxy | H | Methoxy | H | Cl | H |
| A-323. | OCF₃ | H | Methoxy | H | Cl | H |
| A-324. | H | H | Ethoxy | H | Cl | H |
| A-325. | Methoxy | H | Ethoxy | H | Cl | H |
| A-326. | Ethoxy | H | Ethoxy | H | Cl | H |
| A-327. | OCF₃ | H | Ethoxy | H | Cl | H |
| A-328. | H | H | OCH₂F | H | Cl | H |
| A-329. | H | H | OCHF₂ | H | Cl | H |
| A-330. | H | H | OCF₃ | H | Cl | H |
| A-331. | H | H | OCH₂CH₂F | H | Cl | H |
| A-332. | H | H | OCH₂CHF₂ | H | Cl | H |
| A-333. | H | H | OCH₂CF₃ | H | Cl | H |
| A-334. | H | H | Cl | H | Cl | H |
| A-335. | H | H | CN | H | Cl | H |
| A-336. | H | Methoxy | H | H | Cl | H |
| A-337. | H | H | H | Methyl | Cl | H |
| A-338. | Methoxy | H | H | Methyl | Cl | H |
| A-339. | Ethoxy | H | H | Methyl | Cl | H |
| A-340. | OCF₃ | H | H | Methyl | Cl | H |
| A-341. | H | H | Methoxy | Methyl | Cl | H |
| A-342. | Methoxy | H | Methoxy | Methyl | Cl | H |
| A-343. | Ethoxy | H | Methoxy | Methyl | Cl | H |
| A-344. | OCF₃ | H | Methoxy | Methyl | Cl | H |
| A-345. | H | H | Ethoxy | Methyl | Cl | H |
| A-346. | Methoxy | H | Ethoxy | Methyl | Cl | H |
| A-347. | Ethoxy | H | Ethoxy | Methyl | Cl | H |
| A-348. | OCF₃ | H | Ethoxy | Methyl | Cl | H |
| A-349. | H | H | OCH₂F | Methyl | Cl | H |
| A-350. | H | H | OCHF₂ | Methyl | Cl | H |
| A-351. | H | H | OCF₃ | Methyl | Cl | H |
| A-352. | H | H | OCH₂CH₂F | Methyl | Cl | H |
| A-353. | H | H | OCH₂CHF₂ | Methyl | Cl | H |
| A-354. | H | H | OCH₂CF₃ | Methyl | Cl | H |
| A-355. | H | H | Cl | Methyl | Cl | H |
| A-356. | H | H | CN | Methyl | Cl | H |
| A-357. | H | Methoxy | H | Methyl | Cl | H |
| A-358. | H | H | H | Ethyl | Cl | H |
| A-359. | Methoxy | H | H | Ethyl | Cl | H |
| A-360. | Ethoxy | H | H | Ethyl | Cl | H |
| A-361. | OCF₃ | H | H | Ethyl | Cl | H |
| A-362. | H | H | Methoxy | Ethyl | Cl | H |
| A-363. | Methoxy | H | Methoxy | Ethyl | Cl | H |
| A-364. | Ethoxy | H | Methoxy | Ethyl | Cl | H |
| A-365. | OCF₃ | H | Methoxy | Ethyl | Cl | H |
| A-366. | H | H | Ethoxy | Ethyl | Cl | H |
| A-367. | Methoxy | H | Ethoxy | Ethyl | Cl | H |
| A-368. | Ethoxy | H | Ethoxy | Ethyl | Cl | H |
| A-369. | OCF₃ | H | Ethoxy | Ethyl | Cl | H |
| A-370. | H | H | OCH₂F | Ethyl | Cl | H |
| A-371. | H | H | OCHF₂ | Ethyl | Cl | H |
| A-372. | H | H | OCF₃ | Ethyl | Cl | H |
| A-373. | H | H | OCH₂CH₂F | Ethyl | Cl | H |
| A-374. | H | H | OCH₂CHF₂ | Ethyl | Cl | H |
| A-375. | H | H | OCH₂CF₃ | Ethyl | Cl | H |
| A-376. | H | H | Cl | Ethyl | Cl | H |
| A-377. | H | H | CN | Ethyl | Cl | H |
| A-378. | H | Methoxy | H | Ethyl | Cl | H |
| A-379. | H | H | H | n-Propyl | Cl | H |
| A-380. | Methoxy | H | H | n-Propyl | Cl | H |
| A-381. | Ethoxy | H | H | n-Propyl | Cl | H |
| A-382. | OCF₃ | H | H | n-Propyl | Cl | H |
| A-383. | H | H | Methoxy | n-Propyl | Cl | H |
| A-384. | Methoxy | H | Methoxy | n-Propyl | Cl | H |
| A-385. | Ethoxy | H | Methoxy | n-Propyl | Cl | H |
| A-386. | OCF₃ | H | Methoxy | n-Propyl | Cl | H |
| A-387. | H | H | Ethoxy | n-Propyl | Cl | H |
| A-388. | Methoxy | H | Ethoxy | n-Propyl | Cl | H |
| A-389. | Ethoxy | H | Ethoxy | n-Propyl | Cl | H |
| A-390. | OCF₃ | H | Ethoxy | n-Propyl | Cl | H |
| A-391. | H | H | OCH₂F | n-Propyl | Cl | H |
| A-392. | H | H | OCHF₂ | n-Propyl | Cl | H |
| A-393. | H | H | OCF₃ | n-Propyl | Cl | H |
| A-394. | H | H | OCH₂CH₂F | n-Propyl | Cl | H |
| A-395. | H | H | OCH₂CHF₂ | n-Propyl | Cl | H |
| A-396. | H | H | OCH₂CF₃ | n-Propyl | Cl | H |
| A-397. | H | H | Cl | n-Propyl | Cl | H |
| A-398. | H | H | CN | n-Propyl | Cl | H |
| A-399. | H | Methoxy | H | n-Propyl | Cl | H |
| A-400. | H | H | H | Isopropyl | Cl | H |
| A-401. | Methoxy | H | H | Isopropyl | Cl | H |
| A-402. | Ethoxy | H | H | Isopropyl | Cl | H |
| A-403. | OCF₃ | H | H | Isopropyl | Cl | H |
| A-404. | H | H | Methoxy | Isopropyl | Cl | H |
| A-405. | Methoxy | H | Methoxy | Isopropyl | Cl | H |
| A-406. | Ethoxy | H | Methoxy | Isopropyl | Cl | H |
| A-407. | OCF₃ | H | Methoxy | Isopropyl | Cl | H |
| A-408. | H | H | Ethoxy | Isopropyl | Cl | H |
| A-409. | Methoxy | H | Ethoxy | Isopropyl | Cl | H |
| A-410. | Ethoxy | H | Ethoxy | Isopropyl | Cl | H |
| A-411. | OCF₃ | H | Ethoxy | Isopropyl | Cl | H |
| A-412. | H | H | OCH₂F | Isopropyl | Cl | H |
| A-413. | H | H | OCHF₂ | Isopropyl | Cl | H |
| A-414. | H | H | OCF₃ | Isopropyl | Cl | H |
| A-415. | H | H | OCH₂CH₂F | Isopropyl | Cl | H |
| A-416. | H | H | OCH₂CHF₂ | Isopropyl | Cl | H |
| A-417. | H | H | OCH₂CF₃ | Isopropyl | Cl | H |
| A-418. | H | H | Cl | Isopropyl | Cl | H |
| A-419. | H | H | CN | Isopropyl | Cl | H |
| A-420. | H | Methoxy | H | Isopropyl | Cl | H |
| A-421. | H | H | H | H | F | H |
| A-422. | Methoxy | H | H | H | F | H |
| A-423. | Ethoxy | H | H | H | F | H |
| A-424. | OCF₃ | H | H | H | F | H |
| A-425. | H | H | Methoxy | H | F | H |
| A-426. | Methoxy | H | Methoxy | H | F | H |
| A-427. | Ethoxy | H | Methoxy | H | F | H |
| A-428. | OCF₃ | H | Methoxy | H | F | H |
| A-429. | H | H | Ethoxy | H | F | H |
| A-430. | Methoxy | H | Ethoxy | H | F | H |
| A-431. | Ethoxy | H | Ethoxy | H | F | H |
| A-432. | OCF₃ | H | Ethoxy | H | F | H |
| A-433. | H | H | OCH₂F | H | F | H |
| A-434. | H | H | OCHF₂ | H | F | H |
| A-435. | H | H | OCF₃ | H | F | H |
| A-436. | H | H | OCH₂CH₂F | H | F | H |
| A-437. | H | H | OCH₂CHF₂ | H | F | H |
| A-438. | H | H | OCH₂CF₃ | H | F | H |
| A-439. | H | H | Cl | H | F | H |
| A-440. | H | H | CN | H | F | H |
| A-441. | H | Methoxy | H | H | F | H |
| A-442. | H | H | H | Methyl | F | H |
| A-443. | Methoxy | H | H | Methyl | F | H |
| A-444. | Ethoxy | H | H | Methyl | F | H |
| A-445. | OCF₃ | H | H | Methyl | F | H |
| A-446. | H | H | Methoxy | Methyl | F | H |
| A-447. | Methoxy | H | Methoxy | Methyl | F | H |
| A-448. | Ethoxy | H | Methoxy | Methyl | F | H |
| A-449. | OCF₃ | H | Methoxy | Methyl | F | H |
| A-450. | H | H | Ethoxy | Methyl | F | H |
| A-451. | Methoxy | H | Ethoxy | Methyl | F | H |
| A-452. | Ethoxy | H | Ethoxy | Methyl | F | H |
| A-453. | OCF₃ | H | Ethoxy | Methyl | F | H |
| A-454. | H | H | OCH₂F | Methyl | F | H |
| A-455. | H | H | OCHF₂ | Methyl | F | H |
| A-456. | H | H | OCF₃ | Methyl | F | H |
| A-457. | H | H | OCH₂CH₂F | Methyl | F | H |
| A-458. | H | H | OCH₂CHF₂ | Methyl | F | H |
| A-459. | H | H | OCH₂CF₃ | Methyl | F | H |
| A-460. | H | H | Cl | Methyl | F | H |
| A-461. | H | H | CN | Methyl | F | H |
| A-462. | H | Methoxy | H | Methyl | F | H |
| A-463. | H | H | H | Ethyl | F | H |
| A-464. | Methoxy | H | H | Ethyl | F | H |
| A-465. | Ethoxy | H | H | Ethyl | F | H |
| A-466. | OCF₃ | H | H | Ethyl | F | H |
| A-467. | H | H | Methoxy | Ethyl | F | H |
| A-468. | Methoxy | H | Methoxy | Ethyl | F | H |
| A-469. | Ethoxy | H | Methoxy | Ethyl | F | H |
| A-470. | OCF₃ | H | Methoxy | Ethyl | F | H |
| A-471. | H | H | Ethoxy | Ethyl | F | H |
| A-472. | Methoxy | H | Ethoxy | Ethyl | F | H |
| A-473. | Ethoxy | H | Ethoxy | Ethyl | F | H |
| A-474. | OCF₃ | H | Ethoxy | Ethyl | F | H |
| A-475. | H | H | OCH₂F | Ethyl | F | H |
| A-476. | H | H | OCHF₂ | Ethyl | F | H |
| A-477. | H | H | OCF₃ | Ethyl | F | H |
| A-478. | H | H | OCH₂CH₂F | Ethyl | F | H |
| A-479. | H | H | OCH₂CHF₂ | Ethyl | F | H |
| A-480. | H | H | OCH₂CF₃ | Ethyl | F | H |
| A-481. | H | H | Cl | Ethyl | F | H |
| A-482. | H | H | CN | Ethyl | F | H |
| A-483. | H | Methoxy | H | Ethyl | F | H |
| A-484. | H | H | H | n-Propyl | F | H |
| A-485. | Methoxy | H | H | n-Propyl | F | H |
| A-486. | Ethoxy | H | H | n-Propyl | F | H |
| A-487. | OCF₃ | H | H | n-Propyl | F | H |
| A-488. | H | H | Methoxy | n-Propyl | F | H |
| A-489. | Methoxy | H | Methoxy | n-Propyl | F | H |
| A-490. | Ethoxy | H | Methoxy | n-Propyl | F | H |
| A-491. | OCF₃ | H | Methoxy | n-Propyl | F | H |
| A-492. | H | H | Ethoxy | n-Propyl | F | H |
| A-493. | Methoxy | H | Ethoxy | n-Propyl | F | H |
| A-494. | Ethoxy | H | Ethoxy | n-Propyl | F | H |
| A-495. | OCF₃ | H | Ethoxy | n-Propyl | F | H |
| A-496. | H | H | OCH₂F | n-Propyl | F | H |
| A-497. | H | H | OCHF₂ | n-Propyl | F | H |
| A-498. | H | H | OCF₃ | n-Propyl | F | H |
| A-499. | H | H | OCH₂CH₂F | n-Propyl | F | H |
| A-500. | H | H | OCH₂CHF₂ | n-Propyl | F | H |
| A-501. | H | H | OCH₂CF₃ | n-Propyl | F | H |
| A-502. | H | H | Cl | n-Propyl | F | H |
| A-503. | H | H | CN | n-Propyl | F | H |
| A-504. | H | Methoxy | H | n-Propyl | F | H |
| A-505. | H | H | H | Isopropyl | F | H |
| A-506. | Methoxy | H | H | Isopropyl | F | H |
| A-507. | Ethoxy | H | H | Isopropyl | F | H |
| A-508. | OCF₃ | H | H | Isopropyl | F | H |
| A-509. | H | H | Methoxy | Isopropyl | F | H |
| A-510. | Methoxy | H | Methoxy | Isopropyl | F | H |
| A-511. | Ethoxy | H | Methoxy | Isopropyl | F | H |
| A-512. | OCF₃ | H | Methoxy | Isopropyl | F | H |
| A-513. | H | H | Ethoxy | Isopropyl | F | H |
| A-514. | Methoxy | H | Ethoxy | Isopropyl | F | H |
| A-515. | Ethoxy | H | Ethoxy | Isopropyl | F | H |
| A-516. | OCF₃ | H | Ethoxy | Isopropyl | F | H |
| A-517. | H | H | OCH₂F | Isopropyl | F | H |
| A-518. | H | H | OCHF₂ | Isopropyl | F | H |
| A-519. | H | H | OCF₃ | Isopropyl | F | H |
| A-520. | H | H | OCH₂CH₂F | Isopropyl | F | H |
| A-521. | H | H | OCH₂CHF₂ | Isopropyl | F | H |
| A-522. | H | H | OCH₂CF₃ | Isopropyl | F | H |
| A-523. | H | H | Cl | Isopropyl | F | H |
| A-524. | H | H | CN | Isopropyl | F | H |
| A-525. | H | Methoxy | H | Isopropyl | F | H |
| A-526. | H | H | H | H | CN | F |
| A-527. | Methoxy | H | H | H | CN | F |
| A-528. | Ethoxy | H | H | H | CN | F |
| A-529. | OCF₃ | H | H | H | CN | F |
| A-530. | H | H | Methoxy | H | CN | F |
| A-531. | Methoxy | H | Methoxy | H | CN | F |
| A-532. | Ethoxy | H | Methoxy | H | CN | F |
| A-533. | OCF₃ | H | Methoxy | H | CN | F |
| A-534. | H | H | Ethoxy | H | CN | F |
| A-535. | Methoxy | H | Ethoxy | H | CN | F |
| A-536. | Ethoxy | H | Ethoxy | H | CN | F |
| A-537. | OCF₃ | H | Ethoxy | H | CN | F |
| A-538. | H | H | OCH₂F | H | CN | F |
| A-539. | H | H | OCHF₂ | H | CN | F |
| A-540. | H | H | OCF₃ | H | CN | F |
| A-541. | H | H | OCH₂CH₂F | H | CN | F |
| A-542. | H | H | OCH₂CHF₂ | H | CN | F |
| A-543. | H | H | OCH₂CF₃ | H | CN | F |
| A-544. | H | H | Cl | H | CN | F |
| A-545. | H | H | CN | H | CN | F |
| A-546. | H | Methoxy | H | H | CN | F |
| A-547. | H | H | H | Methyl | CN | F |
| A-548. | Methoxy | H | H | Methyl | CN | F |
| A-549. | Ethoxy | H | H | Methyl | CN | F |
| A-550. | OCF₃ | H | H | Methyl | CN | F |
| A-551. | H | H | Methoxy | Methyl | CN | F |
| A-552. | Methoxy | H | Methoxy | Methyl | CN | F |
| A-553. | Ethoxy | H | Methoxy | Methyl | CN | F |
| A-554. | OCF₃ | H | Methoxy | Methyl | CN | F |
| A-555. | H | H | Ethoxy | Methyl | CN | F |
| A-556. | Methoxy | H | Ethoxy | Methyl | CN | F |
| A-557. | Ethoxy | H | Ethoxy | Methyl | CN | F |
| A-558. | OCF₃ | H | Ethoxy | Methyl | CN | F |
| A-559. | H | H | OCH₂F | Methyl | CN | F |
| A-560. | H | H | OCHF₂ | Methyl | CN | F |
| A-561. | H | H | OCF₃ | Methyl | CN | F |
| A-562. | H | H | OCH₂CH₂F | Methyl | CN | F |
| A-563. | H | H | OCH₂CHF₂ | Methyl | CN | F |
| A-564. | H | H | OCH₂CF₃ | Methyl | CN | F |
| A-565. | H | H | Cl | Methyl | CN | F |
| A-566. | H | H | CN | Methyl | CN | F |
| A-567. | H | Methoxy | H | Methyl | CN | F |
| A-568. | H | H | H | Ethyl | CN | F |
| A-569. | Methoxy | H | H | Ethyl | CN | F |
| A-570. | Ethoxy | H | H | Ethyl | CN | F |
| A-571. | OCF₃ | H | H | Ethyl | CN | F |
| A-572. | H | H | Methoxy | Ethyl | CN | F |
| A-573. | Methoxy | H | Methoxy | Ethyl | CN | F |
| A-574. | Ethoxy | H | Methoxy | Ethyl | CN | F |
| A-575. | OCF₃ | H | Methoxy | Ethyl | CN | F |
| A-576. | H | H | Ethoxy | Ethyl | CN | F |
| A-577. | Methoxy | H | Ethoxy | Ethyl | CN | F |
| A-578. | Ethoxy | H | Ethoxy | Ethyl | CN | F |
| A-579. | OCF₃ | H | Ethoxy | Ethyl | CN | F |
| A-580. | H | H | OCH₂F | Ethyl | CN | F |
| A-581. | H | H | OCHF₂ | Ethyl | CN | F |
| A-582. | H | H | OCF₃ | Ethyl | CN | F |
| A-583. | H | H | OCH₂CH₂F | Ethyl | CN | F |
| A-584. | H | H | OCH₂CHF₂ | Ethyl | CN | F |
| A-585. | H | H | OCH₂CF₃ | Ethyl | CN | F |
| A-586. | H | H | Cl | Ethyl | CN | F |
| A-587. | H | H | CN | Ethyl | CN | F |
| A-588. | H | Methoxy | H | Ethyl | CN | F |
| A-589. | H | H | H | n-Propyl | CN | F |
| A-590. | Methoxy | H | H | n-Propyl | CN | F |
| A-591. | Ethoxy | H | H | n-Propyl | CN | F |
| A-592. | OCF₃ | H | H | n-Propyl | CN | F |
| A-593. | H | H | Methoxy | n-Propyl | CN | F |
| A-594. | Methoxy | H | Methoxy | n-Propyl | CN | F |
| A-595. | Ethoxy | H | Methoxy | n-Propyl | CN | F |
| A-596. | OCF₃ | H | Methoxy | n-Propyl | CN | F |
| A-597. | H | H | Ethoxy | n-Propyl | CN | F |
| A-598. | Methoxy | H | Ethoxy | n-Propyl | CN | F |
| A-599. | Ethoxy | H | Ethoxy | n-Propyl | CN | F |
| A-600. | OCF₃ | H | Ethoxy | n-Propyl | CN | F |
| A-601. | H | H | OCH₂F | n-Propyl | CN | F |
| A-602. | H | H | OCHF₂ | n-Propyl | CN | F |
| A-603. | H | H | OCF₃ | n-Propyl | CN | F |
| A-604. | H | H | OCH₂CH₂F | n-Propyl | CN | F |
| A-605. | H | H | OCH₂CHF₂ | n-Propyl | CN | F |
| A-606. | H | H | OCH₂CF₃ | n-Propyl | CN | F |
| A-607. | H | H | Cl | n-Propyl | CN | F |
| A-608. | H | H | CN | n-Propyl | CN | F |
| A-609. | H | Methoxy | H | n-Propyl | CN | F |
| A-610. | H | H | H | Isopropyl | CN | F |
| A-611. | Methoxy | H | H | Isopropyl | CN | F |
| A-612. | Ethoxy | H | H | Isopropyl | CN | F |
| A-613. | OCF₃ | H | H | Isopropyl | CN | F |
| A-614. | H | H | Methoxy | Isopropyl | CN | F |
| A-615. | Methoxy | H | Methoxy | Isopropyl | CN | F |
| A-616. | Ethoxy | H | Methoxy | Isopropyl | CN | F |
| A-617. | OCF₃ | H | Methoxy | Isopropyl | CN | F |
| A-618. | H | H | Ethoxy | Isopropyl | CN | F |
| A-619. | Methoxy | H | Ethoxy | Isopropyl | CN | F |
| A-620. | Ethoxy | H | Ethoxy | Isopropyl | CN | F |
| A-621. | OCF₃ | H | Ethoxy | Isopropyl | CN | F |
| A-622. | H | H | OCH₂F | Isopropyl | CN | F |
| A-623. | H | H | OCHF₂ | Isopropyl | CN | F |
| A-624. | H | H | OCF₃ | Isopropyl | CN | F |
| A-625. | H | H | OCH₂CH₂F | Isopropyl | CN | F |
| A-626. | H | H | OCH₂CHF₂ | Isopropyl | CN | F |
| A-627. | H | H | OCH₂CF₃ | Isopropyl | CN | F |
| A-628. | H | H | Cl | Isopropyl | CN | F |
| A-629. | H | H | CN | Isopropyl | CN | F |
| A-630. | H | Methoxy | H | Isopropyl | CN | F |
| A-631. | H | H | H | H | Cl | F |
| A-632. | Methoxy | H | H | H | Cl | F |
| A-633. | Ethoxy | H | H | H | Cl | F |
| A-634. | OCF₃ | H | H | H | Cl | F |
| A-635. | H | H | Methoxy | H | Cl | F |
| A-636. | Methoxy | H | Methoxy | H | Cl | F |
| A-637. | Ethoxy | H | Methoxy | H | Cl | F |
| A-638. | OCF₃ | H | Methoxy | H | Cl | F |
| A-639. | H | H | Ethoxy | H | Cl | F |
| A-640. | Methoxy | H | Ethoxy | H | Cl | F |
| A-641. | Ethoxy | H | Ethoxy | H | Cl | F |
| A-642. | OCF₃ | H | Ethoxy | H | Cl | F |
| A-643. | H | H | OCH₂F | H | Cl | F |
| A-644. | H | H | OCHF₂ | H | Cl | F |
| A-645. | H | H | OCF₃ | H | Cl | F |
| A-646. | H | H | OCH₂CH₂F | H | Cl | F |
| A-647. | H | H | OCH₂CHF₂ | H | Cl | F |
| A-648. | H | H | OCH₂CF₃ | H | Cl | F |
| A-649. | H | H | Cl | H | Cl | F |
| A-650. | H | H | CN | H | Cl | F |
| A-651. | H | Methoxy | H | H | Cl | F |
| A-652. | H | H | H | Methyl | Cl | F |
| A-653. | Methoxy | H | H | Methyl | Cl | F |
| A-654. | Ethoxy | H | H | Methyl | Cl | F |
| A-655. | OCF₃ | H | H | Methyl | Cl | F |
| A-656. | H | H | Methoxy | Methyl | Cl | F |
| A-657. | Methoxy | H | Methoxy | Methyl | Cl | F |
| A-658. | Ethoxy | H | Methoxy | Methyl | Cl | F |
| A-659. | OCF₃ | H | Methoxy | Methyl | Cl | F |
| A-660. | H | H | Ethoxy | Methyl | Cl | F |
| A-661. | Methoxy | H | Ethoxy | Methyl | Cl | F |
| A-662. | Ethoxy | H | Ethoxy | Methyl | Cl | F |
| A-663. | OCF₃ | H | Ethoxy | Methyl | Cl | F |
| A-664. | H | H | OCH₂F | Methyl | Cl | F |
| A-665. | H | H | OCHF₂ | Methyl | Cl | F |
| A-666. | H | H | OCF₃ | Methyl | Cl | F |
| A-667. | H | H | OCH₂CH₂F | Methyl | Cl | F |
| A-668. | H | H | OCH₂CHF₂ | Methyl | Cl | F |
| A-669. | H | H | OCH₂CF₃ | Methyl | Cl | F |
| A-670. | H | H | Cl | Methyl | Cl | F |
| A-671. | H | H | CN | Methyl | Cl | F |
| A-672. | H | Methoxy | H | Methyl | Cl | F |
| A-673. | H | H | H | Ethyl | Cl | F |
| A-674. | Methoxy | H | H | Ethyl | Cl | F |
| A-675. | Ethoxy | H | H | Ethyl | Cl | F |
| A-676. | OCF₃ | H | H | Ethyl | Cl | F |
| A-677. | H | H | Methoxy | Ethyl | Cl | F |
| A-678. | Methoxy | H | Methoxy | Ethyl | Cl | F |
| A-679. | Ethoxy | H | Methoxy | Ethyl | Cl | F |
| A-680. | OCF₃ | H | Methoxy | Ethyl | Cl | F |
| A-681. | H | H | Ethoxy | Ethyl | Cl | F |
| A-682. | Methoxy | H | Ethoxy | Ethyl | Cl | F |
| A-683. | Ethoxy | H | Ethoxy | Ethyl | Cl | F |
| A-684. | OCF₃ | H | Ethoxy | Ethyl | Cl | F |
| A-685. | H | H | OCH₂F | Ethyl | Cl | F |
| A-686. | H | H | OCHF₂ | Ethyl | Cl | F |
| A-687. | H | H | OCF₃ | Ethyl | Cl | F |
| A-688. | H | H | OCH₂CH₂F | Ethyl | Cl | F |
| A-689. | H | H | OCH₂CHF₂ | Ethyl | Cl | F |
| A-690. | H | H | OCH₂CF₃ | Ethyl | Cl | F |
| A-691. | H | H | Cl | Ethyl | Cl | F |
| A-692. | H | H | CN | Ethyl | Cl | F |
| A-693. | H | Methoxy | H | Ethyl | Cl | F |
| A-694. | H | H | H | n-Propyl | Cl | F |
| A-695. | Methoxy | H | H | n-Propyl | Cl | F |
| A-696. | Ethoxy | H | H | n-Propyl | Cl | F |
| A-697. | OCF₃ | H | H | n-Propyl | Cl | F |
| A-698. | H | H | Methoxy | n-Propyl | Cl | F |
| A-699. | Methoxy | H | Methoxy | n-Propyl | Cl | F |
| A-700. | Ethoxy | H | Methoxy | n-Propyl | Cl | F |
| A-701. | OCF₃ | H | Methoxy | n-Propyl | Cl | F |
| A-702. | H | H | Ethoxy | n-Propyl | Cl | F |
| A-703. | Methoxy | H | Ethoxy | n-Propyl | Cl | F |
| A-704. | Ethoxy | H | Ethoxy | n-Propyl | Cl | F |
| A-705. | OCF₃ | H | Ethoxy | n-Propyl | Cl | F |
| A-706. | H | H | OCH₂F | n-Propyl | Cl | F |
| A-707. | H | H | OCHF₂ | n-Propyl | Cl | F |
| A-708. | H | H | OCF₃ | n-Propyl | Cl | F |
| A-709. | H | H | OCH₂CH₂F | n-Propyl | Cl | F |
| A-710. | H | H | OCH₂CHF₂ | n-Propyl | Cl | F |
| A-711. | H | H | OCH₂CF₃ | n-Propyl | Cl | F |
| A-712. | H | H | Cl | n-Propyl | Cl | F |
| A-713. | H | H | CN | n-Propyl | Cl | F |
| A-714. | H | Methoxy | H | n-Propyl | Cl | F |
| A-715. | H | H | H | Isopropyl | Cl | F |
| A-716. | Methoxy | H | H | Isopropyl | Cl | F |
| A-717. | Ethoxy | H | H | Isopropyl | Cl | F |
| A-718. | OCF₃ | H | H | Isopropyl | Cl | F |
| A-719. | H | H | Methoxy | Isopropyl | Cl | F |
| A-720. | Methoxy | H | Methoxy | Isopropyl | Cl | F |
| A-721. | Ethoxy | H | Methoxy | Isopropyl | Cl | F |
| A-722. | OCF₃ | H | Methoxy | Isopropyl | Cl | F |
| A-723. | H | H | Ethoxy | Isopropyl | Cl | F |
| A-724. | Methoxy | H | Ethoxy | Isopropyl | Cl | F |
| A-725. | Ethoxy | H | Ethoxy | Isopropyl | Cl | F |
| A-726. | OCF₃ | H | Ethoxy | Isopropyl | Cl | F |
| A-727. | H | H | OCH₂F | Isopropyl | Cl | F |
| A-728. | H | H | OCHF₂ | Isopropyl | Cl | F |
| A-729. | H | H | OCF₃ | Isopropyl | Cl | F |
| A-730. | H | H | OCH₂CH₂F | Isopropyl | Cl | F |
| A-731. | H | H | OCH₂CHF₂ | Isopropyl | Cl | F |
| A-732. | H | H | OCH₂CF₃ | Isopropyl | Cl | F |
| A-733. | H | H | Cl | Isopropyl | Cl | F |
| A-734. | H | H | CN | Isopropyl | Cl | F |
| A-735. | H | Methoxy | H | Isopropyl | Cl | F |
| A-736. | H | H | H | H | F | F |
| A-737. | Methoxy | H | H | H | F | F |
| A-738. | Ethoxy | H | H | H | F | F |
| A-739. | OCF₃ | H | H | H | F | F |
| A-740. | H | H | Methoxy | H | F | F |
| A-741. | Methoxy | H | Methoxy | H | F | F |
| A-742. | Ethoxy | H | Methoxy | H | F | F |
| A-743. | OCF₃ | H | Methoxy | H | F | F |
| A-744. | H | H | Ethoxy | H | F | F |
| A-745. | Methoxy | H | Ethoxy | H | F | F |
| A-746. | Ethoxy | H | Ethoxy | H | F | F |
| A-747. | OCF₃ | H | Ethoxy | H | F | F |
| A-748. | H | H | OCH₂F | H | F | F |
| A-749. | H | H | OCHF₂ | H | F | F |
| A-750. | H | H | OCF₃ | H | F | F |
| A-751. | H | H | OCH₂CH₂F | H | F | F |
| A-752. | H | H | OCH₂CHF₂ | H | F | F |
| A-753. | H | H | OCH₂CF₃ | H | F | F |
| A-754. | H | H | Cl | H | F | F |
| A-755. | H | H | CN | H | F | F |
| A-756. | H | Methoxy | H | H | F | F |
| A-757. | H | H | H | Methyl | F | F |
| A-758. | Methoxy | H | H | Methyl | F | F |
| A-759. | Ethoxy | H | H | Methyl | F | F |
| A-760. | OCF₃ | H | H | Methyl | F | F |
| A-761. | H | H | Methoxy | Methyl | F | F |
| A-762. | Methoxy | H | Methoxy | Methyl | F | F |
| A-763. | Ethoxy | H | Methoxy | Methyl | F | F |
| A-764. | OCF₃ | H | Methoxy | Methyl | F | F |
| A-765. | H | H | Ethoxy | Methyl | F | F |
| A-766. | Methoxy | H | Ethoxy | Methyl | F | F |
| A-767. | Ethoxy | H | Ethoxy | Methyl | F | F |
| A-768. | OCF₃ | H | Ethoxy | Methyl | F | F |
| A-769. | H | H | OCH₂F | Methyl | F | F |
| A-770. | H | H | OCHF₂ | Methyl | F | F |
| A-771. | H | H | OCF₃ | Methyl | F | F |
| A-772. | H | H | OCH₂CH₂F | Methyl | F | F |
| A-773. | H | H | OCH₂CHF₂ | Methyl | F | F |
| A-774. | H | H | OCH₂CF₃ | Methyl | F | F |
| A-775. | H | H | Cl | Methyl | F | F |
| A-776. | H | H | CN | Methyl | F | F |
| A-777. | H | Methoxy | H | Methyl | F | F |
| A-778. | H | H | H | Ethyl | F | F |
| A-779. | Methoxy | H | H | Ethyl | F | F |
| A-780. | Ethoxy | H | H | Ethyl | F | F |
| A-781. | OCF₃ | H | H | Ethyl | F | F |
| A-782. | H | H | Methoxy | Ethyl | F | F |
| A-783. | Methoxy | H | Methoxy | Ethyl | F | F |
| A-784. | Ethoxy | H | Methoxy | Ethyl | F | F |
| A-785. | OCF₃ | H | Methoxy | Ethyl | F | F |
| A-786. | H | H | Ethoxy | Ethyl | F | F |
| A-787. | Methoxy | H | Ethoxy | Ethyl | F | F |
| A-788. | Ethoxy | H | Ethoxy | Ethyl | F | F |
| A-789. | OCF₃ | H | Ethoxy | Ethyl | F | F |
| A-790. | H | H | OCH₂F | Ethyl | F | F |
| A-791. | H | H | OCHF₂ | Ethyl | F | F |
| A-792. | H | H | OCF₃ | Ethyl | F | F |
| A-793. | H | H | OCH₂CH₂F | Ethyl | F | F |
| A-794. | H | H | OCH₂CHF₂ | Ethyl | F | F |
| A-795. | H | H | OCH₂CF₃ | Ethyl | F | F |
| A-796. | H | H | Cl | Ethyl | F | F |
| A-797. | H | H | CN | Ethyl | F | F |
| A-798. | H | Methoxy | H | Ethyl | F | F |
| A-799. | H | H | H | n-Propyl | F | F |
| A-800. | Methoxy | H | H | n-Propyl | F | F |
| A-801. | Ethoxy | H | H | n-Propyl | F | F |
| A-802. | OCF₃ | H | H | n-Propyl | F | F |
| A-803. | H | H | Methoxy | n-Propyl | F | F |
| A-804. | Methoxy | H | Methoxy | n-Propyl | F | F |
| A-805. | Ethoxy | H | Methoxy | n-Propyl | F | F |
| A-806. | OCF₃ | H | Methoxy | n-Propyl | F | F |
| A-807. | H | H | Ethoxy | n-Propyl | F | F |
| A-808. | Methoxy | H | Ethoxy | n-Propyl | F | F |
| A-809. | Ethoxy | H | Ethoxy | n-Propyl | F | F |
| A-810. | OCF₃ | H | Ethoxy | n-Propyl | F | F |
| A-811. | H | H | OCH₂F | n-Propyl | F | F |
| A-812. | H | H | OCHF₂ | n-Propyl | F | F |
| A-813. | H | H | OCF₃ | n-Propyl | F | F |
| A-814. | H | H | OCH₂CH₂F | n-Propyl | F | F |
| A-815. | H | H | OCH₂CHF₂ | n-Propyl | F | F |
| A-816. | H | H | OCH₂CF₃ | n-Propyl | F | F |
| A-817. | H | H | Cl | n-Propyl | F | F |
| A-818. | H | H | CN | n-Propyl | F | F |
| A-819. | H | Methoxy | H | n-Propyl | F | F |
| A-820. | H | H | H | Isopropyl | F | F |
| A-821. | Methoxy | H | H | Isopropyl | F | F |
| A-822. | Ethoxy | H | H | Isopropyl | F | F |
| A-823. | OCF₃ | H | H | Isopropyl | F | F |
| A-824. | H | H | Methoxy | Isopropyl | F | F |
| A-825. | Methoxy | H | Methoxy | Isopropyl | F | F |
| A-826. | Ethoxy | H | Methoxy | Isopropyl | F | F |
| A-827. | OCF₃ | H | Methoxy | Isopropyl | F | F |
| A-828. | H | H | Ethoxy | Isopropyl | F | F |
| A-829. | Methoxy | H | Ethoxy | Isopropyl | F | F |
| A-830. | Ethoxy | H | Ethoxy | Isopropyl | F | F |
| A-831. | OCF₃ | H | Ethoxy | Isopropyl | F | F |
| A-832. | H | H | OCH₂F | Isopropyl | F | F |
| A-833. | H | H | OCHF₂ | Isopropyl | F | F |
| A-834. | H | H | OCF₃ | Isopropyl | F | F |
| A-835. | H | H | OCH₂CH₂F | Isopropyl | F | F |
| A-836. | H | H | OCH₂CHF₂ | Isopropyl | F | F |
| A-837. | H | H | OCH₂CF₃ | Isopropyl | F | F |
| A-838. | H | H | Cl | Isopropyl | F | F |
| A-839. | H | H | CN | Isopropyl | F | F |
| A-840. | H | Methoxy | H | Isopropyl | F | F |
| A-841. | H | H | H | H | F | Cl |
| A-842. | Methoxy | H | H | H | F | Cl |
| A-843. | Ethoxy | H | H | H | F | Cl |
| A-844. | OCF₃ | H | H | H | F | Cl |
| A-845. | H | H | Methoxy | H | F | Cl |
| A-846. | Methoxy | H | Methoxy | H | F | Cl |
| A-847. | Ethoxy | H | Methoxy | H | F | Cl |
| A-848. | OCF₃ | H | Methoxy | H | F | Cl |
| A-849. | H | H | Ethoxy | H | F | Cl |
| A-850. | Methoxy | H | Ethoxy | H | F | Cl |
| A-851. | Ethoxy | H | Ethoxy | H | F | Cl |
| A-852. | OCF₃ | H | Ethoxy | H | F | Cl |
| A-853. | H | H | OCH₂F | H | F | Cl |
| A-854. | H | H | OCHF₂ | H | F | Cl |
| A-855. | H | H | OCF₃ | H | F | Cl |
| A-856. | H | H | OCH₂CH₂F | H | F | Cl |
| A-857. | H | H | OCH₂CHF₂ | H | F | Cl |
| A-858. | H | H | OCH₂CF₃ | H | F | Cl |
| A-859. | H | H | Cl | H | F | Cl |
| A-860. | H | H | CN | H | F | Cl |
| A-861. | H | Methoxy | H | H | F | Cl |
| A-862. | H | H | H | Methyl | F | Cl |
| A-863. | Methoxy | H | H | Methyl | F | Cl |
| A-864. | Ethoxy | H | H | Methyl | F | Cl |
| A-865. | OCF₃ | H | H | Methyl | F | Cl |
| A-866. | H | H | Methoxy | Methyl | F | Cl |
| A-867. | Methoxy | H | Methoxy | Methyl | F | Cl |
| A-868. | Ethoxy | H | Methoxy | Methyl | F | Cl |
| A-869. | OCF₃ | H | Methoxy | Methyl | F | Cl |
| A-870. | H | H | Ethoxy | Methyl | F | Cl |
| A-871. | Methoxy | H | Ethoxy | Methyl | F | Cl |
| A-872. | Ethoxy | H | Ethoxy | Methyl | F | Cl |
| A-873. | OCF₃ | H | Ethoxy | Methyl | F | Cl |
| A-874. | H | H | OCH₂F | Methyl | F | Cl |
| A-875. | H | H | OCHF₂ | Methyl | F | Cl |
| A-876. | H | H | OCF₃ | Methyl | F | Cl |
| A-877. | H | H | OCH₂CH₂F | Methyl | F | Cl |
| A-878. | H | H | OCH₂CHF₂ | Methyl | F | Cl |
| A-879. | H | H | OCH₂CF₃ | Methyl | F | Cl |
| A-880. | H | H | Cl | Methyl | F | Cl |
| A-881. | H | H | CN | Methyl | F | Cl |
| A-882. | H | Methoxy | H | Methyl | F | Cl |
| A-883. | H | H | H | Ethyl | F | Cl |
| A-884. | Methoxy | H | H | Ethyl | F | Cl |
| A-885. | Ethoxy | H | H | Ethyl | F | Cl |
| A-886. | OCF₃ | H | H | Ethyl | F | Cl |
| A-887. | H | H | Methoxy | Ethyl | F | Cl |
| A-888. | Methoxy | H | Methoxy | Ethyl | F | Cl |
| A-889. | Ethoxy | H | Methoxy | Ethyl | F | Cl |
| A-890. | OCF₃ | H | Methoxy | Ethyl | F | Cl |
| A-891. | H | H | Ethoxy | Ethyl | F | Cl |
| A-892. | Methoxy | H | Ethoxy | Ethyl | F | Cl |
| A-893. | Ethoxy | H | Ethoxy | Ethyl | F | Cl |
| A-894. | OCF₃ | H | Ethoxy | Ethyl | F | Cl |
| A-895. | H | H | OCH₂F | Ethyl | F | Cl |
| A-896. | H | H | OCHF₂ | Ethyl | F | Cl |
| A-897. | H | H | OCF₃ | Ethyl | F | Cl |
| A-898. | H | H | OCH₂CH₂F | Ethyl | F | Cl |
| A-899. | H | H | OCH₂CHF₂ | Ethyl | F | Cl |
| A-900. | H | H | OCH₂CF₃ | Ethyl | F | Cl |
| A-901. | H | H | Cl | Ethyl | F | Cl |
| A-902. | H | H | CN | Ethyl | F | Cl |
| A-903. | H | Methoxy | H | Ethyl | F | Cl |
| A-904. | H | H | H | n-Propyl | F | Cl |
| A-905. | Methoxy | H | H | n-Propyl | F | Cl |
| A-906. | Ethoxy | H | H | n-Propyl | F | Cl |
| A-907. | OCF₃ | H | H | n-Propyl | F | Cl |
| A-908. | H | H | Methoxy | n-Propyl | F | Cl |
| A-909. | Methoxy | H | Methoxy | n-Propyl | F | CI |
| A-910. | Ethoxy | H | Methoxy | n-Propyl | F | Cl |
| A-911. | OCF₃ | H | Methoxy | n-Propyl | F | Cl |
| A-912. | H | H | Ethoxy | n-Propyl | F | Cl |
| A-913. | Methoxy | H | Ethoxy | n-Propyl | F | Cl |
| A-914. | Ethoxy | H | Ethoxy | n-Propyl | F | Cl |
| A-915. | OCF₃ | H | Ethoxy | n-Propyl | F | Cl |
| A-916. | H | H | OCH₂F | n-Propyl | F | Cl |
| A-917. | H | H | OCHF₂ | n-Propyl | F | Cl |
| A-918. | H | H | OCF₃ | n-Propyl | F | Cl |
| A-919. | H | H | OCH₂CH₂F | n-Propyl | F | Cl |
| A-920. | H | H | OCH₂CHF₂ | n-Propyl | F | Cl |
| A-921. | H | H | OCH₂CF₃ | n-Propyl | F | Cl |
| A-922. | H | H | Cl | n-Propyl | F | Cl |
| A-923. | H | H | CN | n-Propyl | F | Cl |
| A-924. | H | Methoxy | H | n-Propyl | F | Cl |
| A-925. | H | H | H | Isopropyl | F | Cl |
| A-926. | Methoxy | H | H | Isopropyl | F | Cl |
| A-927. | Ethoxy | H | H | Isopropyl | F | Cl |
| A-928. | OCF₃ | H | H | Isopropyl | F | Cl |
| A-929. | H | H | Methoxy | Isopropyl | F | Cl |
| A-930. | Methoxy | H | Methoxy | Isopropyl | F | Cl |
| A-931. | Ethoxy | H | Methoxy | Isopropyl | F | Cl |
| A-932. | OCF₃ | H | Methoxy | Isopropyl | F | Cl |
| A-933. | H | H | Ethoxy | Isopropyl | F | Cl |
| A-934. | Methoxy | H | Ethoxy | Isopropyl | F | Cl |
| A-935. | Ethoxy | H | Ethoxy | Isopropyl | F | Cl |
| A-936. | OCF₃ | H | Ethoxy | Isopropyl | F | Cl |
| A-937. | H | H | OCH₂F | Isopropyl | F | Cl |
| A-938. | H | H | OCHF₂ | Isopropyl | F | Cl |
| A-939. | H | H | OCF₃ | Isopropyl | F | Cl |
| A-940. | H | H | OCH₂CH₂F | Isopropyl | F | Cl |
| A-941. | H | H | OCH₂CHF₂ | Isopropyl | F | Cl |
| A-942. | H | H | OCH₂CF₃ | Isopropyl | F | Cl |
| A-943. | H | H | Cl | Isopropyl | F | Cl |
| A-944. | H | H | CN | Isopropyl | F | Cl |
| A-945. | H | Methoxy | H | Isopropyl | F | Cl |

Unter den oben genannten Verbindungen 1.1 bis 1.56 sind Verbindungen der Formel I.1 am stärksten bevorzugt.

Die erfindungsgemäßen Verbindungen I weisen in der 3-Position des 2-Oxindolrings ein Chiralitätszentrum auf. Die erfindungsgemäßen Verbindungen können daher als ein 1:1-Gemisch von Enantiomeren (Racemat) oder als ein nicht-racemisches Gemisch von Enantiomeren, in dem eines der beiden Enantiomere, entweder das die Schwingungsebene von linear polarisiertem Licht nach links drehende (d.h. minusdrehende) Enantiomer (im Folgenden (-)-Enantiomer) oder das die Schwingungsebene von linear polarisiertem Licht nach rechts drehende (d.h. plusdrehende) Enantiomer (im Folgenden (+)-Enantiomer), angereichert ist, oder als im Wesentlichen enantiomerenreine Verbindungen, also als im Wesentlichen enantiomerenreines (-)-Enantiomer oder (+)-Enantiomer, vorliegen. Da bei den erfindungsgemäßen Verbindungen meist ein einziges Asymmetriezentrum und keine Chiralitätsachse/-ebene existiert, kann man ein nicht-racemisches Gemisch in diesen Fällen auch als ein Gemisch von Enantiomeren definieren, in welchem entweder das R- oder das S-Enantiomer überwiegt. Im Wesentlichen enantiomerenreine Verbindungen können in diesen Fällen dementsprechend auch als im Wesentlichen enantiomerenreines R-Enantiomer bzw. im Wesentlichen enantiomerenreines S-Enantiomer definiert werden.

Unter "im Wesentlichen enantiomerenreinen Verbindungen" versteht man im Rahmen der vorliegenden Erfindung solche Verbindungen, die einen Enantiomerenüberschuss (enantiomeric excess, ee; % ee = (R-S)/(R+S) x 100 bzw. (S-R)/(S+R) x 100) von wenigstens 80 % ee, vorzugsweise wenigsten 85 % ee, stärker bevorzugt wenigstens 90 % ee, noch stärker bevorzugt wenigstens 95 % ee und insbesondere wenigstens 98 % ee aufweisen.

In einer Ausführungsform der Erfindung liegen die erfindungsgemäßen Verbindungen als im Wesentlichen enantiomerenreine Verbindungen vor. Besonders bevorzugt sind Verbindungen, die einen Enantiomerenüberschuss von wenigstens 85 % ee, stärker bevorzugt von wenigstens 90 % ee, noch stärker bevorzugt von wenigstens 95 % ee und insbesondere von wenigstens 98 % ee aufweisen.

Gegenstand der Erfindung sind somit sowohl die reinen Enantiomere als auch deren Gemische, z.B. Gemische, in denen ein Enantiomer in angereichter Form vorliegt, aber auch die Racemate. Gegenstand der Erfindung sind auch die pharmazeutisch verträglichen Salze der reinen Enantiomere von Verbindungen I sowie die Enantiomerengemische in Form der pharmazeutisch verträglichen Salze von Verbindungen I.

Im Folgenden werden beispielhafte Synthesewege zur Herstellung der erfindungsgemäßen Oxindol-Derivate beschrieben.

Die Herstellung der erfindungsgemäßen Verbindungen kann unter Verwendung der in WO 2005/030755 und WO 2006/005609 beschriebenen Vorschriften zur Synthese analoger Verbindungen erfolgen und ist beispielhaft in den Syntheseschemata 1 bis 5 skizziert. In diesen Syntheseschemata besitzen die Variablen die gleichen Bedeutungen wie in der Formel I.

Die 3-Hydroxy-1,3-dihydroindol-2-one IV können durch Addition von metallierten Benzolen oder Heterocyclen III an die 3-Ketogruppe der Isatine II erhalten werden. Die metallierten Benzole oder Heterocyclen, wie beispielsweise die entsprechenden Grignard- (Mg) oder Organyllithium-Verbindung, können in üblicher Weise aus Halogen- oder Kohlenwasserstoffverbindungen erhalten werden. Beispielhafte Vorschriften sind im Houben-Weyl, Methoden der Organischen Chemie, Bd. 13, 1-2, Kap. Mg- bzw. Li-Verbindungen, enthalten. Die Isatine II sind entweder kommerziell erhältlich oder wurden in Analogie zu in der Literatur beschriebenen Methoden hergestellt (Advances in Heterocyclic Chemistry, A.R. Katritzky and A.J. Boulton, Academic Press, New York, 1975, 18, 2-58; J. Brazil. Chem. Soc. 12, 273-324, 2001 ).

Die 3-Hydroxyoxindole IV, die im 6-Ring-Aromaten beispielsweise in der 5- und/oder 6-Position als Rest R⁷ oder R⁸ ein lod oder Brom enthalten, können mit KCN oder Zn(CN₎2 unter Pd(0)-Katalyse in Lösungsmitteln wie Dimethylformamid oder Tetrahydrofuran, gegebenenfalls auch unter Zusatz von Basen wie K₂CO₃ oder anderen Carbonaten oder Aminen, bei höherer Temperatur in das analoge cyanhaltige 3-Hydroxyoxindol IV überführt werden. Als Pd(0)-Salze kann man zum Beispiel Übergangsmetallkomplexe nehmen, die in situ aus PdCl₂ oder Pd(OAc)₂ durch Zugabe von Phosphinen wie Tris(orthotolyl)phosphin hergestellt werden. Ebenso können kommerzielle Palladium-Komplexe wie beispielsweise der Katalysator Tetrakis(triphenylphosphin)-palladium(0) und / oder Zusätze von Phosphin-Liganden eingesetzt werden.

Die 3-Hydroxyoxindole IV können in die Verbindungen V überführt werden, welche eine Fluchtgruppe LG' in 3-Stellung tragen, wobei die Fluchtgruppe LG' eine übliche Abgangsgruppe ist, wie zum Beispiel Chlorid oder Bromid, aber auch aktiviertes OH, wie Tosylat oder Triflat. Das Zwischenprodukt V mit zum Beispiel LG' = Chlor kann durch Behandlung des Alkohols IV mit Thionylchlorid in Gegenwart einer Base, wie zum Beispiel Pyridin, in einem geeigneten Lösungsmittel, wie zum Beispiel Dichlormethan, hergestellt werden.

Die Verbindungen V können anschließend mit einer Carbonsäure, einem Carboxamid, einem Amin oder einem Alkohol VIII in einer Substitutionsreaktion zu den Aminen VI umgesetzt werden. Die Verbindungen VI können anschließend durch Behandlung mit Sulfonsäurechloriden VII nach Deprotonierung mit einer starken Base, wie zum Beispiel Kalium-*tert*-butylat oder Natriumhydrid in DMF, in das sulfonylierte Produkt I überführt werden. Die eingesetzten Sulfonsäurechloride VII können entweder käuflich erworben oder nach bekannten Verfahren (zum Beispiel J. Med. Chem. 40, 1149 (1997)) hergestellt werden. (Syntheseschema 1)

Die Darstellung der erfindungsgemäßen Verbindungen I mit R⁴ = H kann durch Verwendung entsprechender Boc-geschützter Verbindungen VIII (R⁴ = Boc) erfolgen. Die Boc-Schutzgruppe kann anschließend entfernt werden, zum Beispiel durch Behandlung mit Trifluoressigsäure in Dichlormethan.

Die Verbindungen VIII sind entweder käuflich erwerblich oder können durch übliche Substitutionsreaktionen am (Hetero)Aromaten A, gegebenenfalls unter Anwendung von Schutzgruppentechnik, hergestellt werden.

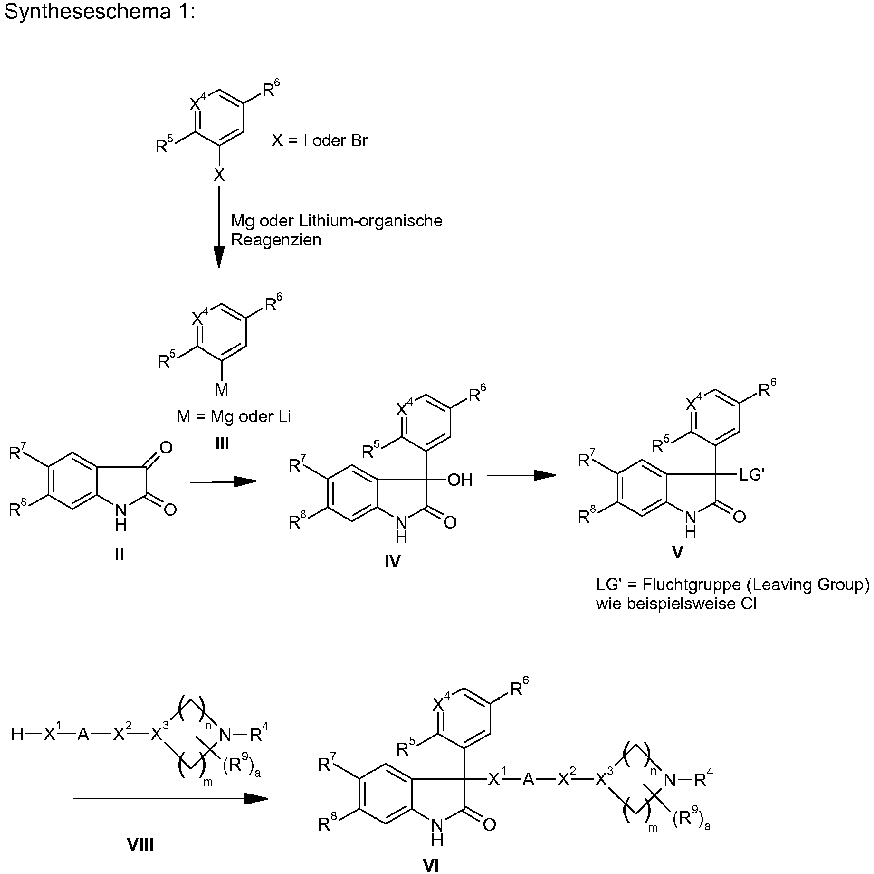

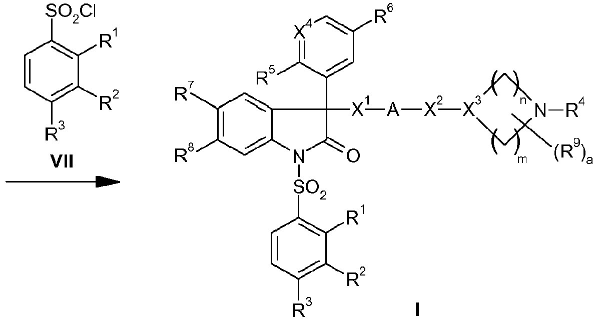

Verbindungen I, in denen X¹ für-NR¹¹-CO-steht, können alternativ dadurch hergestellt werden, dass man die Verbindungen V mit Aminen, wie beispielsweise Ammoniak oder einem Amin NH₂R¹¹, in einer Substitutionsreaktion zu den Aminen IX umsetzt.

Die Verbindungen IX können anschließend durch Behandlung mit Sulfonsäurechloriden VII nach Deprotonierung mit einer starken Base, wie zum Beispiel Kalium-*tert*-butylat oder Natriumhydrid in DMF, in das sulfonylierte Produkt X überführt werden. Die eingesetzten Sulfonsäurechloride VII können entweder käuflich erworben oder nach bekannten Verfahren (zum Beispiel J. Med. Chem. 40, 1149 (1997)) hergestellt werden.

Die anschließende Umsetzung mit Carbonylchloriden XI führt zu den erfindungsgemäßen Verbindungen der allgemeinen Formel (I) mit Carboxamid-Brücke (X¹ = NR¹¹-CO).

Die Darstellung der erfindungsgemäßen Verbindungen I mit R⁴ = H kann durch Verwendung entsprechender Boc-geschützter Verbindungen XI (R⁴ = Boc) erfolgen. Die Boc-Schutzgruppe kann anschließend entfernt werden, zum Beispiel durch Behandlung mit Trifluoressigsäure in Dichlormethan.

Die Verbindungen XI sind entweder käuflich erwerblich oder können durch übliche Substitutionsreaktionen am (Hetero)Aromaten A, gegebenenfalls unter Anwendung von Schutzgruppentechnik, hergestellt werden.

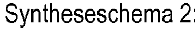

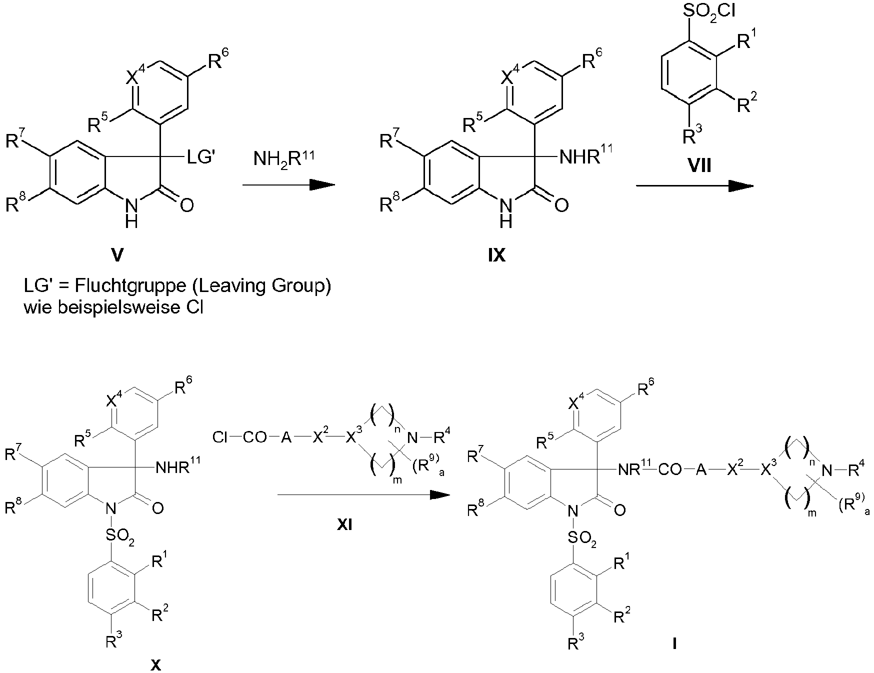

Alternativ kann die Sulfonylierung in Schema 2 auch erst nach der Umsetzung mit der Verbindung XI durchgeführt werden.

Verbindungen der Formel I, worin X² für eine Einfachbindung steht und X³ für N steht, können alternativ auch dadurch hergestellt werden, dass man in den obigen Reaktionsschemata 1 und 2 anstelle der Verbindung VIII (in Schema 1) bzw. anstelle der Verbindung XI (in Schema 2) eine Verbindung XII (Reaktionsschema 3) bzw. eine Verbindung XIII (Reaktionsschema 4) einsetzt, worin R¹² für Halogen, CN, OR^{a} oder NR^{b}R^{c} steht, worin R^{a} für H, C₁-C₄-Alkyl, Phenyl oder Benzyl steht; und R^{b} und R^{c} unabhängig voneinander für H, C₁-C₄-Alkyl, C₁-C₄-Alkoxy, Phenyl oder Benzyl stehen, und vorzugsweise für Halogen, insbesondere Br steht. Die in Schema 3 resultierende Verbindung XIV kann anschließend mit dem cyclischen Amin XV unter Palladium-Katalyse gemäß dem in Tetrahedron Asym. 1999, 10, 1831 beschriebenen Verfahren zur Verbindung XVI gekuppelt werden, welche analog der oben beschriebenen Sulfonylierungsreaktion mit dem Sulfonylchlorid VII in die erfindungsgemäße Verbindung I' überführt werden kann, worin X² für eine Einfachbindung steht und X³ für N steht. Die in Schema 4 resultierende Verbindung XVII kann mit dem cyclischen Amin XV unter Palladium-Katalyse gemäß dem in Tetrahedron Asym. 1999, 10, 1831 beschriebenen Verfahren zur Verbindung I' gekuppelt werden.

Verbindung XIV kann alternativ auch zuerst der Sulfonylierungsreaktion unter Erhalt der Verbindung XVIII unterworfen werden und dann erst mit dem Amin der Formel XV zur Verbindung I umgesetzt werden (Reaktionsschema 5).

Die Darstellung der erfindungsgemäßen Verbindungen I' mit R⁴ = H kann durch Verwendung entsprechender Boc-geschützter Verbindungen XV (R⁴ = Boc) erfolgen. Die Boc-Schutzgruppe kann anschließend entfernt werden, zum Beispiel durch Behandlung mit Trifluoressigsäure in Dichlormethan.

Die Verbindungen XII, XIII und XV sind entweder käuflich erwerblich oder können durch übliche Substitutionsreaktionen am (Hetero)Aromaten A bzw. am N-Heterocyclus, gegebenenfalls unter Anwendung von Schutzgruppentechnik, hergestellt werden.

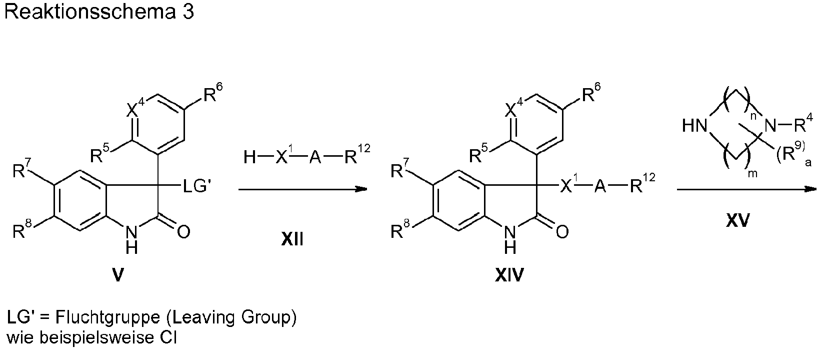

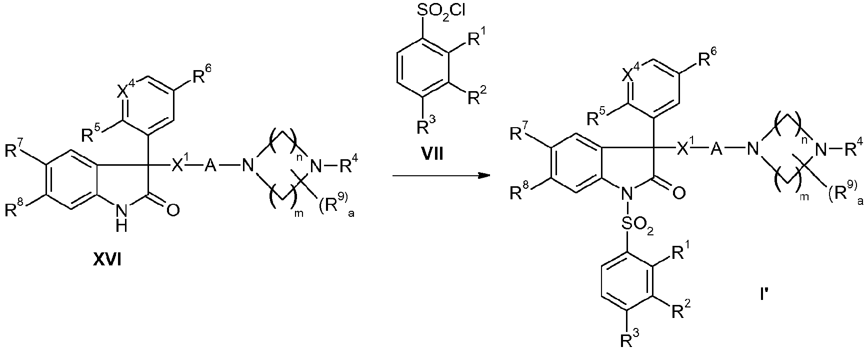

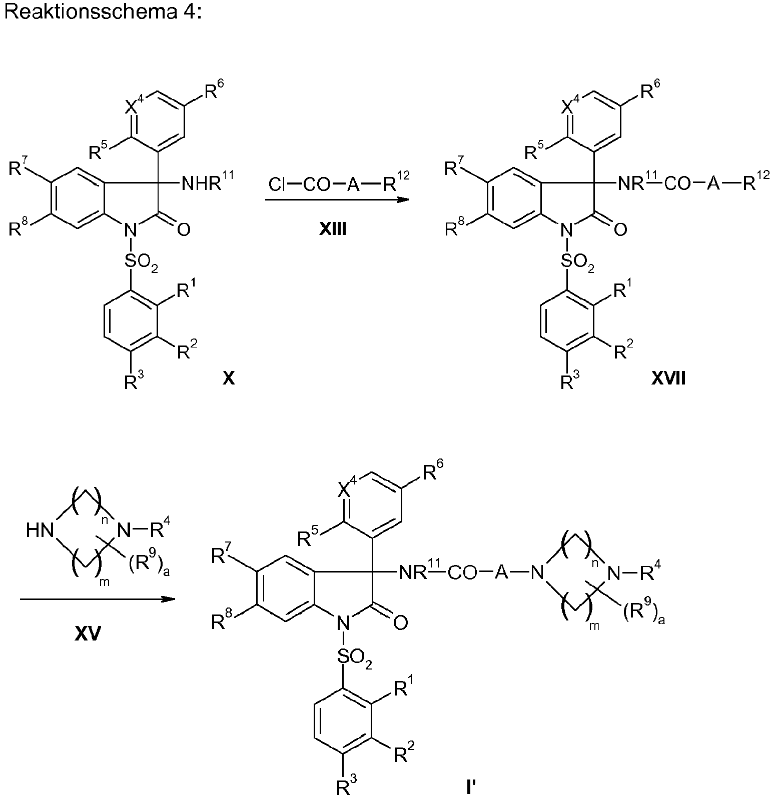

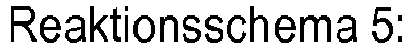

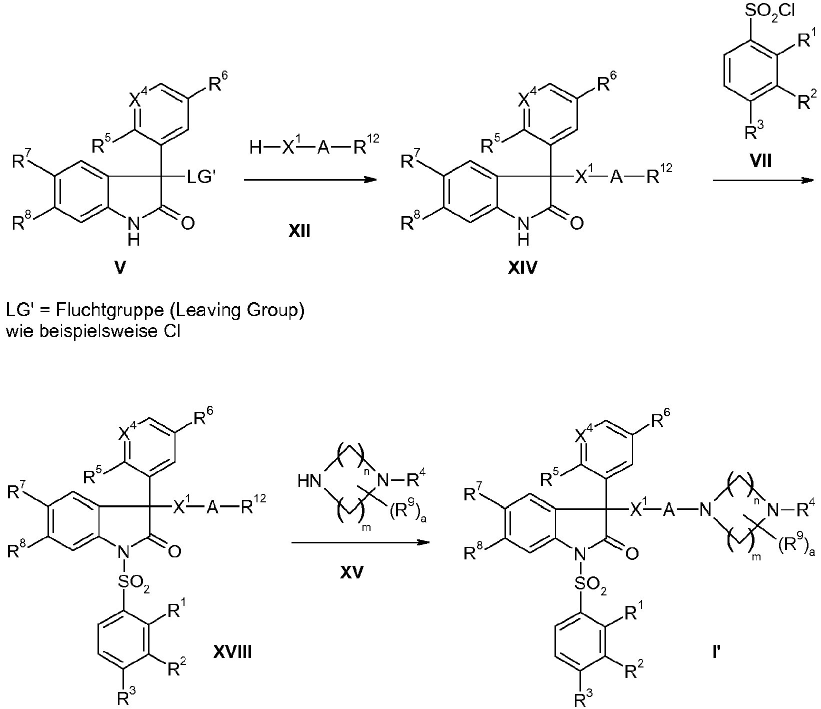

Ein weiterer Gegenstand der vorliegenden Erfindung betrifft Verbindungen der Formel XVIII, die als Zwischenprodukt in der Synthese der erfindungsgemäßen Verbindungen I eingesetzt werden: 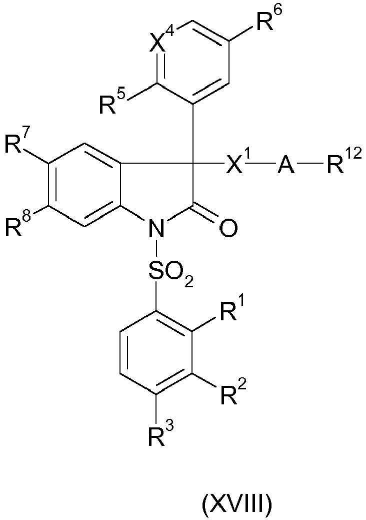 worin R¹, R², R³, R⁵, R⁶, R⁷, R⁸, X¹, X⁴ und A wie vorstehend für Verbindung I definiert sind; und
R¹² für Halogen, CN, OR^{a} oder NR^{b}R^{c} steht, worin
   R^{a} für H, C₁-C₄-Alkyl, Phenyl oder Benzyl steht; und
   R^{b} und R^{c} unabhängig voneinander für H, C₁-C₄-Alkyl, C₁₋C₄-Alkoxy, Phenyl oder Benzyl stehen.

Bezüglich bevorzugter Ausführungsformen der Variablen R¹, R², R³, R⁵, R⁶, R⁷, R⁸, X¹, X⁴ und A wird auf die vorstehenden Ausführungen zu den entsprechenden Variablen in Verbindungen I Bezug genommen.

In der Definition von R¹² steht Halogen vorzugsweise für lod, Brom oder Chlor und insbesondere für Brom.

R¹² steht vorzugsweise für Halogen oder CN und stärker bevorzugt für Halogen. Besonders bevorzugt steht R¹² für lod, Brom oder Chlor und insbesondere für Brom.

Ein weiterer Gegenstand der vorliegenden Erfindung betrifft ein pharmazeutisches Mittel, enthaltend mindestens eine Verbindung der allgemeinen Formel I und/oder ein pharmazeutisch verträgliches Salz davon, wie vorstehend ausgeführt, und einen pharmazeutisch verträglichen Träger. Geeignete Träger hängen unter anderem von der Darreichungsform des Mittels ab und sind dem Fachmann grundsätzlich bekannt. Einige geeignete Träger sind weiter unten beschrieben.

Ein weiterer Gegenstand der vorliegenden Erfindung betrifft die Verwendung von Verbindungen der Formel I und/oder von pharmazeutisch verträglichen Salzen davon zur Herstellung eines Medikaments zur Behandlung und/oder Prophylaxe von Vasopressin-abhängigen Krankheiten.

Vasopressin-abhängige Krankheiten sind solche, bei denen der Krankheitsverlauf zumindest teilweise von Vasopressin abhängt, d.h. Krankheiten, die einen erhöhten Vasopressin-Spiegel zeigen, der unmittelbar oder indirekt zum Krankheitsbild beitragen kann. Anders ausgedrückt sind Vasopressin-abhängige Krankheiten solche, die durch Modulation des Vasopressinrezeptors, beispielsweise durch Gabe eines Vasopressinrezeptorliganden (Agonist, Antagonist, partieller Antagonist/Agonist, inverser Agonist etc.), beeinflusst werden können.

In einer bevorzugten Ausführungsform betrifft die vorliegende Erfindung die Verwendung von erfindungsgemäßen Verbindungen der Formel I oder von pharmazeutisch verträglichen Salzen zur Herstellung eines Medikaments zur Behandlung und/oder Prophylaxe von Krankheiten, die ausgewählt sind unter Diabetes, Insulin-Resistenz, Enuresis nocturna, Inkontinenz und Krankheiten, bei denen Blutgerinnungsstörungen auftreten, und/oder zur Verzögerung der Miktion. Unter dem Begriff "Diabetes" sind alle Diabetesformen zu verstehen, vor allem Diabetes mellitus (einschließlich Typ I und insbesondere Typ II), Diabetes renalis und insbesondere Diabetes insipidus. Bevorzugt handelt es sich bei den Diabetesformen um Diabetes mellitus vom Typ II (mit Insulinresistenz) oder Diabetes insipidus.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung und/oder Prophylaxe von Krankheiten, die ausgewählt sind unter Hypertonie, pulmonaler Hypertonie, Herzinsuffizienz, Myocardinfarkt, koronarem Spasmus, instabiler Angina, PTCA (percutaneous transluminal coronary angioplastie), Ischämien des Herzens, Störungen des renalen Systems, Ödemen, renalem Vasospasmus, Nekrose des renalen Cortex, Hyponatriämie, Hypokaliämie, Schwartz-Bartter Syndrom, Störungen des Gastrointestinaltraktes, gastritischem Vasospasmus, Hepatozirrhose, Magen- und Darmulkus, Emesis, auftretender Emesis bei der Chemotherapie, und Reisekrankheit.

Die erfindungsgemäßen Verbindungen der Formel I bzw. ihre pharmazeutisch verträglichen Salze oder das erfindungsgemäße pharmazeutische Mittel können auch zur Behandlung von verschiedenen Vasopressin-abhängigen Beschwerden, die zentralnervöse Ursachen oder Veränderungen in der HPA-Achse (hypothalamic pituitary adrenal axis) aufweisen, verwendet werden, zum Beispiel bei affektiven Störungen, wie depressiven Störungen und bipolaren Störungen. Dazu gehören zum Beispiel dysthyme Störungen, Phobien, posttraumatische Belastungsstörungen, generelle Angsstörungen, Panikstörungen, saisonale Depressionen und Schlafstörungen.

Ebenso können die erfindungsgemäßen Verbindungen der Formel I bzw. ihre pharmazeutisch verträglichen Salze oder das erfindungsgemäße pharmazeutische Mittel zur Behandlung bei Angststörungen und stressabhängigen Angststörungen eingesetzt werden, wie zum Beispiel generalisierten Angststörungen, Phobien, posttraumatischen Angststörungen, panischen Angststörungen, obsessiv-zwanghaften Angststörungen, akuten stressabhängigen Angststörungen und Sozialphobie.

Weiterhin können die erfindungsgemäßen Verbindungen auch zur Behandlung von Gedächnisleistungsstörungen, Morbus Alzheimer, Psychosen, psychotischen Störungen, Schlafstörungen und/oder dem Cushing Syndrom sowie allen stressabhängigen Krankheiten eingesetzt werden.

Dementsprechend betrifft eine weitere bevorzugte Ausführungsform der vorliegenden Erfindung die Verwendung von erfindungsgemäßen Verbindungen der Formel I oder von pharmazeutisch verträglichen Salzen davon zur Herstellung eines Medikaments zur Behandlung von affektiven Störungen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von Angststörungen und/oder stressabhängigen Angststörungen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von Gedächtnisleistungsstörungen und/oder Morbus Alzheimer.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von Psychosen und/oder psychotischen Störungen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung des Cushing-Syndroms oder sonstigen stressabhängigen Krankheiten.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von Schlafstörungen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von depressiven Erkrankungen. Eine besondere Form von depressiven Erkrankungen sind sogenannte childhood onset mood disorders, d.h. depressive Verstimmungen, die bereits in der Kindheit einsetzen.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung von vasomotorischen Symptomen und/oder thermoregulatorischen Fehlfunktionen, wie beispielsweise das "hot flush"-Symptom.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung und/oder Prophylaxe von Drogen-, Arzneimittel- und/oder durch sonstige Faktoren vermittelten Abhängigkeiten, zur Behandlung und/oder Prophylaxe von Stress bedingt durch den Entzug von einem oder mehreren die Abhängigkeit vermittelnden Faktoren und/oder zur Behandlung und/oder Prophylaxe von Stress-induzierten Rückfällen in die Drogen-, Arzneimittel- und/oder durch sonstige Faktoren vermittelten Abhängigkeiten.

In einer weiteren bevorzugten Ausführungsform betrifft die vorliegende Erfindung die Verwendung von erfindungsgemäßen Verbindungen der Formel I oder von pharmazeutisch verträglichen Salzen davon zur Herstellung eines Medikaments zur Behandlung und/oder Prophylaxe von Schizophrenie und/oder Psychose.

Ein weiterer Gegenstand der Erfindung betrifft ein Verfahren zur Behandlung und/oder Prophylaxe von Vasopressin-abhängigen Krankheiten, bei dem man einem Patienten eine wirksame Menge wenigstens einer erfindungsgemäßen Verbindung der Formel I oder wenigstens eines pharmazeutisch verträglichen Salzes davon oder eines erfindungsgemäßen pharmazeutischen Mittels verabreicht.

Bezüglich der Definition von Vasopressin-abhängigen Krankheiten wird auf die vorstehenden Ausführungen Bezug genommen.

In einer bevorzugten Ausführungsform der Erfindung dient das erfindungsgemäße Verfahren zur Behandlung und/oder Prophylaxe von Erkrankungen, die ausgewählt sind unter Diabetes, Insulin-Resistenz, Enuresis nocturna, Inkontinenz und Krankheiten, bei denen Blutgerinnungsstörungen auftreten, und/oder zur Verzögerung der Miktion. Bezüglich der Definition von Diabetes wird auf die vorstehenden Ausführungen verwiesen.

In einer weiteren bevorzugten Ausführungsform dient das erfindungsgemäße Verfahren zur Behandlung und/oder Prophylaxe von Erkrankungen, die ausgewählt sind unter Hypertonie, pulmonaler Hypertonie, Herzinsuffizienz, Myocardinfarkt, koronarem Spasmus, instabiler Angina, PTCA (percutaneous transluminal coronary angioplastie), Ischämien des Herzens, Störungen des renalen Systems, Ödeme, renalem Vasospasmus, Nekrose des renalen Cortex, Hyponatriämie, Hypokaliämie, Schwartz-Bartter Syndrom, Störungen des Gastrointestinaltraktes, gastritischem Vasospasmus, Hepatozirrhose, Magen- und Darmulkus, Emesis, auftretender Emesis bei der Chemotherapie und Reisekrankheit.

In einer weiteren bevorzugten Ausführungsform dient das erfindungsgemäße Verfahren zur Behandlung und/oder Prophylaxe von affektiven Störungen.

In einer weiteren bevorzugten Ausführungsform dient das erfindungsgemäße Verfahren zur Behandlung und/oder Prophylaxe von Angststörungen und/oder stressabhängigen Angststörungen.

In einer weiteren bevorzugten Ausführungsform dient das erfindungsgemäße Verfahren zur Behandlung und/oder Prophylaxe Gedächtnisleistungsstörungen und/oder Morbus Alzheimer.

In einer weiteren bevorzugten Ausführungsform dient das erfindungsgemäße Verfahren zur Behandlung und/oder Prophylaxe von Psychosen und/oder psychotischen Störungen.

In einer weiteren bevorzugten Ausführungsform dient das erfindungsgemäße Verfahren zur Behandlung und/oder Prophylaxe des Cushing-Syndroms.

In einer weiteren bevorzugten Ausführungsform dient das erfindungsgemäße Verfahren zur Behandlung und/oder Prophylaxe von Schlafstörungen bei einem Patienten.

In einer weiteren bevorzugten Ausführungsform dient das erfindungsgemäße Verfahren zur Behandlung und/oder Prophylaxe von depressiven Erkrankungen. Bei den depressiven Erkrankungen sind speziell auch die chlidhood onset mood disorders zu nennen, d.h. depressive Verstimmungen, die bereits im Kindesalter einsetzen.

In einer weiteren bevorzugten Ausführungsform dient das erfindungsgemäße Verfahren zur Behandlung und/oder Prophylaxe von vasomotorischen Symptomen und/oder thermoregulatorischen Fehlfunktionen, wie beispielsweise dem "hot flush"-Symptom.

In einer weiteren bevorzugten Ausführungsform dient das erfindungsgemäße Verfahren zur Behandlung und/oder Prophylaxe von Drogen-, Arzneimittel- und/oder durch sonstige Faktoren vermittelte Abhängigkeiten, zur Behandlung und/oder Prophylaxe von Stress bedingt durch den Entzug von einem oder mehreren die Abhängigkeit vermittelnden Faktoren und/oder zur Behandlung und/oder Prophylaxe von Stress-induzierten Rückfällen in die Drogen-, Arzneimittel- und/oder durch sonstige Faktoren vermittelten Abhängigkeiten.

In einer weiteren bevorzugten Ausführungsform dient das erfindungsgemäße Verfahren zur Behandlung und/oder Prophylaxe von Schizophrenie und/oder Psychose.

Bei dem mit dem erfindungsgemäßen Verfahren prophylaktisch oder therapeutisch zu behandelnden Patienten handelt es sich vorzugsweise um ein Säugetier, beispielsweise um einen Menschen oder um ein nichtmenschliches Säugetier oder um ein nichtmenschliches transgenes Säugetier. Speziell handelt es sich um einen Menschen.

Die Verbindungen der allgemeinen Formel I und ihre pharmazeutisch verträglichen Salze, wie vorstehend ausgeführt, können von einem Fachmann in Kenntnis der erfindungsgemäßen technischen Lehre in Ausführung und/oder in analoger Ausführung von an sich bekannten Verfahrensschritten hergestellt werden.

Die Verbindungen I und/oder deren pharmazeutisch verträglichen Salze zeichnen sich dadurch aus, dass sie eine Selektivität zum Vasopressin-Rezeptorsubtyp V1b gegenüber mindestens einem der nahe verwandten Vasopressin/Oxytozin-Rezeptorsubtypen (zum Beispiel Vasopressin V1a, Vasopressin V2 und/oder Oxytozin) aufweisen.

Alternativ oder vorzugsweise zusätzlich zeichnen sich die Verbindungen I und/oder deren pharmazeutisch verträglichen Salze dadurch aus, dass sie eine verbesserte metabolische Stabilität aufweisen.

Die metabolische Stabilität einer Verbindung kann beispielsweise gemessen werden, indem man eine Lösung dieser Verbindung mit Lebermikrosomen von bestimmten Spezies (zum Beispiel Ratte, Hund oder Mensch) inkubiert und die Halbwertszeit der Verbindung unter diesen Bedingungen bestimmt (RS Obach, Curr Opin Drug Discov Devel. 2001, 4, 36-44). Dabei kann aus einer beobachteten größeren Halbwertszeit auf eine verbesserte metabolische Stabilität der Verbindung geschlossen werden. Die Stabilität in Gegenwart von humanen Lebermikrosomen ist von besonderem Interesse, da sie eine Vorhersage für den metabolischen Abbau der Verbindung in der menschlichen Leber ermöglicht. Verbindungen mit erhöhter metabolischer Stabilität (gemessen in dem Lebermikrosomen-Test) werden deshalb wahrscheinlich auch in der Leber langsamer abgebaut. Der langsamere metabolische Abbau in der Leber kann zu höheren und/oder länger anhaltenden Konzentrationen (Wirkspiegel) der Verbindung im Körper führen, so dass die Eliminierungs-Halbwertszeit der erfindungsgemäßen Verbindungen erhöht ist.

Erhöhte und/oder länger anhaltende Wirkspiegel können zu einer besseren Wirksamkeit der Verbindung in der Behandlung oder Prophylaxe von verschiedenen Vasopressin-abhängigen Krankheiten führen. Außerdem kann eine verbesserte metabolische Stabilität zu einer erhöhten Bioverfügbarkeit nach oraler Gabe führen, da die Verbindung nach erfolgter Resorption im Darm einem geringeren metabolischen Abbau in der Leber (sogenannter "first pass effect") unterliegt. Eine erhöhte orale Bioverfügbarkeit kann aufgrund erhöhter Konzentration (Wirkspiegel) der Verbindung zu einer besseren Wirksamkeit der Verbindung nach oraler Gabe führen.

Alternativ oder vorzugsweise zusätzlich zeichnen sich die Verbindungen I und/oder deren pharmazeutisch verträglichen Salze dadurch aus, dass sie in Patienten oder relevanten Tiermodellen, die prognostische Aussagen für die Anwendung in der Behandlung ermöglichen, gegenüber den aus dem Stand der Technik bekannten Oxindol-Verbindungen eine verbesserte pharmakologische Aktivität aufweisen.

Die erfindungsgemäßen Verbindungen sind nach Verabreichung auf verschiedenen Wegen wirksam. Die Verabreichung kann beispielsweise intravenös, intramuskulär, subkutan, topisch, intratracheal, intranasal, transdermal, vaginal, rektal, sublingual, bukkal oder oral erfolgen und erfolgt häufig intravenös, intramuskulär oder insbesondere oral.

Die vorliegende Erfindung betrifft auch pharmazeutische Zusammensetzungen, die eine wirksame Dosis einer erfindungsgemäßen Verbindung I oder eines pharmazeutisch verträglichen Salzes davon und geeignete pharmazeutische Träger (Arzneiträger) enthalten.

Diese Arzneiträger werden entsprechend der pharmazeutischen Form und der gewünschten Applikationsart gewählt und sind dem Fachmann grundsätzlich bekannt.

Die erfindungsgemäßen Verbindungen der Formel I oder gegebenenfalls geeignete Salze dieser Verbindungen können zur Herstellung von pharmazeutischen Zusammensetzungen zur oralen, sublingualen, bukkalen, subkutanen, intramuskulären, intravenösen, topischen, intratrachealen, intranasalen, transdermalen, vaginalen oder rektalen Verabreichung verwendet werden und Tieren oder Menschen in einheitlichen Verabreichungsformen, gemischt mit herkömmlichen pharmazeutischen Trägern, zur Prophylaxe oder Behandlung der obigen Störungen oder Krankheiten verabreicht werden.

Die geeigneten Verabreichungsformen (Dosiereinheiten) umfassen Formen zur oralen Verabreichung, wie Tabletten, Gelatinekapseln, Pulver, Körnchen und Lösungen oder Suspensionen zur oralen Einnahme, Formen zur sublingualen, bukkalen, intratrachealen oder intranasalen Verabreichung, Aerosole, Implantate, Formen der subkutanen, intramuskulären oder intravenösen Verabreichung und Formen der rektalen Verabreichung.

Zur topischen Verabreichung können die erfindungsgemäßen Verbindungen in Cremes, Salben oder Lotionen verwendet werden.

Um den gewünschten prophylaktischen oder therapeutischen Effekt zu erzielen, kann die Dosis des Wirkstoffs zwischen 0.01 und 50 mg pro kg Körpergewicht und pro Tag variieren.

Jede Einheitsdosis kann 0.05 bis 5000 mg, vorzugsweise 1 bis 1000 mg, des Wirkstoffs in Kombination mit einem pharmazeutischen Träger enthalten. Diese Einheitsdosis kann 1-bis 5-mal am Tag verabreicht werden, so dass eine tägliche Dosis von 0,5 bis 25000 mg, vorzugsweise 1 bis 5000 mg, verabreicht wird.

Falls eine feste Zusammensetzung in Form von Tabletten zubereitet wird, wird der Wirkstoff mit einem festen pharmazeutischen Träger, wie Gelatine, Stärke, Laktose, Magnesiumstearat, Talk, Siliziumdioxid oder Ähnlichem, gemischt.

Die Tabletten können mit Saccharose, einem Cellulosederivat oder einer anderen geeigneten Substanz beschichtet werden oder anders behandelt werden, um eine anhaltende oder verzögerte Aktivität aufzuweisen und um eine vorbestimmte Menge des Wirkstoffs kontinuierlich freizugeben.

Eine Zubereitung in Form von Gelatinekapseln wird durch Mischen des Wirkstoffs mit einem Streckmittel und Aufnehmen der resultierenden Mischung in weiche oder harte Gelatinekapseln erhalten.

Eine Zubereitung in Form eines Sirups oder Elixiers oder zur Verabreichung in Form von Tropfen kann Wirkstoffe zusammen mit einem Süßstoff, der vorzugsweise kalorienfrei ist, Methylparaben oder Propylparaben als Antiseptika, einen Aromastoff und einen geeigneten Farbstoff enthalten.

Die wasserdispergierbaren Pulver oder Körnchen können die Wirkstoffe, gemischt mit Dispergiermitteln, Benetzungsmitteln oder Suspensionsmitteln, wie Polyvinylpyrrolidone, sowie Süßstoffe oder Geschmackskorrigentien, enthalten.

Eine rektale oder vaginale Verabreichung wird durch Verwendung von Zäpfchen erreicht, die mit Bindemitteln zubereitet werden, die bei rektaler Temperatur schmelzen, zum Beispiel Kakaobutter oder Polyethylenglykole. Eine parenterale Verabreichung erfolgt unter Verwendung von wässrigen Suspensionen, isotonischen Salzlösungen oder sterilen und injizierbaren Lösungen, die pharmakologisch verträgliche Dispergiermittel und/oder Benetzungsmittel, zum Beispiel Propylenglykol oder Polyethylenglykol, enthalten.

Der Wirkstoff kann auch als Mikrokapseln oder Zentrosome, falls geeignet mit einem oder mehreren Trägern oder Additiven, formuliert werden.

Zusätzlich zu den erfindungsgemäßen Verbindungen können die erfindungsgemäßen Mittel andere Wirkstoffe enthalten, die zur Behandlung der oben angegebenen Störungen oder Krankheiten nützlich sein können.

Die vorliegende Erfindung betrifft somit weiterhin pharmazeutische Mittel, in denen mehrere Wirkstoffe zusammen anwesend sind, wobei mindestens einer von diesen eine erfindungsgemäße Verbindung I oder ein Salz davon ist.

Die Erfindung wird nachstehend anhand von Beispielen näher erläutert, wobei die Beispiele nicht einschränkend zu verstehen sind.

Die Herstellung der erfindungsgemäßen Verbindungen kann über verschiedene Syntheserouten erfolgen. In den nachfolgenden Synthesebeispielen erfolgte die Herstellung der genannten Verbindungen analog zu den in den Reaktionsschemata 1 bis 5 skizzierten Vorgehensweisen. Die Syntheseschemata 1 bis 5 sind jedoch nur exemplarisch und nicht einschränkend zu verstehen; die Synthese kann auch auf weiteren Routen erfolgen.

### EXPERIMENTELLER TEIL

Verwendete Abkürzungen:
- DIPEA:: Diisopropylethylamin
- DMSO:: Dimethylsulfoxid
- THF:: Tetrahydrofuran
- TFA:: Trifluoressigsäure
- p:: pseudo (beispielsweise pt pseudo Triplett)
- b:: breit (beispielsweise bs breites Singulett)
- s:: Singulett
- d:: Doublett
- t:: Triplett
- m:: Multiplett
- dd:: doppeltes Doublett
- dt:: doppeltes Triplett
- tt:: dreifaches Triplett

### I. Herstellung der Verbindungen I

Die Verbindungen der Formel I wurden in Analogie zu den in den Syntheseschemata 1 bis 5 beschriebenen Herstellungsverfahren synthetisiert. Die Verbindungen können durch Kristallisation und / oder präparative HPLC (RP, Eluenten Acetonitril / Wasser, 0.1 % TFA oder 0.1 % Essigsäure) gereinigt werden. Die Verbindungen I fallen dann gegebenenfalls als Trifluoressigsäure-Salz, Bis(trifluoressigsäure)-Salz bzw. Essigsäure-Salz an.

### I.1 Herstellung der Verbindungen I, worin X¹ für -O-C(=O)- steht

### BEISPIEL 1:

### 4-(4-Methyl-piperazin-1-yl)-benzoesäure-1-(2,4-dimethoxy-phenylsulfonyl)-3-(2-ethoxy-pyridin-3-yl)-5-iod-2-oxo-2,3-dihydro-1H-indol-3ylester

### 1.1 4-(4-Methyl-piperazin-1-yl)-benzoesäure-3-(2-ethoxy-pyridin-3-yl)-5-iod-2-oxo-2,3dihydro-1 H-indol-3ylester

ESI-MS: 599.10 [M+H]⁺
¹H-NMR (500 MHz, d₆-DMSO): δ [ppm] 10.40 (s, 1 H); 8.30 (d, 1 H); 8.20 (d, 1 H); 7.90 (d, 2H); 7.60 (d, 1H); 7.35 (s, 1H); 7.20 (m, 1 H); 7.05 (d, 2H); 6.75 (d, 1 H); 4.15 (m, 2H); 3.35 (bs, 4H); 2.45 (bs, 4H); 2.20 (s, 3H); 1.05 (t, 3H).

### 1.2 4-(4-Methyl-piperazin-1-yl)-benzoesäure-1-(2,4-dimethoxy-phenylsulfonyl)-3-(2ethoxy-pyridin-3-yl)-5-iod-2-oxo-2,3-dihydro-1 H-indol-3ylester

ESI-MS: 799.20 [M+H]⁺
¹H-NMR (500 MHz, d₆-DMSO): δ [ppm] 8.35 (d, 1 H); 8.20 (d, 1 H); 7.90 (d, 1 H); 7.80 (d, 1 H); 7.75 (d, 2H); 7.65 (d, 1 H); 7.45 (s, 1 H); 7.20 (m, 1 H); 7.00 (d, 2H); 6.75 (s, 1 H); 6.70 (d, 1 H); 4.15 (m, 2H); 3.90 (s, 3H); 3.65 (s, 3H); 3.35 (bs, 4H); 2.45 (bs, 4H); 2.20 (s, 3H); 1.05 (t, 3H).

### BEISPIEL 2:

### 4-(4-Methyl-piperazin-1-yl)-benzoesäure-5-cyano-3-(2-ethoxy-pyridin-3-yl)-1-(4-methoxy-phenylsulfonyl)-2-oxo-2,3-dihydro-1H-indol-3ylester

### 2.1 4-(4-Methyl-piperazin-1-yl)-benzoesäure-5-cyano-3-(2-ethoxy-pyridin-3-yl)-2-oxo2,3-dihydro-1 H-indol-3ylester

ESI-MS: 498.20 [M+H]⁺

### 2.2 4-(4-Methyl-piperazin-1-yl)-benzoesäure-5-cyano-3-(2-ethoxy-pyridin-3-yl)-1-(4-methoxy-phenylsulfonyl)-2-oxo-2,3-dihydro-1H-indol-3ylester

ESI-MS: 668.20 [M+H]⁺
¹H-NMR (500 MHz, d₆-DMSO): δ [ppm] 8.45 (d, 1 H); 8.20 (m, 1 H); 8.05 (m, 3H); 7.95 (d, 1H); 7.75 (m, 1H); 7.70 (d, 2H); 7.25 (m, 3H); 6.95 (d, 2H); 4.10 (m, 1H); 4.00 (m, 1H); 3.90 (s, 3H); 3.35 (bs, 4H); 2.45 (bs, 4H); 2.20 (s, 3H); 0.95 (t, 3H).

### BEISPIEL 3:

### 4-(4-Methyl-piperazin-1-yl)-benzoesäure-5-cyano-1-(2,4-dimethoxy-phenylsulfonyl)-3-(2-ethoxy-pyridin-3-yl)-2-oxo-2,3-dihydro-1H-indol-3ylester

ESI-MS: 698.25 [M+H]⁺
¹H-NMR (500 MHz, d₆-DMSO): δ [ppm] 8.40 (d, 1 H); 8.20 (m, 1 H); 8.00 (d, 1 H); 7.95 (d, 1 H); 7.85 (d, 1 H); 7.70 (m, 3H); 7.20 (m, 1 H); 6.95 (d, 2H); 6.75 (d, 1 H); 6.65 (dd, 1 H); 4.10 (m, 2H); 3.85 (s, 3H); 3.55 (s, 3H); 3.30 (bs, 4H); 2.40 (bs, 4H); 2.15 (s, 3H); 1.00 (t, 3H).

### I.2 Herstellung der Verbindungen I, worin X¹ für -NH-C(=O)- steht

### BEISPIEL 4:

### N-[5-Cyano-3-(2-ethoxy-pyridin-3-yl)-1-(4-methoxy-phenylsulfonyl)-2-oxo-2,3-dihydro-1 H-indol-3-yl]-4-(4-methyl-piperazin-1-yl)-benzamid

### 4.1 3-Amino-3-(2-ethoxy-pyridin-3-yl)-1-(4-methoxy-phenylsulfonyl)-2-oxo-2,3-dihydro-1 H-indol-5-carbonitril

ESI-MS: 465.10 [M+H]⁺

### 4.2 N-[5-Cyano-3-(2-ethoxy-pyridin-3-yl)-1-(4-methoxy-phenylsulfonyl)-2-oxo-2,3-dihydro-1 H-indol-3-yl]-benzamid

ESI-MS: 569.15 [M+H]⁺
¹H-NMR (500 MHz, d₆-DMSO): δ [ppm] 9.65 (s, 1 H); 8.20 (m, 1 H); 8.05 (d, 2H); 8.00 (m, 2H); 7.90 (d, 1H); 7.80 (m, 3H); 7.55 (m, 1H); 7.45 (m, 2H); 7.20 (d, 1H); 7.10 (m, 1H); 4.20 (m, 1 H); 4.15 (m, 1 H); 3.90 (s, 3H); 1.05 (t, 3H).

### 4.3 4-Brom-N-[5-cyano-3-(2-ethoxy-pyridin-3-yl)-1-(4-methoxy-phenylsulfonyl)-2-oxo-2,3-dihydro-1 H-indol-3-yl]-benzamid

ESI-MS: 647.05/649.05 [M+H]⁺
¹H-NMR (500 MHz, d₆-DMSO): δ [ppm] 9.75 (s, 1 H); 8.20 (m, 1 H); 8.05 (d, 2H); 8.00 (m, 2H); 7.85 (d, 1H); 7.75 (s, 1H); 7.70 (m, 4H); 7.20 (d, 1H); 7.10 (m, 1H); 4.20 (m, 1H); 4.15 (m, 1 H); 3.90 (s, 3H); 1.05 (t, 3H).

### 4.4 N-[5-Cyano-3-(2-ethoxy-pyridin-3-yl)-1-(4-methoxy-phenylsulfonyl)-2-oxo-2,3-dihydro-1 H-indol-3-yl]-4-(4-methyl-piperazin-1-yl)-benzamid

ESI-MS: 667.25 [M+H]⁺
¹H-NMR (500 MHz, d₆-DMSO): δ [ppm] 9.30 (s, 1 H); 8.20 (m, 1 H); 8.05 (m, 2H); 7.95 (m, 2H); 7.85 (d, 1H); 7.75 (s, 1H); 7.65 (d, 1 H); 7.20 (d, 2H); 7.10 (m, 1H); 6.95 (d, 2H); 4.15 (m, 1 H); 4.10 (m, 1 H); 3.90 (s, 3H); 3.25 (m, 4H); 2.45 (m, 4H); 2.25 (s, 3H); 1.05 (t, 3H).

### BEISPIEL5:

### N-[5-Cyano-3-(2-ethoxy-pyridin-3-yl)-1-(4-methoxy-phenylsulfonyl)-2-oxo-2,3-dihydro-1 H-indol-3-yl]-4-(4-ethyl-piperazin-1-yl)-benzamid

ESI-MS: 681.30 [M+H]⁺
¹H-NMR (500 MHz, d₆-DMSO): δ [ppm] 9.30 (s, 1 H); 8.20 (m, 1 H); 8.05 (d, 2H); 7.95 (m, 2H); 7.85 (d, 1H); 7.75(s, 1H); 7.65 (d, 1 H); 7.20 (d, 2H); 7.10 (m, 1 H); 6.95 (d, 2H); 4.15 (m, 1 H); 4.10 (m, 1 H); 3.90 (s, 3H); 3.30 (bs, 4H); 2.50 (bs, 4H); 2.40 (bs, 2H); 1.05 (m, 6H).

### BEISPIEL 6:

### N-[5-Cyano-1-(2,4-dimethoxy-phenylsulfonyl)-3-(2-ethoxy-pyridin-3-yl)-2-oxo-2,3-dihydro-1 H-indol-3-yl]-4-(4-methyl-piperazin-1-yl)-benzamid

### 6.1 3-Amino-1-(2,4-dimethoxy-phenylsulfonyl)-3-(2-ethoxy-pyridin-3-yl)-2-oxo-2,3-dihydro-1H-indol-5-carbonitril

ESI-MS: 495.10 [M+H]⁺

### 6.2 N-[5-Cyano-1-(2,4-dimethoxy-phenylsulfonyl)-3-(2-ethoxy-pyridin-3-yl)-2-oxo-2,3-dihydro-1H-indol-3-yl]-4-(4-methyl-piperazin-1-yl)-benzamid

ESI-MS: 697.30 [M+H]⁺
¹H-NMR (500 MHz, d₆-DMSO): δ [ppm] 9.35 (s, 1 H); 8.25 (m, 1 H); 8.05 (m, 2H); 7.95 (d, 1 H); 7.90 (s, 1 H); 7.75 (m, 3H); 7.15 (m, 1 H); 7.00 (d, 2H); 6.80 (m, 2H); 4.35 (m, 2H); 3.95 (s, 3H); 3.60 (s, 3H); 3.35 (m, 4H); 2.50 (bs, 4H); 2.30 (s, 3H); 1.20 (t, 3H).

### BEISPIEL 7:

### N-[5-Cyano-1-(2,4-dimethoxy-phenylsulfonyl)-3-(2-ethoxy-pyridin-3-yl)-2-oxo-2,3-dihydro-1 H-indol-3-yl]-4-(4-ethyl-piperazin-1-yl)-benzamid

ESI-MS: 711.25 [M+H]⁺
¹H-NMR (500 MHz, d₆-DMSO): δ [ppm] 9.30 (s, 1 H); 8.20 (m, 1 H); 7.95 (m, 2H); 7.85 (d, 1 H); 7.80 (s, 1 H); 7.70 (m, 3H); 7.05 (m, 1 H); 6.95 (d, 2H); 6.75 (m, 2H); 4.25 (m, 2H); 3.90 (s, 3H); 3.50 (s, 3H); 3.30 (bs, 4H); 2.50 (bs, 4H); 2.35 (m, 2H); 1.15 (t, 3H); 1.05 (t, 3H).

### BEISPIEL 8:

### N-[1-[4-(2,2-Difluor-ethoxy)-phenylsulfonyl]-3-(2-ethoxy-pyridin-3-yl)-5-iod-2-oxo-2,3-dihydro-1 H-indol-3-yl]-4-(4-methyl-piperazin-1-yl)-benzamid

ESI-MS: 818.20 [M+H]⁺
¹H-NMR (500 MHz, d₆-DMSO): δ [ppm] 9.25 (s, 1 H); 8.20 (m, 1 H); 8.05 (d, 2H); 7.75 (d, 1 H); 7.70 (m, 2H); 7.65 (d, 2H); 7.60 (d, 1 H); 7.30 (d, 2H); 7.05 (m, 1 H); 6.95 (d, 2H); 6.45 (t, *J =* 70 Hz, 1 H); 4.50 (m, 2H); 4.20 (m, 2H); 3.30 (m, 4H); 2.50 (m, 4H); 2.25 (m, 3H); 1.15 (t, 3H).

### I.3 Herstellung der Verbindungen I, worin X¹ für -NH-CH₂- steht,

### I.3.1 Verbindungen I, worin X¹ für-NH-CH₂- und A für 1,4-Phenylen stehen

### BEISPIEL 9:

### 1-(2,4-Dimethoxy-phenylsulfonyl)-3-(2-ethoxy-pyridin-3-yl)-5-iod-3-[4-(4-methyl-piperazin-1-yl)-benzylamino]-1,3-dihydro-indol-2-on

### 9.1 3-(2-Ethoxy-pyridin-3-yl)-5-iod-3-[4-(4-methyl-piperazin-1-yl)-benzylamino]-1,3-dihydro-indol-2-on

ESI-MS: 584.10 [M+H]⁺

### 9.2 1-(2,4-Dimethoxy-phenylsulfonyl)-3-(2-ethoxy-pyridin-3-yl)-5-iod-3-[4-(4-methylpiperazin-1-yl)-benzylamino]-1,3-dihydro-indol-2-on

ESI-MS: 784.20 [M+H]^{+ 1}H-NMR (500 MHz, d₆-DMSO): δ [ppm] 8.20 (m, 1H); 8.15 (d, 1H); 8.10 (m, 1H); 7.80 (d, 1 H); 7.70 (d, 1 H); 7.25 (s, 1 H); 6.90 (m, 3H); 6.80 (d, 2H); 6.65 (d, 1 H); 6.35 (s, 1 H); 4.25 (m, 1 H); 4.20 (m, 1 H); 3.85 (s, 3H); 3.55 (s, 3H); 3.20 (s, 4H); 3.05 (m, 1 H); 2.90 (m, 1 H); 2.55 (s, 4H); 2.35 (s, 3H); 2.05 (m, 1 H); 1.20 (t, 3H).

### BEISPIEL 10:

### 1-(2,4-Dimethoxy-phenylsulfonyl)-3-(2-ethoxy-pyridin-3-yl)-5-iod-3-(4-piperazin-1-yl-benzylamino)-1,3-dihydro-indol-2-on als Essigsäure-Salz

ESI-MS: 770.15 [M+H]⁺

### BEISPIEL 11:

### 5-Chlor-3-(2-ethoxy-pyridin-3-yl)-1-(4-methoxy-phenylsulfonyl)-3-[4-(4-methyl-piperazin-1-yl)-benzylamino]-1,3-dihydro-indol-2-on

### 11.1 5-Chlor-3-(2-ethoxy-pyridin-3-yl)-3-[4-(4-methyl-piperazin-1-yl)-benzylamino]-1,3-dihydro-indol-2-on

ESI-MS: 492.25 [M+H]⁺

### 11.2 5-Chlor-3-(2-ethoxy-pyridin-3-yl)-1-(4-methoxy-phenylsulfonyl)-3-[4-(4-methylpiperazin-1-yl)-benzylamino]-1,3-dihydro-indol-2-on

ESI-MS: 662.25 [M+H]⁺
¹H-NMR (500 MHz, CDCl₃): δ [ppm] 8.15 (m, 1 H); 8.10 (d, 2H); 8.05 (m, 1 H); 7.95 (d, 1 H); 7.35 (d, 1 H); 7.00 (d, 2H); 6.90 (m, 4H); 6.80 (d, 2H); 4.10 (m, 1 H); 4.05 (m, 1 H); 3.85 (s, 3H); 3.15 (m, 4H); 2.95 (m, 1H); 2.70 (m, 1H); 2.55 (m, 4H); 2.35 (s, 3H); 2.05 (m, 1H); 1.05 (t, 3H).

### BEISPIEL 12:

### 5-Chlor-3-(2-ethoxy-pyridin-3-yl)-6-fluor-1-(4-methoxy-phenylsulfonyl)-3-[4-(4-methylpiperazin-1-yl)-benzylamino]-1,3-dihydro-indol-2-on

### 12.1 5-Chlor-3-(2-ethoxy-pyridin-3-yl)-6-fluor-3-[4-(4-methyl-piperazin-1-yl)-benzylamino]-1,3-dihydro-indol-2-on

ESI-MS: 510.20 [M+H]⁺

### 12.2 5-Chlor-3-(2-ethoxy-pyridin-3-yl)-6-fluor-1-(4-methoxy-phenylsulfonyl)-3-[4-(4-methyl-piperazin-1-yl)-benzylamino]-1,3-dihydro-indol-2-on

ESI-MS: 680.20 [M+H]⁺
¹H-NMR (500 MHz, CDCl₃): δ [ppm] 8.15 (m, 1 H); 8.10 (d, 2H); 8.05 (m, 1 H); 7.90 (d, 1 H); 7.00 (d, 2H); 6.90 (m, 4H); 6.80 (d, 2H); 4.10 (m, 2H); 3.85 (s, 3H); 3.15 (m, 4H); 2.90 (m, 1H); 2.70 (m, 1H); 2.55 (m, 4H); 2.35 (s, 3H); 2.10 (m, 1H); 1.10 (t, 3H).

### BEISPIEL 13:

### 6-Chlor-3-(2-ethoxy-pyridin-3-yl)-5-fluor-1-(4-methoxy-phenylsulfonyl)-3-[4-(4-methylpiperazin-1-yl)-benzylamino]-1,3-dihydro-indol-2-on

### 13.1 6-Chlor-3-(2-ethoxy-pyridin-3-yl)-5-fluor-3-[4-(4-methyl-piperazin-1-yl)-benzylamino]-1,3-dihydro-indol-2-on

ESI-MS: 510.25 [M+H]⁺

### 13.2 6-Chlor-3-(2-ethoxy-pyridin-3-yl)-5-fluor-1-(4-methoxy-phenylsulfonyl)-3-[4-(4-methyl-piperazin-1-yl)-benzylamino]-1,3-dihydro-indol-2-on

ESI-MS: 680.20 [M+H]⁺
¹H-NMR (400 MHz, CDCl₃): δ [ppm] 8.20-8.05 (m, 5H); 7.00 (d, 2H); 6.90 (m, 3H); 6.80 (d, 2H); 6.75 (d, 1 H); 4.10 (m, 2H); 3.85 (s, 3H); 3.20 (m, 4H); 2.90 (m, 1 H); 2.70 (m, 1 H); 2.60 (m, 4H); 2.35 (s, 3H); 2.10 (m, 1H); 1.10 (t, 3H).

### BEISPIEL 14:

### 5,6-Difluor-3-(2-ethoxy-pyridin-3-yl)-1-(4-methoxy-phenylsulfonyl)-3-[4-(4-methylpiperazin-1-yl)-benzylamino]-1,3-dihydro-indol-2-on

ESI-MS: 664.25 [M+H]⁺
¹H-NMR (500 MHz, CDCl₃): δ [ppm] 8.15 (d, 1 H); 8.10 (d, 2H); 8.05 (m, 1 H); 7.95 (m, 1 H); 7.00 (d, 2H); 6.90 (m, 3H); 6.80 (m, 3H); 4.15 (m, 1 H); 4.10 (m, 1 H); 3.85 (s, 3H); 3.15 (m, 4H); 2.90 (m, 1 H); 2.70 (m, 1 H); 2.60 (m, 4H); 2.35 (s, 3H); 2.10 (m, 1 H); 1.10 (t, 3H).

### BEISPIEL 15:

### 3-(2-Ethoxy-pyridin-3-yl)-5-fluor-1-(4-methoxy-phenylsulfonyl)-3-[4-(4-methyl-piperazin-1-yl)-benzylamino]-1,3-dihydro-indol-2-on

ESI-MS: 646.25 [M+H]⁺

### BEISPIEL 16:

### 3-(2-Ethoxy-pyridin-3-yl)-1-(4-methoxy-phenylsulfonyl)-3-[4-(4-methyl-piperazin-1-yl)-benzylamino]-2-oxo-2,3-dihydro-1 H-indol-5-carbonitril

### 16.1 3-(2-Ethoxy-pyridin-3-yl)-3-[4-(4-methyl-piperazin-1-yl)-benzylamino]-2-oxo-2,3-dihydro-1H-indol-5-carbonitril

ESI-MS: 483.25 [M+H]⁺
¹H-NMR (500 MHz, d₆-DMSO): δ [ppm] 8.25 (d, 1 H); 8.10 (m, 1 H); 7.65 (d, 1 H); 7.15 (m, 3H); 7.10 (m, 1H); 7.05 (d, 1H); 6.90 (d, 2H); 4.05 (m, 3H); 3.50-3.15 (m, 6H); 3.00 (bs, 4H); 2.60 (s, 3); 0.95 (t, 3H).

### 16.2 3-(2-Ethoxy-pyridin-3-yl)-1-(4-methoxy-phenylsulfonyl)-3-[4-(4-methyl-piperazin-1-yl)-benzylamino]-2-oxo-2,3-dihydro-1 H-indol-5-carbonitril

ESI-MS: 653.20 [M+H]⁺
¹H-NMR (500 MHz, d₆-DMSO): δ [ppm] 8.30 (d, 1H); 8.20 (m, 3H); 8.15 (d, 1H); 7.95 (d, 1 H); 7.40 (s, 1 H); 7.30 (d, 2H); 7.20 (m, 1 H); 6.95 (d, 2H); 6.85 (d, 2H); 4.05 (m, 1 H); 3.90 (m, 4H); 3.80 (m, 1 H); 3.15 (m, 4H); 3.00 (m, 1 H); 2.95 (m, 1 H); 2.50 (m, 4H); 2.30 (s, 3H); 0.90 (t, 3H).

### BEISPIEL 17:

### 1-(2,4-Dimethoxy-phenylsulfonyl)-3-(2-ethoxy-pyridin-3-yl)-3-[4-(4-methyl-piperazin-1-yl)-benzylamino]-2-oxo-2,3-dihydro-1 H-indol-5-carbonitril

ESI-MS: 683.25 [M+H]⁺
¹H-NMR (500 MHz, d₆-DMSO): δ [ppm] 8.20 (d, 1H); 8.05 (m, 3H); 7.70 (d, 1H); 7.20 (s, 1 H); 6.95 (m, 1 H); 6.80 (d, 2H); 6.75 (d, 2H); 6.65 ( d, 1 H); 6.50 (s, 1 H); 4.05 (m, 2H); 3.80 (s, 3H); 3.50 (s, 3H); 3.05 (m, 4H); 2.90 (m, 1 H); 2.80 (m, 1 H); 2.50 (m, 4H); 2.25 (bs, 3H); 1.05 (t, 3H).

### BEISPIEL 18:

### 3-(2-Ethoxy-pyridin-3-yl)-1-(4-methoxy-2-trifluormethoxy-phenylsulfonyl)-3-[4-(4-methylpiperazin-1-yl)-benzylamino]-2-oxo-2,3-dihydro-1H-indol-5-carbonitril

ESI-MS: 737.25 [M+H]⁺
¹H-NMR (500 MHz, d₆-DMSO): δ [ppm] 8.35 (d, 1H); 8.15 (m, 2H); 8.10 (m, 1 H); 7.70 (d, 1 H); 7.25 (m, 1 H); 7.00-6.90 (m, 4H); 6.85 (m, 3H); 4.25 (m, 2H); 3.90 (s, 3H); 3.20 (m, 4H); 3.05 (m, 1 H); 2.95 (m, 1 H); 2.55 (m, 4H); 2.35 (s, 3H); 2.10 (m, 1 H); 1.10 (t, 3H).

### BEISPIEL 19:

### 3-(2-Ethoxy-pyridin-3-yl)-3-[4-(4-methyl-piperazin-1-yl)-benzylamino]-2-oxo-1-(4-trifluormethoxy-phenylsulfonyl)-2,3-dihydro-1 H-indol-5-carbonitril

ESI-MS: 707.20 [M+H]⁺
¹H-NMR (500 MHz, CDCl₃): δ [ppm] 8.30 (d, 2H); 8.20 (m, 1 H); 8.10 (m, 2H); 7.70 (d, 1 H); 7.40 (d, 2H); 7.25 (s, 1H); 6.95 (m, 1H); 6.90 (d, 2H); 6.80 (d, 2H); 4.15 (m, 2H); 4.00 (m, 1 H); 3.20 (m, 4H); 2.95 (m, 1 H); 2.85 (m, 1 H); 2.55 (m, 4H); 2.35 (s, 3H); 2.15 (m, 1 H); 0.95 (t, 3H).

### BEISPIEL 20:

### 3-(2-Ethoxy-pyridin-3-yl)-3-[4-(4-methyl-piperazin-1-yl)-benzylamino]-2-oxo-1-[4-(2,2,2-trifluor-ethoxy)-phenylsulfonyl]-2,3-dihydro-1 H-indol-5-carbonitril

ESI-MS: 721.20 [M+H]⁺
¹H-NMR (500 MHz, CDCl₃): δ [ppm] 8.20 (m, 3H); 8.15 (d, 1 H); 8.10 (m, 1 H); 7.65 (d, 1 H); 7.25 (s, 1H); 7.10 (d, 2H); 6.95 (m, 1H); 6.85 (d, 2H); 6.80 (d, 2H); 4.35 (m, 2H); 4.15 (m, 1 H); 4.05 (m, 1 H); 3.15 (m, 4H); 2.90 (m, 1 H); 2.70 (m, 1 H); 2.55 (m, 4H); 2.35 (s, 3H); 2.10 (m, 1 H); 1.05 (t, 3H).

### BEISPIEL 21:

### 1-[4-(2,2-Difluor-ethoxy)-phenylsulfonyl]-3-(2-ethoxy-pyridin-3-y1)-3-[4-(4-methyl-piperazin-1-yl)-benzylamino]-2-oxo-2,3-dihydro-1 H-indol-5-carbonitril

ESI-MS: 703.20 [M+H]⁺
¹H-NMR (500 MHz, CDCl₃): δ [ppm] 8.15 (m, 4H); 8.10 (d, 1 H); 7.65 (d, 1 H); 7.25 (s, 1 H); 7.05 (d, 2H); 6.95 (m, 1 H); 6.85 (d, 2H); 6.80 (d, 2H); 6.05 (t, *J* = 70 Hz, 1 H); 4.25-4.00 (m, 4H); 3.15 (m, 4H); 2.85 (m, 1H); 2.70 (m, 1H); 2.55 (m, 4H); 2.35 (s, 3H); 2.10 (m, 1H); 1.05 (t, 3H).

### BEISPIEL 22:

### 3-(2-Ethoxy-pyridin-3-yl)-1-[4-(2-fluor-ethoxy)-phenylsulfonyl]-3-[4-(4-methyl-piperazin-1-yl)-benzylamino]-2-oxo-2,3-dihydro-1 H-indol-5-carbonitril

ESI-MS: 685.20 [M+H]⁺
¹H-NMR (500 MHz, CDCl₃): δ [ppm] 8.20 (d, 1 H); 8.15 (m, 3H); 8.10 (m, 1 H); 7.65 (d, 1 H); 7.20 (s, 1 H); 7.05 (d, 2H); 6.95 (m, 1 H); 6.85 (d, 2H); 6.80 (d, 2H); 4.80 (t, 1 H); 4.70 (t, 1 H); 4.25 (m, 1 H); 4.20 (m, 1 H); 4.15 (m, 1 H); 4.05 (m, 1 H); 3.15 (m, 4H); 2.90 (m, 1 H); 2.75 (m, 1H); 2.55 (m, 4H); 2.35 (s, 3H); 2.10 (m, 1H); 1.05 (t, 3H).

### BEISPIEL 23:

### 1-(4-Difluormethoxy-phenylsulfonyl)-3-(2-ethoxy-pyridin-3-yl)-3-[4-(4-methyl-piperazin-1-yl)-benzylamino]-2-oxo-2,3-dihydro-1 H-indol-5-carbonitril

ESI-MS: 689.20 [M+H]⁺
¹H-NMR (500 MHz, CDCl₃): δ [ppm] 8.20 (m, 3H); 8.10 (m, 2H); 7.70 (d, 1 H); 7.30 (d, 2H); 7.25 (s, 1 H); 6.95 (m, 1 H); 6.85 (d, 2H); 6.80 (d, 2H); 6.55 (t, J = 70 Hz, 1 H); 4.15 (m, 1 H); 4.05 (m, 1H); 3.20 (m, 4H); 2.95 (m, 1H); 2.75 (m, 1H); 2.55 (m, 4H); 2.35 (s, 3H); 2.15 (m, 1 H); 1.00 (t, 3H).

### BEISPIEL 24:

### 3-(2-Ethoxy-pyridin-3-yl)-1-(2-methoxy-phenylsulfonyl)-3-[4-(4-methyl-piperazin-1-yl)-benzylamino]-2-oxo-2,3-dihydro-1 H-indol-5-carbonitril

ESI-MS: 653.20 [M+H]⁺
¹H-NMR (500 MHz, CDCl3): δ [ppm] 8.30 (d, 1 H); 8.20 (m, 2H); 8.10 (m, 1 H); 7.70 (d, 1 H); 7.65 (t, 1 H); 7.30 (s, 1 H); 7.20 (d, 1 H); 6.95 (m, 2H); 6.85 (d, 2H); 6.80 (d, 2H); 4.25 (m, 1 H); 4.20 (m, 1 H); 3.55 (s, 3H); 3.20 (m, 4H); 3.00 (m, 1 H); 2.85 (m, 1 H); 2.55 (m, 4H); 2.35 (s, 3H); 2.10 (m, 1 H); 1.20 (t, 3H).

### BEISPIEL 25:

### 3-(2-Ethoxy-pyridin-3-yl)-1-(3-methoxy-phenylsulfonyl)-3-[4-(4-methyl-piperazin-1-yl)-benzylamino]-2-oxo-2,3-dihydro-1H-indol-5-carbonitril

ESI-MS: 653.20 [M+H]⁺
¹H-NMR (500 MHz, CDCl₃): δ [ppm] 8.20 (m, 1 H); 8.15 (d, 1 H); 8.10 (m, 1 H); 7.75 (m, 2H); 7.65 (m, 1 H); 7.45 (t, 1 H); 7.20 (m, 2H); 6.95 (m, 1 H); 6.90 (d, 2H); 6.80 (d, 2H); 4.15 (m, 1 H); 4.05 (m, 1 H); 3.85 (s, 3H); 3.20 (m, 4H); 2.90 (m, 1 H); 2.85 (m, 1 H); 2.55 (m, 4H); 2.35 (s, 3H); 2.15 (m, 1 H); 1.05 (t, 3H).

### BEISPIEL 26:

### 1-(4-Chlor-phenylsulfonyl)-3-(2-ethoxy-pyridin-3-yl)- 3-[4-(4-methyl-piperazin-1-yl)-benzylamino]-2-oxo-2,3-dihydro-1 H-indol-5-carbonitril

ESI-MS: 657.20 [M+H]⁺
¹H-NMR (500 MHz, CDCl₃): δ [ppm] 8.20 (d, 1H); 8.15 (m, 4H); 7.70 (d, 1H); 7.55 (d, 2H); 7.25 (s, 1 H); 6.95 (m, 1 H); 6.85 (d, 2H); 6.80 (d, 2H); 4.10 (m, 1 H); 4.05 (m, 1 H); 3.20 (m, 4H); 2.95 (m, 1 H); 2.75 (m, 1 H); 2.55 (m, 4H); 2.35 (s, 3H); 2.10 (m, 1 H); 1.05 (t, 3H).

### BEISPIEL 27:

### 1-Phenylsulfonyl-3-(2-ethoxy-pyridin-3-yl)-3-[4-(4-methyl-piperazin-1-yl)-benzylamino]-2-oxo-2,3-dihydro-1 H-indol-5-carbonitril

ESI-MS: 623.25 [M+H]⁺
¹H-NMR (500 MHz, CDCl₃): δ [ppm] 8.20 (m, 3H); 8.15 (d, 1H); 8.10 (m, 1H); 7.70 (m, 2H); 7.60 (m, 2H); 7.20 (s, 1 H); 6.85 (d, 2H); 6.80 (d, 2H); 4.10 (m, 1 H); 4.00 (m, 1 H); 3.20 (m, 4H); 2.90 (m, 1 H); 2.75 (m, 1 H); 2.55 (m, 4H); 2.35 (s, 3H); 2.15 (m, 1 H); 1.00 (t, 3H).

### BEISPIEL 28:

### 1-(4-Cyano-phenylsulfonyl)-3-(2-ethoxy-pyridin-3-yl)- 3-[4-(4-methyl-piperazin-1-yl)-benzylamino]-2-oxo-2,3-dihydro-1 H-indol-5-carbonitril

ESI-MS: 648.25 [M+H]⁺
¹H-NMR (500 MHz, d₆-DMSO): δ [ppm] 8.35 (m, 2H); 8.25 (m, 3H); 8.15 (m, 1 H); 8.05 (d, 1H); 7.90 (d, 1H); 7.35 (s, 1H); 7.15 (m, 1H); 6.90 (d, 2H); 6.80 (d, 2H); 3.95 (m, 1 H); 3.80 (m, 2H); 3.10 (m, 4H); 3.00 (m, 1H); 2.95 (m, 1H); 2.45 (m, 4H); 2.20 (s, 3H); 0.80 (t, 3H).

### BEISPIEL 29:

### 1-(2,4-Dimethoxy-phenylsulfonyl)-3-(2-ethoxy-pyridin-3-yl)-2-oxo-3-(4-piperazin-1-yl-benzylamino)-2,3-dihydro-1 H-indol-5-carbonitril als Hydrochlorid

### 29.1 4-(4-{[5-Cyano-3-(2-ethoxy-pyridin-3-yl)-2-oxo-2,3-dihydro-1 H-indol-3-ylamino]-methyl}-phenyl)-piperazin-1-carbonsäure-tert-butylester

ESI-MS: 569.30 [M+H]⁺

### 29.2 4-(4-{[5-Cyano-1-(2,4-dimethoxy-phenylsulfonyl)-3-(2-ethoxy-pyridin-3-yl)-2-oxo-2,3-dihydro-1 H-indol-3-ylamino]-methyl}-phenyl)-piperazin-1-carbonsäure-tert-butylester

### 29.3 4-(4-{[5-Cyano-3-(2-ethoxy-pyridin-3-yl)-1-(4-methoxy-phenylsulfonyl)-2-oxo-2,3-dihydro-1H-indol-3-ylamino]-methyl}-phenyl)-piperazin-1-carbonsäure-tert-butylester

ESI-MS: 639.20 [M-Boc+H]⁺
¹H-NMR (400 MHz, d₆-DMSO): δ [ppm] 8.25 (d, 1H); 8.10 (m, 3H); 8.05 (d, 1H); 7.90 (d, 1 H); 7.30 (s, 1 H); 7.25 (d, 2H); 7.10 (m, 1 H); 6.90 (d, 2H); 6.80 (d, 2H); 3.95 (m, 1 H); 3.85 (s, 3H); 3.80 (m, 1 H); 3.75 (m, 1 H); 3.45 (m, 4H); 3.05 (m, 4H); 2.95 (m, 1 H); 2.90 (m, 1 H); 1.40 (s, 9H); 0.80 (t, 3H).

### 29.4 1-(2,4-Dimethoxy-phenylsulfonyl)-3-(2-ethoxy-pyridin-3-yl)-2-oxo-3-(4-piperazin-1-yl-benzylamino)-2,3-dihydro-1 H-indol-5-carbonitril als Hydrochlorid

ESI-MS: 669.25 [M+H]⁺
¹H-NMR (500 MHz, d₆-DMSO): δ [ppm] 8.25 (d, 1 H); 8.10 (m, 1 H); 8.05 (d, 2H); 7.95 (d, 1 H); 7.45 (s, 1 H); 7.10 (m, 1 H); 6.95 (d, 2H); 6.85 (d, 2H); 6.80 (d, 1 H); 6.75 (s, 1 H); 4.05 (m, 2H); 3.85 (s, 3H); 3.65 (m, 1 H); 3.55 (s, 3H); 3.20 (bs, 4H); 3.05 (bs, 4H); 2.95 (m, 2H); 0.95 (t, 3H).

### BEISPIEL 30:

### 3-(2-Ethoxy-pyridin-3-yl)-1-(4-methoxy-phenylsulfonyl)-2-oxo-3-(4-piperazin-1-yl-benzylamino)-2,3-dihydro-1 H-indol-5-carbonitril

ESI-MS: 639.30 [M+H]⁺
¹H-NMR (500 MHz, d₆-DMSO): δ [ppm] 8.25 (d, 1H); 8.10 (m, 3H); 8.05 (d, 1H); 7.90 (d, 1 H); 7.35 (s, 1H); 7.25 (d, 2H); 7.10 (m, 1H); 6.90 (d, 2H); 6.80 (d, 1H); 3.95 (m, 1 H); 3.85 (s, 3H); 3.80 (m, 1 H); 3.75 (m, 1 H); 3.05 (bs, 4H); 2.95 (m, 6H); 0.80 (t, 3H).

### BEISPIEL 31:

### 3-(2-Ethoxy-pyridin-3-yl)-3-[4-(4-ethyl-piperazin-1-yl)-benzylamino]-1-(4-methoxy-phenylsulfonyl)-2-oxo-2,3-dihydro-1 H-indol-5-carbonitril

ESI-MS: 667.20 [M+H]⁺
¹H-NMR (500 MHz, ds-DMSO): δ [ppm] 8.25 (d, 1H); 8.10 (m, 3H); 8.05 (d, 1H); 7.90 (d, 1H); 7.35 (s, 1H); 7.25 (d, 2H); 7.10 (m, 1H); 6.90 (d, 2H); 6.80 (d, 1H); 3.95 (m, 1H); 3.85 (s, 3H); 3.80 (m, 1 H); 3.70 (m, 1 H); 3.10 (bs, 4H); 2.95 (m, 1 H); 2.85 (m, 1 H); 2.50 (bs, 4H); 2.40 (m, 2H); 1.05 (t, 3H); 0.80 (t, 3H).

### BEISPIEL 32:

### 1-(2,4-Dimethoxy-phenylsulfonyl)-3-(2-ethoxy-pyridin-3-yl)-3-[4-(4-ethyl-piperazin-1-yl)-benzylamino]-2-oxo-2,3-dihydro-1H-indol-5-carbonitril

ESI-MS: 697.20 [M+H]⁺

### BEISPIEL 33:

### 3-(2-Ethoxy-pyridin-3-yl)-6-fluor-1-(4-methoxy-phenylsulfonyl)-3-[4-(4-methyl-piperazin-1-yl)-benzylamino]-2-oxo-2,3-dihydro-1 H-indol-5-carbonitril

### 33.1 3-Chlor-3-(2-ethoxy-pyridin-3-yl)-6-fluor-2-oxo-2,3-dihydro-1H-indol-5-carbonitril

ESI-MS: 332.00 [M+H]⁺

### 33.2 3-(2-Ethoxy-pyridin-3-yl)-6-fluor-3-[4-(4-methyl-piperazin-1-yl)-benzylamino]-2-oxo-2,3-dihydro-1 H-indol-5-carbonitril

ESI-MS: 501.25 [M+H]⁺

### 33.3 3-(2-Ethoxy-pyridin-3-yl)-6-fluor-1-(4-methoxy-phenylsulfonyl)-3-[4-(4-methyl-piperazin-1-yl)-benzylamino]-2-oxo-2,3-dihydro-1H-indol-5-carbonitril

ESI-MS: 671.25 [M+H]⁺
¹H-NMR (500 MHz, CDCl₃): δ [ppm] 8.20 (d, 1 H); 8.15 (d, 2H); 8.10 (m, 1 H); 7.95 (d, 1 H); 7.10 (d, 1H); 7.05 (d, 2H); 6.95 (m, 1H); 6.85 (d, 2H); 6.80 (d, 2H); 4.15 (m, 1H); 4.10 (m, 1 H); 3.85 (s, 3H); 3.20 (m, 4H); 2.90 (m, 1 H); 2.80 (m, 1 H); 2.65 (m, 4H); 2.35 (s, 3H); 2.15 (m, 1H); 1.05 (t, 3H).

### BEISPIEL 34:

### 3-(2-Ethoxy-pyridin-3-yl)-6-fluor-1-(4-methoxy-phenylsulfonyl)-2-oxo-3-(4-piperazin-1-yl-benzylamino)-2,3-dihydro-1 H-indol-5-carbonitril

### 34.1 4-(4-{[5-Cyano-3-(2-ethoxy-pyridin-3-yl)-6-fluor-2-oxo-2,3-dihydro-1 H-indol-3-ylamino]-methyl}-phenyl)-piperazin-1-carbonsäure-tert-butylester

ESI-MS: 587.25 [M+H]⁺

### 34.2 4-(4-{[5-Cyano-3-(2-ethoxy-pyridin-3-yl)-6-fluor-1-(4-methoxy-phenylsulfonyl)-2-oxo-2,3-dihydro-1 H-indol-3-ylamino]-methyl}-phenyl)-piperazin-1-carbonsäure-tert-butylester

ESI-MS: 757.25 [M+H]⁺

### 34.3 3-(2-Ethoxy-pyridin-3-yl)-6-fluor-1-(4-methoxy-phenylsulfonyl)-2-oxo-3-(4-piperazin-1-yl-benzylamino)-2,3-dihydro-1 H-indol-5-carbonitril

ESI-MS: 657.25 [M+H]⁺
¹H-NMR (500 MHz, d₆-DMSO): δ [ppm] 8.25 (d, 1 H); 8.20 (d, 2H); 8.10 (m, 1 H); 7.90 (d, 1 H); 7.40 (d, 1 H); 7.25 (d, 2H); 7.10 (m, 1 H); 6.90 (d, 2H); 6.80 (d, 1 H); 3.95 (m, 1 H); 3.85 (s, 3H); 3.80 (m, 1 H); 3.75 (m, 1 H); 3.10 (m, 4H); 2.95 (m, 6H); 0.85 (t, 3H).

### BEISPIEL 35:

### 3-(2-Ethoxy-pyridin-3-yl)-3-[4-(4-ethyl-piperazin-1-yl)-benzylamino]-6-fluor-1-(4-methoxy-phenylsulfonyl)-2-oxo-2,3-dihydro-1H-indol-5-carbonitril

ESI-MS: 685.20 [M+H]⁺
¹H-NMR (500 MHz, d₆-DMSO): δ [ppm] 8.25 (d, 1H); 8.20 (d, 2H); 8.15 (m, 1H); 7.40 (d, 1 H); 7.25 (d, 2H); 7.10 (m, 1 H); 6.85 (d, 2H); 6.80 (d, 1 H); 3.95 (m, 1 H); 3.85 (m, 4H); 3.75 (m, 1 H); 3.10 (m, 4H); 3.00 (m, 1 H); 2.95 (m, 1 H); 2.50 (bs, 4H); 2.40 (m, 2H); 1.05 (t, 3H); 0.80 (t, 3H).

### BEISPIEL 36:

### 1-[4-(2,2-Difluor-ethoxy)-phenylsulfonyl]-3-(2-ethoxy-pyridin-3-y1)-6-fluor-3-[4-(4-methyl-piperazin-1-yl)-benzylamino]-2-oxo-2,3-dihydro-1H-indol-5-carbonitril

ESI-MS: 721.25 [M+H]⁺
¹H-NMR (500 MHz, d₆-DMSO): δ [ppm] 8.25 (m, 3H); 8.10 (m, 2H); 7.90 (d, 1 H); 7.40 (d, 1H); 7.35 (d, 2H); 7.15 (m, 1H); 6.85 (d, 2H); 6.75 (d, 2H); 6.40 (t, *J* = 70 Hz, 1H); 4.45 (m, 2H); 3.95 (m, 1 H); 3.85 (m, 1 H); 3.80 (m, 1 H); 3.05 (m, 4H); 3.00 (m, 1 H); 2.95 (m, 1 H); 2.45 (m, 4H); 2.25 (m, 3H); 0.85 (t, 3H).

### BEISPIEL 37:

### 3-(2-Ethoxy-phenyl)-1-(4-methoxy-phenylsulfonyl)-3-[4-(4-methyl-piperazin-1-yl)-benzylamino]-2-oxo-2,3-dihydro-1H-indol-5-carbonitril

ESI-MS: 652.20 [M+H]⁺
¹H-NMR (500 MHz, d₆-DMSO): δ [ppm] 8.15 (d, 2H); 8.10 (d, 1H); 8.00 (d, 1 H); 7.95 (d, 1H); 7.40 (t, 1H); 7.30 (m, 3H); 7.15 (t, 1H); 6.95 (d, 1H); 6.90 (d, 2H); 6.80 (d, 2H); 4.05 (m, 1 H); 3.90 (s, 3H); 3.80 (m, 1 H); 3.70 (m, 1 H); 3.15 (m, 4H); 2.95 (m, 1 H); 2.85 (m, 1 H); 2.50 (m, 4H); 2.30 (s, 3H); 0.95 (t, 3H).

### I.3.2 Verbindungen I, worin X¹ für -NH-CH₂- und A für 1,3-Phenylen stehen

### BEISPIEL 38:

### 1-(2,4-Dimethoxy-phenylsulfonyl)-3-(2-ethoxy-pyridin-3-yl)-5-iod-3-[3-(4-methyl-piperazin-1-yl)-benzylamino]-1,3-dihydro-indol-2-on

### 38.1 3-(2-Ethoxy-pyridin-3-yl)-5-iod-3-[3-(4-methyl-piperazin-1-yl)-benzylamino]-1,3-dihydro-indol-2-on

ESI-MS: 584.15 [M+H]⁺

### 38.2 1-(2,4-Dimethoxy-phenylsulfonyl)-3-(2-ethoxy-pyridin-3-yl)-5-iod-3-[3-(4-methylpiperazin-1-yl)-benzylamino]-1,3-dihydro-indol-2-on

ESI-MS: 784.15 [M+H]⁺
¹H-NMR (500 MHz, d₆-DMSO): δ [ppm] 8.20 (m, 2H); 8.10 (m, 1 H); 7.80 (d, 1 H); 7.70 (d, 1H); 7.30 (d, 1H); 7.15 (m, 1H); 6.90 (m, 1H); 6.80 (d, 1H); 6.65 (d, 1H) ;6.60 (m,2H); 6.40 (s, 1H); 4.25 (m, 2H); 3.85 (s, 3H); 3.55 (s, 3H); 3.20 (m, 4H); 3.10 (m, 1H); 2.90 (m, 1 H); 2.60 (m, 4H); 2.40 (s, 3H); 2.15 (m, 1 H); 1.20 (t, 3H).

### BEISPIEL 39:

### 3-(2-Ethoxy-pyridin-3-yl)-5-iod-1-(4-methoxy-2-trifluormethoxy-phenylsulfonyl)-3-[3-(4-methyl-piperazin-1-yl)-benzylamino]-1,3-dihydro-indol-2-on

ESI-MS: 838.10 [M+H]⁺
¹H-NMR (500 MHz, d₆-DMSO): δ [ppm] 8.35 (d, 1H); 8.20 (d, 1H); 8.10 (m, 1H); 7.80 (d, 1H); 7.70 (d, 1H); 7.25 (s, 1H); 7.15 (m, 1 H); 6.95 (m, 2H); 6.80 (m, 2H); 6.60 (d, 1 H); 6.55 (d, 1 H); 4.25 (m, 2H); 3.90 (s, 3H); 3.20 (m, 5H); 3.00 (m, 1H); 2.60 (m, 4H); 2.35 (s, 3H); 2.10 (m, 1H); 1.15 (t, 3H).

### BEISPIEL 40:

### 3-(2-Ethoxy-pyridin-3-yl)-5-iod-1-(4-methoxy-phenylsulfonyl)-3-[3-(4-methyl-piperazin-1-yl)-benzylamino]-1,3-dihydro-indol-2-on

ESI-MS: 754.15 [M+H]⁺
¹H-NMR (500 MHz, d₆-DMSO): δ [ppm] 8.20 (d, 1H); 8.15 (m, 3H); 7.80 (d, 1H); 7.70 (d, 1 H); 7.20 (s, 1 H); 7.15 (m, 1 H); 7.00 (d, 2H); 6.95 (m, 1 H); 6.60 (d, 1 H); 6.55 (m, 2H); 4.10 (m, 2H); 3.85 (s, 3H); 3.20 (m, 4H); 2.95 (m, 1 H); 2.75 (m, 1 H); 2.60 (m, 4H); 2.35 (s, 3H); 2.15 (m, 1H); 1.10 (t, 3H).

### BEISPIEL 41:

### 3-(2-Ethoxy-pyridin-3-yl)-5-iod-1-(2-methoxy-phenylsulfonyl)-3-[3-(4-methyl-piperazin-1-yl)-benzylamino]-1,3-dihydro-indol-2-on

ESI-MS: 754.15 [M+H]⁺
¹H-NMR (500 MHz, d₆-DMSO): δ [ppm] 8.30 (d, 1H); 8.15 (d, 1H); 8.10 (m, 1H); 7.85 (d, 1H); 7.70 (d, 1H); 7.60 (m, 1H); 7.30 (d, 1H); 7.15 (m, 2H); 6.90 (m, 2H); 6.80 (d, 1H); 6.55 (s, 1H); 6.50(d, 1 H); 4.25 (m, 2H); 3.60 (s, 3H); 3.15 (m, 4H); 3.10 (m, 1H); 2.90 (m, 1 H); 2.60 (m, 4H); 2.40 (s, 3H); 2.15 (m, 1 H); 1.20 (t, 3H).

### BEISPIEL 42:

### 1-(2,4-Dimethoxy-phenylsulfonyl)-3-(2-ethoxy-pyridin-3-yl)-3-[3-(4-methyl-piperazin-1-yl)-benzylamino]-2-oxo-2,3-dihydro-1 H-indol-5-carbonitril

### 42.1 3-(2-Ethoxy-pyridin-3-yl)-3-[3-(4-methyl-piperazin-1-yl)-benzylamino]-2-oxo-2,3-dihydro-1H-indol-5-carbonitril

ESI-MS: 483.20 [M+H]⁺

### 42.2 1-(2,4-Dimethoxy-phenylsulfonyl)-3-(2-ethoxy-pyridin-3-yl)-3-[3-(4-methyl-piperazin-31-yl)-benzylamino]-2-oxo-2,3-dihydro-1H-indol-5-carbonitril

ESI-MS: 683.10 [M+H]⁺
¹H-NMR (500 MHz, d₆-DMSO): δ [ppm] 8.35 (d, 1H); 8.20 (m, 1H); 8.10 (m, 2H); 8.00 (d, 1H); 7.50 (s, 1H); 7.15 (m, 2H); 6.90 (d, 1H); 6.85 (m, 2H); 6.65 (m, 2H); 4.15 (m, 2H); 3.95 (s, 3H); 3.80 (m, 1H); 3.65 (s, 3H); 3.15 (bs, 4H); 3.10 (m, 1H); 3.00 (m, 1H); 2.50 (m, 4H); 2.30 (s, 3H); 1.05 (t, 3H).

### BEISPIEL 43:

### 3-(2-Ethoxy-pyridin-3-yl)-1-(4-methoxy-phenylsulfonyl)-3-[3-(4-methyl-piperazin-1-yl)-benzylamino]-2-oxo-2,3-dihydro-1 H-indol-5-carbonitril

ESI-MS: 653.10 [M+H]⁺
¹H-NMR (500 MHz, d₆-DMSO): δ [ppm] 8.35 (d, 1H); 8.20 (m, 3H); 8.10 (d, 1H); 7.95 (d, 1H); 7.45 (s, 1H); 7.30 (d, 2H); 7.20 (m, 1H); 7.15 (t, 1H); 6.80 (d, 1H); 6.60 (m, 2H); 4.05 (m, 1 H); 3.95 (m, 1 H); 3.90 (m, 4H); 3.15 (m, 4H); 3.05 (m, 1 H); 2.95 (m, 1 H); 2.50 (m, 4H); 2.30 (s, 3H); 0.90 (t, 3H).

### BEISPIEL 44:

### 3-(2-Ethoxy-pyridin-3-yl)-1-(2-methoxy-phenylsulfonyl)-3-[3-(4-methyl-piperazin-1-yl)-benzylamino]-2-oxo-2,3-dihydro-1 H-indol-5-carbonitril

ESI-MS: 653.10 [M+H]⁺
¹H-NMR (500 MHz, d₆-DMSO): δ [ppm] 8.35 (d, 1H); 8.20 (m, 2H); 8.10 (d, 1H); 8.00 (d, 1 H); 7.85 (m, 1 H); 7.55 (s, 1 H); 7.35 (d, 1 H); 7.30 (m, 1 H); 7.15 (m, 2H); 6.80 (d, 1 H); 6.65 (s, 1H); 6.60 (d, 1H); 4.15 (m, 2H); 3.85 (m, 1H); 3.70 (s, 3H); 3.15 (bs, 4H); 3.10 (m, 1H); 3.00 (m, 1H); 2.50 (m, 4H); 2.25 (s, 3H); 1.05 (t, 3H).

### I.3.3 Verbindungen I, worin X¹ für-NH-CH₂- und A für 1,2-Phenylen stehen

### BEISPIEL 45:

### 1-(2,4-Dimethoxy-phenylsulfonyl)-3-(2-ethoxy-pyridin-3-yl)-3-[2-(4-methyl-piperazin-1-yl)-benzylamino]-2-oxo-2,3-dihydro-1H-indol-5-carbonitril

### 45.1 3-(2-Ethoxy-pyridin-3-yl)-3-[2-(4-methyl-piperazin-1-yl)-benzylamino]-2-oxo-2,3-dihydro-1H-indol-5-carbonitril

ESI-MS: 483.20 [M+H]⁺

### 45.2 1-(2,4-Dimethoxy-phenylsulfonyl)-3-(2-ethoxy-pyridin-3-yl)-3-[2-(4-methyl-piperazin-1-yl)-benzylamino]-2-oxo-2,3-dihydro-1H-indol-5-carbonitril

ESI-MS: 683.15 [M+H]⁺
¹H-NMR (500 MHz, d₆-DMSO): δ [ppm] 8.20 (d, 2H); 8.10 (d, 2H); 7.75 (d, 1 H); 7.25 (t, 1 H); 7.20 (s, 1 H); 7.10 (d, 1 H); 7.00 (t, 1 H); 6.90 (t, 2H); 6.85 (d, 1 H); 6.60 (d, 1 H); 6.25 (d, 1 H); 4.20 (m, 2H); 3.75 (s, 3H); 3.55 (s, 3H); 3.20 (m, 1 H); 3.10 (m, 1 H); 3.05 (m, 1 H); 2.90 (bs, 2H); 2.75 (m, 2H); 2.35 (m, 4H); 2.30 (s, 3H); 1.15 (t, 3H).

### BEISPIEL 46:

### 3-(2-Ethoxy-pyridin-3-yl)-1-(4-methoxy-phenylsulfonyl)-3-[2-(4-methyl-piperazin-1-yl)-benzylamino]-2-oxo-2,3-dihydro-1 H-indol-5-carbonitril

ESI-MS: 653.10 [M+H]⁺
¹H-NMR (500 MHz, d₆-DMSO): δ [ppm] 8.20 (m, 1H); 8.10 (m, 3H); 8.05 (d, 1 H); 7.95 (d, 1 H); 7.30 (m, 2H); 7.20 (m, 3H); 7.10 (t, 1 H); 7.00 (m, 2H); 3.95 (m, 1 H); 3.90 (m, 1 H); 3.80 (s, 3H); 3.65 (m, 1 H); 3.25 (m, 1 H); 3.15 (m, 1 H); 2.70 (m, 2H); 2.60 (m, 2H); 2.25 (m, 4H); 2.20 (s, 3H); 0.70 (t, 3H).

### BEISPIEL 47:

### 3-(2-Ethoxy-pyridin-3-yl)-1-(2-methoxy-phenylsulfonyl)-3-[2-(4-methyl-piperazin-1-yl)-benzylamino]-2-oxo-2,3-dihydro-1 H-indol-5-carbonitril

ESI-MS: 653.10 [M+H]⁺
¹H-NMR (500 MHz, d₆-DMSO): δ [ppm] 8.30 (d, 1H); 8.25 (d, 1H); 8.10 (m, 2H); 7.75 (d, 1 H); 7.55 (t, 1 H); 7.25 (m, 2H); 7.15 (t, 1 H); 7.10 (d, 1 H); 7.00 (t, 1 H); 6.90 (m, 1 H); 6.80 (d, 2H); 4.20 (m, 2H); 3.60 (s, 3H); 3.25 (m, 1 H); 3.05 (m, 2H); 2.85 (m, 2H); 2.70 (m, 2H); 2.35 (m, 4H); 2.30 (s, 3H); 1.15 (t, 3H).

### I.4 Herstellung der Verbindungen I, worin X¹ für -NH- steht

### 1.4.1 Verbindungen I, worin X¹ für -NH-, A für 1,4-Phenylen, X² für eine Einfachbindung, X³ für N, n für 2 und m für 2 stehen

### BEISPIEL 48:

### 1-(2,4-Dimethoxy-phenylsulfonyl)-3-(2-ethoxy-pyridin-3-yl)-3-[4-(4-ethyl-piperazin-1-yl)-phenylamino]-5-iod-1,3-dihydro-indol-2-on

### 48.1 1-(2,4-Dimethoxy-phenylsulfonyl)-3-(2-ethoxy-pyridin-3-yl)-3-hydroxy-5-iod-1,3-dihydro-indol-2-on

Man gab Natriumhydrid (145 mg einer 50%igen Dispersion in Mineralöl, 3.03 mmol) zu einer eisgekühlten Lösung von 3-(2-Ethoxy-pyridin-3-yl)-3-hydroxy-5-iod-1,3-dihydro-indol-2-on (1.0 g, 2.52 mmol) in DMF (20 ml). Man rührte die Reaktionsmischung 15 Minuten bei 0 °C und gab danach 2,4-Dimethoxy-phenylsulfonylchlorid (99 mg, 0.48 mmol) zu. Man rührte die Reaktionsmischung 2 Stunden bei Raumtemperatur, versetzte mit Wasser und filtrierte den erhaltenen Niederschlag ab. Man saugte den nach Rühren in Ethylether (50 ml) über Nacht gebildeten Niederschlag ab, wobei man 730 mg der Titelverbindung (45 % Ausbeute) erhielt.
ESI-MS: 597.05 [M+H]⁺

### 48.2 3-Chlor-1-(2,4-dimethoxy-phenylsulfonyl)-3-(2-ethoxy-pyridin-3-yl)-5-iod-1,3-dihydro-indol-2-on

Zu einer eisgekühlten Lösung von 1-(2,4-Dimethoxy-phenylsulfonyl)-3-(2-ethoxy-pyridin-3-yl)-3-hydroxy-5-iod-1,3-dihydro-indol-2-on (100 mg, 0.15 mmol) in Dichlormethan (30 ml) gab man sukzessive Pyridin (40 µl, 0.46 mmol) und Thionylchlorid (35 µl, 0.46 mmol). Man ließ die Reaktionsmischung auf Raumtemperatur erwärmen und rührte 3 Stunden. Man gab Eiswasser zu der Reaktionsmischung und extrahierte die wässrige Phase mit Dichlormethan. Die organiche Phase wurde über Magnesiumsulfat getrocknet, filtriert und unter vermindertem Druck eingeengt. Das erhaltene rohe Produkt (110 mg) wurde ohne weitere Aufreinigung im nächsten Schritt eingesetzt.
ESI-MS: 614.90 [M+H]⁺

### 48.3 1-(2,4-Dimethoxy-phenylsulfonyl)-3-(2-ethoxy-pyridin-3-yl)-3-[4-(4-ethyl-piperazin-1-yl)-phenylamino]-5-iod-1,3-dihydro-indol-2-on

Man gab 4-(4-Ethyl-piperazin-1-yl)-phenylamin (44 mg, 0.21 mmol) zu einer Lösung von 3-Chlor-1-(2,4-dimethoxy-phenylsulfonyl)-3-(2-ethoxy-pyridin-3-yl)-5-iod-1,3-dihydro-indol-2-on (110 mg, 0.18 mmol) in Dichlormethan (15 ml). Die Reaktionsmischung wurde in einem Biotage Microwellen-Fläschen 4 Stunden bei 120 °C gerührt. Man engte die Reaktionsmischung unter vermindertem Druck ein. Nach chromatographischer Reinigung (Kieselgel, 0-8% Methanol in Dichloromethan) erhielt man 16 mg der Titelverbindung (11 % Ausbeute).
ESI-MS: 784.20 [M+H]⁺
¹H-NMR (500 MHz, d₆-DMSO): δ [ppm] 8.15 (m, 1 H); 8.05 (d, 1 H); 7.85 (d, 1 H); 7.75 (d, 1 H); 7.50 (d, 1 H); 7.30 (s, 1 H), 7.10 (dd, 1 H); 6.70 (d, 1 H); 6.65 (s, 1 H); 6.45 (d, 2H); 6.40 (d, 2H); 4.05 (m, 2H); 3.85 (s, 3H); 3.55 (s, 3H); 2.95 (bs, 4H); 2.45 (bs, 4H); 2.35 (m, 2H); 1.00 (t, 3H); 0.90 (t, 3H).

### BEISPIEL 49:

### 1-(2,4-Dimethoxy-phenylsulfonyl)-3-(2-ethoxy-pyridin-3-yl)-5-iod-3-[4-(4-methyl-piperazin-1-yl)-phenylamino]-1,3-dihydro-indol-2-on

### 49.1 3-(2-Ethoxy-pyridin-3-yl)-5-iod-3-[4-(4-methyl-piperazin-1-yl)-phenylamino]-1,3-dihydro-indol-2-on

ESI-MS: 570.15 [M+H]⁺

### 49.2 1-(2,4-Dimethoxy-phenylsulfonyl)-3-(2-ethoxy-pyridin-3-yl)-5-iod-3-[4-(4-methyl-piperazin-1-yl)-phenylamino]-1,3-dihydro-indol-2-on

ESI-MS: 770.70 [M+H]^{+ 1}H-NMR (500 MHz, CDCl₃): δ [ppm] 8.25 (d, 1 H); 8.15 (m, 1 H); 8.05 (d, 1 H); 7.60 (m, 2H); 7.45 (s, 1 H); 6.95 (m, 1 H); 6.55 (d, 1 H); 6.35 (m, 4H); 6.25 (m, 1 H); 4.25 (m, 2H); 3.90 (s, 3H); 3.35 (s, 3H); 3.05 (m, 4H); 2.55 (m, 4H); 2.35 (s, 3H); 1.20 (t, 3H).

### BEISPIEL 50:

### 3-(2-Ethoxy-pyridin-3-yl)-5-iod-1-(4-methoxy-phonylsulfonyl)-3-[4-(4-methyl-piperazin-1-yl)-phenylamino]-1,3-dihydro-indol-2-on

ESI-MS: 740.15 [M+H]⁺
¹H-NMR (500 MHz, d₆-DMSO): δ [ppm] 8.35 (d, 1 H); 8.15 (m, 1 H); 7.85 (d, 2H); 7.75 (d, 1H); 7.60 (d, 1H); 7.35 (s, 1H); 7.10 (m, 1H); 7.05 (d, 1H); 6.45 (d, 2H); 6.35 (d, 2H); 4.15 (m, 1 H); 4.00 (m, 1 H); 3.95 (s, 3H); 3.05 (m, 4H); 2.60 (m, 4H); 2.35 (s, 3H); 0.95 (t, 3H).

### BEISPIEL 51:

### 5-Chlor-3-(2-ethoxy-pyridin-3-yl)-1-(4-methoxy-phenylsulfonyl)-3-[4-(4-methyl-piperazin-1-yl)-phenylamino]-1,3-dihydro-indol-2-on

### 51.1 5-Chlor-3-(2-ethoxy-pyridin-3-yl)-3-[4-(4-methyl-piperazin-1-yl)-phenylamino]-1,3-dihydro-indol-2-on

ESI-MS: 478.20 [M+H]⁺

### 51.2 5-Chlor-3-(2-ethoxy-pyridin-3-yl)-1-(4-methoxy-phenylsulfonyl)-3-[4-(4-methylpiperazin-1-yl)-phenylamino]-1,3-dihydro-indol-2-on

ESI-MS: 648.15 [M+H]⁺
¹H-NMR (500 MHz, CDCl₃): δ [ppm] 8.25 (d, 1H); 8.15 (m, 1H); 7.80 (d, 2H); 7.75 (d, 1 H); 7.30 (m, 1H); 7.10 (s, 1H); 7.00 (m, 1H); 6.90 (d, 2H); 6.45 (d, 2H); 6.35 (d, 2H); 4.15 (m, 1 H); 4.10 (m, 1 H); 4.00 (s, 1 H); 3.90 (s, 3H); 3.05 (m, 4H); 2.55 (m, 4H); 2.35 (s, 3H); 1.05 (t, 3H).

### BEISPIEL 52:

### 5-Chlor-3-(2-ethoxy-pyridin-3-yl)-6-fluor-1-(4-methoxy-phenylsulfonyl)-3-[4-(4-methylpiperazin-1-yl)-phenylamino]-1,3-dihydro-indol-2-on

### 52.1 5-Chlor-3-(2-ethoxy-pyridin-3-yl)-6-fluor-3-[4-(4-methyl-piperazin-1-yl)-phenylamino]-1,3-dihydro-indol-2-on

ESI-MS: 496.20 [M+H]⁺

### 52.2 5-Chlor-3-(2-ethoxy-pyridin-3-yl)-6-fluor-1-(4-methoxy-phenylsulfonyl)-3-[4-(4-methyl-piperazin-1-yl)-phenylamino]-1,3-dihydro-indol-2-on

ESI-MS: 666.20 [M+H]⁺
¹H-NMR (400 MHz, CDCl₃): δ [ppm] 8.25 (d, 1H); 8.15 (m, 1H); 7.80 (d, 2H); 7.65 (d, 1H); 7.10 (d, 1H); 7.00 (t, 1H); 6.95 (d, 2H); 6.35 (d, 2H); 6.25 (d, 2H); 4.15 (m, 2H); 3.95 (s, 1 H); 3.90 (s, 3H); 3.05 (m, 4H); 2.55 (m, 4H); 2.35 (s, 3H); 1.05 (t, 3H).

### BEISPIEL 53:

### 6-Chlor-3-(2-ethoxy-pyridin-3-yl)-5-fluor-1-(4-methoxy-phenylsulfonyl)-3-[4-(4-methylpiperazin-1-yl)-phenylamino]-1,3-dihydro-indol-2-on

### 53.1 6-Chlor-3-(2-ethoxy-pyridin-3-yl)-5-fluor-3-[4-(4-methyl-piperazin-1-yl)-phenylamino]-1,3-dihydro-indol-2-on

ESI-MS: 496.20 [M+H]⁺

### 53.2 6-Chlor-3-(2-ethoxy-pyridin-3-yl)-5-fluor-1-(4-methoxy-phenylsulfonyl)-3-[4-(4-methyl-piperazin-1-yl)-phenylamino]-1,3-dihydro-indol-2-on

ESI-MS: 666.20 [M+H]⁺
¹H-NMR (400 MHz, CDCl₃): δ [ppm] 8.25 (d, 1 H); 8.15 (m, 1 H); 7.90 (d, 1 H); 7.85 (d, 2H); 6.95 (m, 1 H); 6.90 (m, 3H); 6.35 (d, 2H); 6.25 (d, 2H); 4.15 (m, 2H); 3.95 (s, 1 H); 3.90 (s, 3H); 3.05 (m, 4H); 2.55 (m, 4H); 2.35 (s, 3H); 1.05 (t, 3H).

### BEISPIEL 54:

### 3-(2-Ethoxy-pyridin-3-yl)-5,6-difluor-1-(4-methoxy-phenylsulfonyl)-3-[4-(4-methyl-piperazin-1-yl)-phenylamino]-1,3-dihydro-indol-2-on

### 54.1 3-(2-Ethoxy-pyridin-3-yl)-5,6-difluor-3-[4-(4-methyl-piperazin-1-yl)-phenylamino]-1,3-dihydro-indol-2-on

ESI-MS: 480.20 [M+H]⁺

### 54.2 3-(2-Ethoxy-pyridin-3-yl)-5,6-difluor-1-(4-methoxy-phenylsulfonyl)-3-[4-(4-methyl-piperazin-1-yl)-phenylamino]-1,3-dihydro-indol-2-on

ESI-MS: 650.25 [M+H]⁺
¹H-NMR (400 MHz, CDCl₃): δ [ppm] 8.25 (d, 1 H); 8.15 (m, 1 H); 7.80 (d, 2H); 7.70 (m, 1 H); 6.95 (m, 4H); 6.35 (d, 2H); 6.25 (d, 2H); 4.15 (m, 2H); 3.95 (s, 1 H); 3.90 (s, 3H); 3.05 (m, 4H); 2.55 (m, 4H); 2.35 (s, 3H); 1.05 (t, 3H).

### BEISPIEL 55:

### 1-(2,4-Dimethoxy-phenylsulfonyl)-3-(2-ethoxy-pyridin-3-yl)-3-[4-(4-methyl-piperazin-1-yl)-phenylamino]-2-oxo-2,3-dihydro-1H-indol-5-carbonitril

### 55.1 3-(2-Ethoxy-pyridin-3-yl)-3-[4-(4-methyl-piperazin-1-yl)-phenylamino]-2-oxo-2,3-dihydro-1H-indol-5-carbonitril

ESI-MS: 469.25 [M+H]⁺

### 55.2 1-(2,4-Dimethoxy-phenylsulfonyl)-3-(2-ethoxy-pyridin-3-yl)-3-[4-(4-methyl-piperazin-1-yl)-phenylamino]-2-oxo-2,3-dihydro-1H-indol-5-carbonitril

ESI-MS: 669.25 [M+H]⁺
¹H-NMR (500 MHz, d₆-DMSO): δ [ppm] 8.30 (d, 1 H); 8.15 (m, 1 H); 8.05 (d, 1 H); 7.95 (d, 1 H); 7.60 (d, 1 H); 7.45 (s, 1 H); 7.00 (m, 1 H); 6.55 (d, 1 H); 6.30 (m, 5H); 4.25 (m, 2H); 3.90 (s, 3H); 3.35 (s, 3H); 3.05 (m, 4H); 2.55 (m, 4H); 2.35 (s, 3H); 1.15 (t, 3H).

### BEISPIEL 56:

### 1-(2,4-Dimethoxy-phenylsulfonyl)-3-(2-ethoxy-pyridin-3-yl)-3-[4-(4-ethyl-piperazin-1-yl)-phenylamino]-2-oxo-2,3-dihydro-1 H-indol-5-carbonitril

### 56.1 3-(2-Ethoxy-pyridin-3-yl)-3-[4-(4-ethyl-piperazin-1-yl)-phenylamino]-2-oxo-2,3-dihydro-1 H-indol-5-carbonitril

ESI-MS: 483.30 [M+H]⁺

### 56.2 1-(2,4-Dimethoxy-phenylsulfonyl)-3-(2-ethoxy-pyridin-3-yl)-3-[4-(4-ethyl-piperazin-1-yl)-phenylamino]-2-oxo-2,3-dihydro-1 H-indol-5-carbonitril

ESI-MS: 683.30 [M+H]⁺
¹H-NMR (500 MHz, CDCl₃): δ [ppm] 8.30 (d, 1 H); 8.15 (m, 1 H); 8.05 (d, 1 H); 7.95 (d, 1 H); 7.60 (d, 1 H); 7.45 (s, 1 H); 7.00 (m, 1 H); 6.60 (d, 1 H); 6.30 (m, 5H); 4.25 (m, 2H); 4.00 (m, 1H); 3.90 (s, 3H); 3.30 (s, 3H); 3.10 (m, 4H); 2.65 (m, 4H); 2.55 (m, 2H); 1.15 (m, 6H).

### BEISPIEL 57:

### 3-(2-Ethoxy-pyridin-3-yl)-3-[4-(4-ethyl-piperazin-1-yl)-phenylamino]-1-(4-methoxy-phenylsulfonyl)-2-oxo-2,3-dihydro-1H-indol-5-carbonitril

ESI-MS: 653.30 [M+H]⁺
¹H-NMR (500 MHz, CDCl₃): δ [ppm] 8.30 (d, 1 H); 8.15 (m, 1 H); 7.95 (d, 1 H); 7.90 (m, 2H); 7.60 (d, 1 H); 7.40 (s, 1 H); 7.00 (t, 1 H); 6.95 (d, 2H); 6.35 (d, 2H); 6.25 (d, 2H); 4.15 (m, 1 H); 4.05 (m, 1 H); 3.90 (s, 3H); 3.80 (m, 1 H); 3.30 (s, 3H); 3.10 (m, 4H); 2.65 (m, 4H); 2.55 (m, 2H); 1.15 (t, 3H); 0.95 (t, 3H).

### BEISPIEL 58:

### 3-(2-Ethoxy-pyridin-3-yl)-3-[4-(4-ethyl-piperazin-1-yl)-phenylamino]-1-(2-methoxy-phenylsulfonyl)-2-oxo-2,3-dihydro-1 H-indol-5-carbonitril

ESI-MS: 653.30 [M+H]⁺
¹H-NMR (500 MHz, CDCl₃): δ [ppm] 8.30 (d, 1 H); 8.15 (m, 1 H); 8.10 (d, 1 H); 7.95 (d, 1 H); 7.65 (d, 1 H); 7.55 (t, 1 H); 7.45 (s, 1 H); 7.10 (t, 2H); 7.00 (m, 1H); 6.85 (d, 1 H); 6.30 (m, 4H); 4.25 (m, 2H); 3.95 (s, 1 H); 3.80 (m, 1 H); 3.35 (s, 3H); 3.15 (bs, 4H); 2.65 (bs, 4H); 2.55 (m, 2H); 1.15 (m, 6H).

### BEISPIEL 59:

### 3-(2-Ethoxy-pyridin-3-yl)-6-fluor-1-(4-methoxy-phenylsulfonyl)-3-[4-(4-methyl-piperazin-1-yl)-phenylamino]-2-oxo-2,3-dihydro-1 H-indol-5-carbonitril

### 59.1 3-(2-Ethoxy-pyridin-3-yl)-6-fluor-3-[4-(4-methyl-piperazin-1-yl)-phenylamino]-2-oxo-2,3-dihydro-1 H-indol-5-carbonitril

ESI-MS: 487.25 [M+H]⁺

### 59.2 3-(2-Ethoxy-pyridin-3-yl)-6-fluor-1-(4-methoxy-phenylsulfonyl)-3-[4-(4-methyl-piperazin-1-yl)-phenylamino]-2-oxo-2,3-dihydro-1H-indol-5-carbonitril

ESI-MS: 657.25 [M+H]⁺
¹H-NMR (500 MHz, d₆-DMSO): δ [ppm] 8.15 (m, 2H); 8.00 (d, 2H); 7.80 (d, 1 H); 7.70 (d, 1 H); 7.15 (m, 3H); 6.45 (d, 2H); 6.35 (d, 2H); 6.00 (s, 1 H); 3.95 (m, 1 H); 3.90 (s, 3H); 3.80 (m, 1H); 2.95 (bs, 4H); 2.45 (bs, 4H); 2.25 (s, 3H); 0.75 (m, 6H).

### I.4.2 Verbindungen I, worin X¹ für -NH-, A für 1,3-Phenylen, X² für eine Einfachbindung, X³ für N, n für 2 und m für 2 stehen

### BEISPIEL 60:

### 1-(2,4-Dimethoxy-phenylsulfonyl)-3-(2-ethoxy-pyridin-3-yl)-5-iod-3-[3-(4-methyl-piperazin-1-yl)-phenylamino]-1,3-dihydro-indol-2-on

### 60.1 3-(2-Ethoxy-pyridin-3-yl)-5-iod-3-[3-(4-methyl-piperazin-1-yl)-phenylamino]-1,3-dihydro-indol-2-on

ESI-MS: 570.15 [M+H]⁺
¹H-NMR (500 MHz, CDCl₃): δ [ppm] 8.20 (d, 1H); 8.15(m, 1H); 7.55 (d, 1H); 7.40 (s, 1 H); 7.00 (m, 2H); 6.55 (d, 1H); 6.45 (d, 1H); 6.25 (d, 1H); 6.15 (s, 1H); 4.25 (m, 2H); 2.50 (bs, 4H); 2.30 (s, 3H); 2.05 (bs, 3H); 1.15 (t, 3H).

### 60.2 1-(2,4-Dimethoxy-phenylsulfonyl)-3-(2-ethoxy-pyridin-3-yl)-5-iod-3-[3-(4-methylpiperazin-1-yl)-phenylamino]-1,3-dihydro-indol-2-on

ESI-MS: 770.10 [M+H]⁺
¹H-NMR (500 MHz, d₆-DMSO): δ [ppm] 8.15 (m, 1 H); 7.95 (d, 1 H); 7.90 (d, 1 H); 7.75 (d, 1 H); 7.50 (d, 1 H); 7.30 (s, 1 H); 7.05 (d, 1 H); 6.70 (m, 2H); 6.65 (s, 1 H); 6.25 (m, 2H); 6.20 (s, 1 H); 6.00 (d, 1 H); 4.05 (m, 2H); 3.85 (s, 3H); 3.55 (s, 3H); 2.85 (m, 4H); 2.35 (bs, 4H); 2.20 (bs, 3H); 0.90 (t, 3H).

### BEISPIEL 61:

### 3-(2-Ethoxy-pyridin-3-yl)-1-(4-methoxy-phenylsulfonyl)-3-[3-(4-methyl-piperazin-1-yl)-phenylamino]-2-oxo-2,3-dihydro-1 H-indol-5-carbonitril

### 61.1 3-(2-Ethoxy-pyridin-3-yl)-3-[3-(4-methyl-piperazin-1-yl)-phenylamino]-2-oxo-2,3-dihydro-1 H-indol-5-carbonitril

ESI-MS: 469.25 [M+H]⁺

### 61.2 3-(2-Ethoxy-pyridin-3-yl)-1-(4-methoxy-phenylsulfonyl)-3-[3-(4-methyl-piperazin-1-yl)-phenylamino]-2-oxo-2,3-dihydro-1 H-indol-5-carbonitril

ESI-MS: 639.25 [M+H]⁺
¹H-NMR (500 MHz, d₆-DMSO): δ [ppm] 8.15 (m, 1H); 8.05 (d, 1 H); 7.95 (m, 4H); 7.55 (s, 1H); 7.15 (d, 1 H); 7.10 (m, 2H); 6.70 (t, 1 H); 6.30 (s, 1 H); 6.25 (m, 1H); 6.10 (s, 1 H); 5.95 (d, 1 H); 3.95 (m, 1 H); 3.85 (s, 3H); 3.65 (m, 1 H); 2.85 (m, 4H); 2.35 (bs, 4H); 2.20 (s, 3H); 0.65 (t, 3H).

### BEISPIEL 62:

### 1-(2,4-Dimethoxy-phenylsulfonyl)-3-(2-ethoxy-pyridin-3-yl)-3-[3-(4-methyl-piperazin-1-yl)-phenylamino]-2-oxo-2,3-dihydro-1 H-indol-5-carbonitril

ESI-MS: 669.25 [M+H]⁺
¹H-NMR (500 MHz, d₆-DMSO): δ [ppm] 8.15 (m, 1H); 8.05 (d, 1 H); 7.90 (m, 3H); 7.55 (s, 1H); 7.10 (m, 1 H); 6.70 (m, 2H); 6.65 (m, 1 H); 6.30 (m, 2H); 6.20 (s, 1 H); 5.95 (d, 1 H); 4.05 (m, 2H); 3.85 (s, 3H); 3.55 (s, 3H); 2.85 (m, 4H); 2.35 (bs, 4H); 2.20 (s, 3H); 0.85 (t, 3H).

### I.4.3 Verbindungen I, worin X¹ für -NH-, A für 1,4-Phenylen, X² für eine Einfachbindung, X³ für CH, n für 1 und m für 1 stehen

### BEISPIEL 63:

### 3-(4-Azetidin-3-yl-phenylamino)-1-(2,4-dimethoxy-phenylsulfonyl)-3-(2-ethoxy-pyridin-3-yl)-5-iod-1,3-dihydro-indol-2-on als Hydrochlorid

### 63.1 3-{4-[3-(2-Ethoxy-pyridin-3-yl)-5-iod-2-oxo-2,3-dihydro-1H-indol-3ylamino]-phenyl}-azetidine-1-carbonsäure-tert-butylester

ESI-MS: 626.90 [M+H]⁺

### 63.2 3-{4-[1-(2,4-Dimethoxy-phenylsulfonyl)-3-(2-ethoxy-pyridin-3-yl)-5-iod-2-oxo-2,3-dihydro-1H-indol-3ylamino]-phenyl}-azetidin-1-carbonsäure-tert-butylester

### 63.3 3-(4-Azetidin-3-yl-phenylamino)-1-(2,4-dimethoxy-phenylsulfonyl)-3-(2-ethoxy-pyridin-3-yl)-5-iod-1,3-dihydro-indol-2-on als Hydrochlorid

ESI-MS: 765.00 [M+H]⁺
¹H-NMR (500 MHz, CDCl3): δ [ppm] 9.75 (bs, 1H); 9.45 (bs, 1H); 8.15 (d, 1H); 8.00 (d, 1 H); 7.80 (d, 1 H); 7.65 (d, 1 H); 7.60 (d, 1 H), 7.35 (s, 1 H); 7.00 (m, 2H); 6.90 (t, 1 H); 6.55 (m, 3H); 6.45 (m, 1 H); 4.55 (m, 1 H); 4.30-4.00 (m, 7H); 3.85 (s, 3H); 3.55 (s, 3H); 1.15 (t, 3H).

### BEISPIEL 64:

### 1-(2,4-Dimethoxy-phenylsulfonyl)-3-(2-ethoxy-pyridin-3-yl)-3-[4-(1-ethyl-azetidin-3-yl)-phenylamino]-5-iod-1,3-dihydro-indol-2-on als Trifluoressigsäuresalz

¹H-NMR (500 MHz, CDCl₃): δ [ppm] 13.55 (bs, 1 H); 8.15 (m, 1 H); 8.05 (d, 1 H); 7.80 (d, 1 H); 7.65 (m, 2H); 7.35 (s, 1 H); 6.90 (m, 1 H); 6.85 (d, 2H); 6.55 (m, 4H); 6.35 (s, 1 H); 4.60 (m, 2H); 4.25 (m, 1 H); 4.15 (m, 2H); 3.85 (s, 3H); 3.60 (m, 2H); 3.55 (s, 3H); 3.15 (m, 2H); 1.25 (t, 3H); 1.10 (t, 3H).

### BEISPIEL 65:

### 3-(4-Azetidin-3-yl-phenylamino)-3-(2-ethoxy-pyridin-3-yl)-5-iod-1-(4-methoxy-phenylsulfonyl)-1,3-dihydro-indol-2-on

### 65.1 3-{4-[3-(2-Ethoxy-pyridin-3-yl)-5-iod-1-(4-methoxy-phenylsulfonyl)-2-oxo-2,3-dihydro-1H-indol-3ylamino]-phenyl}-azetidine-1-carbonsäure-tert-butylester

ESI-MS: 741.10 [M-*^{t}*Bu+H]⁺

### 65.2 3-(4-Azetidin-3-yl-phenylamino)-3-(2-ethoxy-pyridin-3-yl)-5-iod-1-(4-methoxy-phenylsulfonyl)-1,3-dihydro-indol-2-on

¹H-NMR (500 MHz, CDCl₃): δ [ppm] 9.80 (bs, 1H); 9.45 (bs, 1H); 8.15 (d, 1H); 7.95 (d, 1 H); 7.90 (d, 2H); 7.65 (m, 2H); 7.30 (s, 1 H); 7.00-6.80 (m, 5H); 6.45 (m, 2H); 4.30 (bs, 2H); 4.20-3.95 (m, 5H); 3.85 (s, 3H); 0.95 (t, 3H).

### BEISPIEL 66:

### 3-(2-Ethoxy-pyridin-3-yl)-3-[4-(1-ethyl-azetidin-3-yl)-phenylamino]-5-iod-1-(4-methoxy-phenylsulfonyl)-1,3-dihydro-indol-2-on als Trifluoressigsäuresalz

ESI-MS: 725.15 [M+H]⁺
¹H-N MR (500 MHz, CDCl₃): δ [ppm] 13.55 (bs, 1 H); 8.15 (m, 1 H); 7.95 (m, 3H); 7.70 (d, 1 H); 7.65 (d, 1 H); 7.30 (s, 1 H); 6.95 (m, 1 H); 6.90 (d, 2H); 6.80 (d, 2H); 6.45 (d, 2H); 4.60 (m, 2H); 4.10 (m, 2H); 3.95 (m, 1 H); 3.90 (s, 3H); 3.65 (m, 2H); 3.15 (m, 2H); 1.25 (t, 3H); 0.90 (t, 3H).

### I.4.4 Verbindungen I, worin X¹ für -NH-, A für Pyridylen, X² für eine Einfachbindung, X³ für N, n für 2 und m für 2 stehen

### BEISPIEL 67:

### 1-(2,4-Dimethoxy-phenylsulfonyl)-3-(2-ethoxy-pyridin-3-yl)-5-iod-3-[6-(4-methyl-piperazin-1-yl)-pyridin-3-ylamino]-1,3-dihydro-indol-2-on

### 67.1 3-(2-Ethoxy-pyridin-3-yl)-5-iod-3-[6-(4-methyl-piperazin-1-yl)-pyridin-3-ylamino]-1,3-dihydro-indol-2-on

ESI-MS: 571.20 [M+H]⁺

### 67.2 1-(2,4-Dimethoxy-phenylsulfonyl)-3-(2-ethoxy-pyridin-3-yl)-5-iod-3-[6-(4-methylpiperazin-1-yl)-pyridin-3-ylamino]-1,3-dihydro-indol-2-on

ESI-MS: 771.15 [M+H]⁺
¹H-NMR (500 MHz, d₆-DMSO): δ [ppm] 8.20 (d, 1 H); 8.15 (m, 1 H); 7.85 (d, 1 H); 7.75 (d, 1 H); 7.55 (d, 1 H); 7.35 (s, 1 H); 7.25 (m, 1 H); 7.10 (m, 1 H); 6.70 (d, 1 H); 6.55 (s, 1 H); 6.30 (d, 1 H); 5.85 (s, 1 H); 4.05 (m, 2H); 3.85 (s, 3H); 3.55 (s, 3H); 3.25 (m, 4H); 2.40 (m, 4H); 2.20 (s, 3H); 0.95 (t, 3H).

### BEISPIEL 68:

### 3-(2-Ethoxy-pyridin-3-yl)-5-iod-1-(4-methoxy-phenylsulfonyl)-3-[6-(4-methyl-piperazin-1-yl)-pyridin-3-ylamino]-1,3-dihydro-indol-2-on

ESI-MS: 741.10 [M+H]⁺
¹H-NMR (500 MHz, d₆-DMSO): δ [ppm] 8.15 (m, 1H); 8.10 (d, 1H); 7.90 (d, 2H); 7.80 (d, 1H); 7.60 (d, 1H); 7.40 (m, 1H);7.30(s, 1 H); 7.15 (m, 3H); 6.85 (d, 1 H); 6.65 (d, 1 H); 4.15 (m, 2H); 3.95 (m, 1 H); 3.85 (s, 3H); 3.75 (m, 1 H); 3.50 (m, 2H); 3.05 (m, 4H); 2.85 (s, 3H); 0.70 (t, 3H).

### BEISPIEL 69:

### 1-Phenylsulfonyl-3-(2-ethoxy-pyridin-3-yl)-5-iod-3-[6-(4-methyl-piperazin-1-yl)-pyridin-3-ylamino]-1,3-dihydro-indol-2-on

ESI-MS: 711.10 [M+H]⁺
¹H-NMR (500 MHz, d₆-DMSO): δ [ppm]
8.15 (m, 2H); 7.95 (d, 2H); 7.80 (m, 2H); 7.65 (m, 3H); 7.40 (m, 1H); 7.35 (s, 1H); 7.15 (m, 1H); 6.80 (d, 1H); 6.60 (d, 1H); 4.15 (m, 2H); 3.95 (m, 1H); 3.70 (m, 1H); 3.50 (m, 2H); 3.05 (m, 4H); 2.85 (s, 3H); 0.65 (t, 3H).

### I.4.5 Verbindungen I, worin X¹ für -NH-, A für 1,4-Phenylen, X² für -C(=O)-, X³ für N, n für 2 und m für 2 stehen

### BEISPIEL 70:

### 3-(2-Ethoxy-pyridin-3-yl)-5-iod-1-(4-methoxy-phenylsulfonyl)-3-[4-(4-methyl-piperazin-1-carbonyl)-phenylamino]-1,3-dihydro-indol-2-on

### 70.1 3-(2-Ethoxy-pyridin-3-yl)-5-iod-3-[4-(4-methyl-piperazin-1-carbonyl)-phenylamino]-1,3-dihydro-indol-2-on

ESI-MS: 598.15 [M+H]⁺

### 70.2 3-(2-Ethoxy-pyridin-3-yl)-5-iod-1-(4-methoxy-phenylsulfonyl)-3-[4-(4-methyl-piperazin-1-carbonyl)-phenylamino]-1,3-dihydro-indol-2-on

ESI-MS: 768.10 [M+H]^{+ 1}H-NMR (500 MHz, d₆-DMSO): δ [ppm] 8.15 (m, 1H); 8.00 (d, 2H); 7.85 (m, 2H); 7.7 (d, 1 H); 7.25 (s, 1 H), 7.15 (d, 2H); 7.10 (m, 1 H); 7.05 (d, 2H); 7.0 (s, 1 H); 6.60 (d, 2H); 3.95 (m, 1 H); 3.85 (s, 3H); 3.70 (m, 1 H); 3.45 (bs, 4H); 2.30 (bs, 4H); 2.20 (s, 3H); 0.65 (t, 3H).

### BEISPIEL 71:

### 1-(2,4-Dimethoxy-phenylsulfonyl)-3-(2-ethoxy-pyridin-3-yl)-5-iod-3-[4-(4-methyl-piperazin-1-carbonyl)-phenylamino]-1,3-dihydro-indol-2-on

ESI-MS: 798.10 [M+H]⁺

### BEISPIEL 72:

### 3-(2-Ethoxy-pyridin-3-yl)-1-(4-methoxy-phenylsulfonyl)-3-[4-(4-methyl-piperazin-1-carbonyl)-phenylamino]-2-oxo-2,3-dihydro-1H-indol-5-carbonitril

### 72.1 3-(2-Ethoxy-pyridin-3-yl)-3-[4-(4-methyl-piperazin-1-carbonyl)-phenylamino]-2-oxo-2,3-dihydro-1H-indol-5-carbonitril

ESI-MS: 497.20 [M+H]⁺

### 72.2 3-(2-Ethoxy-pyridin-3-yl)-1-(4-methoxy-phenylsulfonyl)-3-[4-(4-methyl-piperazin-1-carbonyl)-phenylamino]-2-oxo-2,3-dihydro-1H-indol-5-carbonitril

ESI-MS: 667.25 [M+H]⁺
¹H-NMR (500 MHz, d₆-DMSO): δ [ppm] 8.10 (m, 1H); 8.00 (m, 3H); 7.95 (d, 1 H); 7.85 (d, 1 H); 7.5 (m, 1 H); 7.15 (d, 2H), 7.05 (m, 3H); 7.0 (s, 1 H); 6.55 (d, 2H); 3.85 (m, 1 H); 3.80 (s, 3H); 3.60 (m, 1 H); 3.50 (bs, 4H); 2.50 (bs, 4H); 2.35 (bs, 3H); 0.55 (t, 3H).

### BEISPIEL 73:

### 1-(2,4-Dimethoxy-phenylsulfonyl)-3-(2-ethoxy-pyridin-3-yl)-3-[4-(4-methyl-piperazin-1 - carbonyl)-phenylamino]-2-oxo-2,3-dihydro-1H-indol-5-carbonitril

ESI-MS: 697.25 [M+H]⁺
¹H-NMR (500 MHz, d₆-DMSO): δ [ppm] 8.10 (m, 1H); 8.00 (m, 3H); 7.90-7.80 (m, 4H); 7.5 (m, 1 H); 7.05 (m, 1 H); 7.0 (d, 2H); 6.90 (s, 1 H); 6.70 (m, 2H); 6.60 (d, 2H); 4.05-3.95 (m, 2H); 3.80 (s, 3H); 3.60 (s, 3H); 3.40 (bs, 4H); 2.25 (bs, 4H); 2.15 (s, 3H); 0.80 (t, 3H).

### I.4.6 Verbindungen I, worin X¹ für -NH-, A für 1,4-Phenylen, X² für -CH₂-, X³ für N, n für 2 und m für 2 stehen

### BEISPIEL 74

### 1-(2,4-Dimethoxy-phenylsulfonyl)-3-(2-ethoxy-pyridin-3-yl)-3-[4-(4-methyl-piperazin-1-ylmethyl)-phenylamino]-2-oxo-2,3-dihydro-1H-indol-5-carbonitril

### 74.1 3-(2-Ethoxy-pyridin-3-yl)-3-[4-(4-methyl-piperazin-1-ylmethyl)-phenylamino]-2-oxo-2,3-dihydro-1H-indol-5-carbonitril

Man gab 4-(4-Methyl-piperazin-1-ylmethyl)-phenylamin (425 mg, 2.07 mmol) zu einer Lösung von 3-Chlor-3-(2-ethoxy-pyridin-3-yl)-2-oxo-2,3-dihydro-1H-indol-5-carbonitril (500 mg, 1.59 mmol) in Dichlormethan (50 ml) und DIPEA (0.27 ml, 159 mmol) bei 0°C. Man rührte die Reaktionsmischung 2 h bei Raumtemperatur und gab danach eine wässrige NaHCO₃-Lösung zu. Das wässrige Reaktionsgemisch extrahierte man mit Dichormethan. Die vereinte organische Phase wurde über Magnesiumsulfat getrocknet, filtriert und unter verrmindertem Druck eingeengt. Die chromatographische Reinigung (Kieselgel, 0-20% Methanol in Dichloromethan) ergab 791 mg der Titelverbindung (99 % Ausbeute). ESI-MS: 483.25 [M+H]⁺

### 74.2 1-(2,4-Dimethoxy-phenylsulfonyl)-3-(2-ethoxy-pyridin-3-yl)-3-[4-(4-methyl-piperazin-1-ylmethyl)-phenylamino]-2-oxo-2,3-dihydro-1H-indol-5-carbonitril

ESI-MS: 683.25 [M+H]⁺
¹H-NMR (500 MHz, d₆-DMSO): δ [ppm] 8.15 (m, 1 H); 7.95 (d, 1 H); 7.90 (m, 3H); 7.5 (s, 1 H); 7.1 (m, 1 H), 6.80 (d, 2H); 6.70 (m, 2H); 6.50 (d, 2H); 6.45 (s, 1 H); 4.05 (m, 2H); 3.85 (s, 3H); 3.60 (s, 3H); 3.25 (s, 2H); 2.30 (bs, 8H); 2.15 (s, 3H); 0.85 (t, 3H).

### BEISPIEL 75

### 3-(2-Ethoxy-pyridin-3-yl)-1-(4-methoxy-phenylsulfonyl)-3-[4-(4-methyl-piperazin-1-ylmethyl)-phenylamino]-2-oxo-2,3-dihydro-1H-indol-5-carbonitril

¹H-NMR (500 MHz, d⁶-DMSO): δ [ppm] 8.15 (m, 1H); 8.05-7.95 (m, 5H); 7.55 (m, 1H); 7.20 (d, 2H); 7.10 (t, 1 H); 6.85 (d, 2H); 6.55 (m, 2H); 3.95 (m, 1 H); 3.85 (s, 3H); 3.75 (m, 2H); 3.60 (m, 1 H); 3.35 (m, 4H); 2.90 (m, 4H); 2.60 (m, 3H); 0.60 (t, 3H).

### BEISPIEL 76

### 3-(2-(2,2-Difluorethoxy)-pyridin-3-yl)-1-(4-methoxy-phenylsulfonyl)-3-[4-(4-methyl-piperazin-1-yl)-benzylamino]-2-oxo-2,3-dihydro-1H-indol-5-carbonitril

ESI-MS: 689.10 [M+H]⁺

### BEISPIEL 77

### 1-(4-Methoxy-phenylsulfonyl)-3-(2-(2,2,2-trifluorethoxy)-pyridin-3-yl)-3-[4-(4-methyl-piperazin-1-yl)-benzylamino]-2-oxo-2,3-dihydro-1H-indol-5-carbonitril

ESI-MS: 707.20 [M+H]⁺

### BEISPIEL 78

### 3-(2-(2,2-Difluorethoxy)-phenyl)-1-(4-methoxy-phenylsulfonyl)-3-[4-(4-methyl-piperazin-1-yl)-benzylamino]-2-oxo-2,3-dihydro-1H-indol-5-carbonitril

ESI-MS: 688.20 [M+H]⁺

### BEISPIEL 79

### 3-[4-(4-Ethyl-piperazin-1-yl)-phenylamino]-1-(4-methoxy-phenylsulfonyl)-3-(2-(2,2-difluorethoxy)-pyridin-3-yl)-2-oxo-2,3-dihydro-1H-indol-5-carbonitril

ESI-MS: 689.10 [M+H]⁺

### BEISPIEL 80

### 3-[4-(4-Ethyl-piperazin-1-yl)-phenylamino]-1-(4-methoxy-phenylsulfonyl)-3-(2-(2,2,2-trifluorethoxy)-pyridin-3-yl)-2-oxo-2,3-dihydro-1H-indol-5-carbonitril

ESI-MS: 693.20 [M+H]⁺

### BEISPIEL 81

### 3-(2-(2,2-Difluorethoxy)-phenyl)-3-[4-(4-ethyl-piperazin-1-yl)-phenylamino]-1-(4-methoxy-phenylsulfonyl)-2-oxo-2,3-dihydro-1H-indol-5-carbonitril

ESI-MS: 674.20 [M+H]⁺

### BEISPIEL 82

### 1-(2,4-Dimethoxy-phenylsulfonyl)-3-[4-(4-methyl-piperazin-1-yl)-phenylamino]-3-(2-(trifluormethoxy)-phenyl)-2-oxo-2,3-dihydro-1H-indol-5-carbonitril

ESI-MS: 736.20 [M+H]⁺

### II. BESTIMMUNG DER BIOLOGISCHEN AKTIVITÄT

### 1. Vasopressin V1b Rezeptorbindungstest:

### Substanzen:

Die Testsubstanzen wurden in einer Konzentration von 10⁻² M in DMSO gelöst und in DMSO auf 5x10⁻⁴ M bis 5x10⁻⁹ M weiter verdünnt. Diese DMSO-Vorverdünnungsreihe wurde mit Testpuffer 1:10 verdünnt. Im Testansatz wurde die Substanzkonzentration nochmals 1:5 verdünnt (2 % DMSO im Ansatz).

### Membran-Präparation:

CHO-K1 Zellen mit stabil exprimiertem humanem Vasopressin V1 b Rezeptor (Klon 3H2) wurden abgeerntet und in 50 mM Tris-HCl und in Gegenwart von Proteaseinhibitoren (Roche complete Mini # 1836170) mit einem Polytron Homogenizer auf mittlerer Stellung 2x10 Sekunden homogenisiert und anschließend 1h bei 40.000 x g abzentrifugiert. Das Membranpellet wurde nochmals wie beschrieben homogenisiert und zentrifugiert und anschließend in 50 mM Tris-HCl, pH 7,4 aufgenommen, homogenisiert und in Aliquots bei -190°C in flüssigem Stickstoff eingefroren aufbewahrt.

### Bindungstest:

Der Bindungstest wurde in Anlehnung an die Methode von Tahara et al. (Tahara A et al., Brit. J. Pharmacol. 125, 1463-1470 (1998)) durchgeführt.

Inkubationspufferwar: 50 mM Tris, 10 mM MgCl₂, 0.1% BSA, pH 7.4.
Im Testansatz (250 µl) wurden Membranen (50 µg/ml Protein in Inkubationspuffer) von CHO-K1-Zellen mit stabil exprimierten humanen V1b Rezeptoren (Zelllinie hV1b_3H2_CHO) mit 1,5 nM ³H-AVP (8-Arg-Vasopressin, PerkinElmer#18479) in Inkubationspuffer (50 mM Tris, 10 mM MgCl₂, 0.1% BSA, pH 7.4) (totale Bindung) oder zusätzlich mit steigenden Konzentrationen an Testsubstanz (Verdrängungsexperiment) inkubiert. Die unspezifische Bindung wurde mit 1 µM AVP (Bachem # H1780) bestimmt. Alle Bestimmungen wurden als Dreifachbestimmungen durchgeführt. Nach Inkubation (60 Minuten bei Raumtemperatur), wurde der freie Radioligand mittels Vakuumfiltration (Skatron cell harvester 7000) über Wathman GF/B Glasfaserfiltermatten abfiltriert und die Filter in Szintillationsgefäße überführt. Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

### Auswertung:

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)). Der Kd-Wert von ³H-AVP zu den rekombinanten humanen V1b-Rezeptoren beträgt 0,4 nM und wurde zur Bestimmung des Ki-Wertes herangezogen.

### 2. Vasopressin V1a Rezeptorbindungstest:

### Substanzen:

Die Testsubstanzen wurden in einer Konzentration von 10⁻² M in DMSO gelöst. Die weitere Verdünnung dieser DMSO-Lösungen erfolgte in Inkubationspuffer (50 mM Tris, 10 mM MgCl₂, 0.1 % BSA, pH 7,4).

### Membran-Präparation:

CHO-K1 Zellen mit stabil exprimiertem humanem Vasopressin V1a Rezeptor (Klon 5) wurden abgeerntet und in 50 mM Tris-HCl und in Gegenwart von Proteaseinhibitoren (Roche complete Mini # 1836170) mit einem Polytron Homogenizer auf mittlerer Stellung 2x10 Sekunden homogenisiert und anschließend 1h bei 40.000 x g abzentrifugiert. Das Membranpellet wurde nochmals wie beschrieben homogenisiert und zentrifugiert und anschließend in 50 mM Tris-HCl, pH 7,4 aufgenommen, homogenisiert und in Aliquots bei -190°C in flüssigem Stickstoff eingefroren aufbewahrt.

### Bindungstest:

Der Bindungstest wurde in Anlehnung an die Methode von Tahara et al. (Tahara A et al., Brit. J. Pharmacol. 125, 1463-1470 (1998)) durchgeführt.

Inkubationspufferwar: 50 mM Tris, 10 mM MgCl₂, 0.1% BSA, pH 7.4.

Im Testansatz (250 µl) wurden Membranen (20 µg/ml Protein in Inkubationspuffer) von CHO-K1-Zellen mit stabil exprimierten humanen V1a Rezeptoren (Zelllinie hV1a_5_CHO) mit 0.04 nM ¹²⁵I-AVP (8-Arg-Vasopressin, NEX 128) in Inkubationspuffer (50 mM Tris, 10 mM MgCl₂, 0.1% BSA, pH 7.4) (totale Bindung) oder zusätzlich mit steigenden Konzentrationen an Testsubstanz (Verdrängungsexperiment) inkubiert. Die unspezifische Bindung wurde mit 1 µM AVP (Bachem # H1780) bestimmt. Dreifachbestimmungen wurden durchgeführt.

Nach Inkubation (60 Minuten bei Raumtemperatur), wurde der freie Radioligand mittels Vakuumfiltration (Skatron cell harvester 7000) über Wathman GF/B Glasfaserfiltermatten abfiltriert und die Filter in Szintillationsgefäße überführt.

Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

### Auswertung:

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)). Der Kd-Wert von ¹²⁵I-AVP zu den rekombinanten hV1a-Rezeptoren wurde in Sättigungsexperimenten bestimmt. Ein Kd-Wert von 1,33 nM wurde zur Bestimmung des Ki-Wertes herangezogen.

### 3. Vasopressin V2 Rezeptorbindungstest:

### Substanzen:

Die Testsubstanzen wurden in einer Konzentration von 10⁻² M in DMSO gelöst. Die weitere Verdünnung dieser DMSO-Lösung erfolgte in Inkubationspuffer (50 mM Tris, 10 mM MgCl₂, 0.1 % BSA, pH 7,4).

### Membran-Präparation:

CHO-K1 Zellen mit stabil exprimiertem humanem Vasopressin V2 Rezeptor (Klon 23) wurden abgeerntet und in 50 mM Tris-HCl und in Gegenwart von Proteaseinhibitoren (Roche complete Mini # 1836170) mit einem Polytron Homogenizer auf mittlerer Stellung 2x10 Sekunden homogenisiert und anschließend 1h bei 40.000 x g abzentrifugiert. Das Membranpellet wurde nochmals wie beschrieben homogenisiert und zentrifugiert und anschließend in 50 mM Tris-HCl, pH 7,4 aufgenommen, homogenisiert und in Aliquots bei -190°C in flüssigem Stickstoff eingefroren aufbewahrt.

### Bindungstest:

Der Bindungstest wurde in Anlehnung an die Methode von Tahara et al. (Tahara A et al., Brit. J. Pharmacol. 125, 1463-1470 (1998)) durchgeführt.

Inkubationspufferwar: 50 mM Tris, 10 mM MgCl₂, 0.1% BSA, pH 7.4.

Im Testansatz (250 µl) wurden Membranen (50 µg/ml Protein in Inkubationspuffer) von CHO-K1-Zellen mit stabil exprimierten humanen V2 Rezeptoren (Zelllinie hV2_23_CHO) mit 1-2 nM ³H-AVP (8-Arg-Vasopressin, PerkinElmer #18479) in Inkubationspuffer (50 mM Tris, 10 mM MgCl₂, 0.1% BSA, pH 7.4) (totale Bindung) oder zusätzlich mit steigenden Konzentrationen an Testsubstanz (Verdrängungsexperiment) inkubiert. Die unspezifische Bindung wurde mit 1 µM AVP (Bachem # H1780) bestimmt. Dreifachbestimmungen wurden durchgeführt.

Nach Inkubation (60 Minuten bei Raumtemperatur), wurde der freie Radioligand mittels Vakuumfiltration (Skatron cell harvester 7000) über Wathman GF/B Glasfaserfiltermatten abfiltriert und die Filter in Szintillationsgefäße überführt.

Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

### Auswertung:

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)). Der Kd-Wert von ³H-AVP zu den rekombinanten hV2-Rezeptoren beträgt 2,4 nM und wurde zur Bestimmung des Ki-Wertes herangezogen.

### 4. Oxytozin-Rezeptorbindungstest

### Substanzen:

Die Substanzen wurden in einer Konzentration von 10⁻² M in DMSO gelöst und mit Inkubationspuffer (50 mM Tris, 10 mM MgCl₂, 0.1% BSA, pH 7,4) verdünnt.

### Zellpräparation:

Konfluente HEK-293 Zellen mit transient exprimierenden rekombinanten humanen Oxytozinrezeptoren wurden bei 750 x g für 5 Minuten bei Raumtemperatur zentrifugiert. Der Rückstand wurde in eiskaltem Lysispuffer (50 mM Tris-HCl, 10 % Glycerin, pH 7,4 und Roche Complete Protease-Inhibitor) aufgenommen und 20 Minuten bei 4 °C einem osmotischen Schock unterworfen. Danach wurden die lysierten Zellen bei 750 x g für 20 Minuten bei 4°C zentrifugiert, der Rückstand in Inkubationspuffer aufgenommen und Aliquots von 10⁷ Zellen/ml hergestellt. Die Aliquots wurden bis zur Verwendung bei -80°C eingefroren.

### Bindungstest:

Am Tag des Versuches wurden die Zellen aufgetaut, mit Inkubationspuffer verdünnt und mit einer Multipette Combitip (Eppendorf, Hamburg) homogenisiert. Der Reaktionsansatz von 0,250 ml setzte sich zusammen aus 2 bis 5x10⁴ rekombinante Zellen, 3-4 nM ³H-Oxytozin (PerkinElmer, NET 858) in Anwesenheit von Testsubstanz (Hemmkurve) oder nur Inkubationspuffer (totale Bindung). Die unspezifische Bindung wurde mit 10⁻⁶ M Oxytozin (Bachem AG, H2510) bestimmt. Dreifachbestimmungen wurden angesetzt. Gebundener und freier Radioligand wurden getrennt durch Filtration unter Vakuum mit Whatman GF/B Glasfaserfilter mit Hilfe eines Skatron Cell Harvester 7000. Die gebundene Radioaktivität wurde durch Flüssigkeitszintillationsmessung in einem Tricarb Beta-Zählgerät, Modell 2000 oder 2200CA (Packard) bestimmt.

### Auswertung:

Die Bindungsparameter wurden durch nicht-lineare Regressionsanalyse berechnet (SAS), analog zum Programm LIGAND von Munson und Rodbard (Analytical Biochem 1980; 107: 220-239). Der Kd-Wert von ³H-Oxytozin zu den rekombinanten hOT-Rezeptoren beträgt 7.6 nM und wurde zur Bestimmung des Ki-Wertes herangezogen.

### 5. Bestimmung der mikrosomalen Halbwertszeit:

Die metabolische Stabilität der erfindungsgemäßen Verbindungen wurde in dem folgenden Test bestimmt.

Die Testsubstanzen werden in einer Konzentration von 0.5 µM wie folgt inkubiert:
In Mikrotiterplatten werden 0.5 µM Testsubstanz zusammen mit Lebermikrosomen verschiedener Spezies (von Ratte, Mensch oder andere Spezies) (0.25 mg mikrosomales Protein/ml) in 0.05M Kalium-Phosphatpuffer pH 7,4 bei 37 °C für 5 min vorinkubiert. Der Start der Reaktion erfolgt durch die Zugabe von NADPH (1 mg/mL). Nach 0, 5, 10, 15, 20 und 30 min werden 50 µl Aliquots entnommen und die Reaktion wird sofort mit dem gleichen Volumen an Acetonitril abgestoppt und runtergekühlt. Die Proben werden bis zur Analyse eingefroren. Mittels MSMS wird die verbliebene Konzentration an nicht abgebauter Testsubstanz bestimmt. Aus der Steigung der Kurve Signal Testsubstanz/Zeiteinheit wird die Halbwertszeit (T1/2) ermittelt, wobei die Halbwertszeit der Testsubstanz unter Annahme einer Kinetik erster Ordnung aus der zeitlichen Abnahme der Konzentration der Verbindung berechnet werden kann. Die mikrosomale Clearance (mCl) berechnet sich aus mCl= In2/T1/2 / (Gehalt an mikrosomalem Protein in mg/ml) x 1000 [ml/min/mg] (modifiziert nach Literaturstellen: Di, The Society for Biomoleculaur Screening, 2003, 453-462; Obach, DMD, 1999 vol 27. N 11, 1350-1359).

### 6. Methoden zur in vitro Bestimmung der Cytochrom P450 (CYP) Inhibierung

### Lumineszenzsubstrate für 2C9 und 3A4:

0.4 mg/ml humane Lebermikrosomen werden 10 min mit den zu untersuchenden Testsubstanzen (0-20 µM), den CYP-spezifischen Substraten, in 0.05 M Kaliumphosphatpuffer pH 7,4 bei 37 °C vorinkubiert. Das Cyp-spezifische Substrat für CYP 2C9 ist Luciferin H, für CYP 3A4 Luciferin BE. Die Reaktion wird durch Hinzufügen von NADPH gestartet. Nach 30 min Inkubation bei RT wird das Luciferin Detektionsreagenz hinzugefügt, und das entstandene Lumineszenzsignal gemessen (modifiziert nach Literaturstelle: Promega, Technical Bulletin P450-GLO™ Assays).

### Midazolam CYP 3A4 Time dependent Inhibition

Der Test besteht aus 2 Teilen. Einmal wird die Testsubstanz mit den Lebermikrosomen vorinkubiert (mit NADPH = Prä-Inkubation, danach Zugabe des Substrates, im 2 .Teil wird das Substrat und die Testsubstanz gleichzeitig zugegeben = Co-Inkubation.

### Prä-Inkubation:

0.05 mg/ml mikrosomales Protein (humane Lebermikrosomen) werden mit 0-10 µM (bzw 50 µM) Testsubstanz in 50 mM Kaliumphosphatpuffer 5 Min vorinkubiert. Die Reaktion wird mit NADPH gestartet. Nach 30 min werden 4 µM Midazolam (Endkonzentration) hinzugefügt und für weitere 10 Min inkubiert. 75 µl der Reaktionslösung werden nach 10 min entnommen und mit 150 µl Acetonitrillösung abgestoppt.

### Co-Inkubation:

0.05 mg/ml mikrosomales Protein (humane Lebermikrosomen) werden mit 4 µM Midazolam (Endkonzentration) und 0-10 µM (bzw 50µM) Testsubstanz in 50 mM Kaliumphosphatpuffer 5 min vorinkubiert. Die Reaktion wird mit NADPH gestartet. 75 µl der Reaktionslösung werden nach 10 min entnommen und mit 150 µl Acetonitrillösung abgestoppt. Die Proben werden bis zur MSMS-Analytik eingefroren (modifiziert nach Literaturstellen: Obdach, Journal of Pharmacology & Experimental Therapeutics, Vol 316, 1, 336-348, 2006; Walsky, Drug Metabolism and Disposition Vol 32, 6, 647-660, 2004).

### 7. Methode zur Bestimmung der Wasserlöslichkeit (in mg/ml)

Die Wasserlöslichkeit der erfindungsgemässen Verbindungen kann zum Beispiel nach der sogenannten "shake flask" Methode bestimmt werden (gemäss *ASTM International: E 1148-02, Standard test methods for measurement of aqueous solubility, Book of Standards Volume 11.05.).* Dabei wird ein Überschuss der festen Verbindung in eine Pufferlösung mit einem bestimmten pH-Wert (zum Beispiel Phosphatpuffer pH 7.4) gegeben und die entstehende Mischung geschüttelt oder gerührt, bis sich das Gleichgewicht eingestellt hat (typischerweise 24 oder 48 Stunden, manchmal auch bis zu 7 Tage). Anschließend wird der ungelöste Feststoff durch Filtrieren oder Zentrifugieren abgetrennt und die Konzentration der gelösten Verbindung durch UV-Spektroskopie oder HochdruckflüssigkeitsChromatographie (HPLC) anhand einer entsprechenden Kalibrierkurve bestimmt.

### 8. Ergebnisse

Die Ergebnisse der Rezeptor-Bindungsuntersuchungen sind als Rezeptorbindungskonstanten [Kᵢ(V1b)] bzw. Selektivitäten [Kᵢ(V1a)/ Kᵢ(V1b)] ausgedrückt. Die Ergebnisse der Untersuchung der metabolischen Stabilität sind als mikrosomale Clearance (mCl) angegeben.

In diesen Tests zeigen die erfindungsgemäßen Verbindungen sehr hohe Affinitäten für den V1 b-Rezeptor (höchstens 100 nM, oder höchstens 10 nM, häufig < 1 nM). Außerdem zeigen die Verbindungen auch hohe Selektivitäten gegenüber dem V1a-Rezeptor und dem Oxytozin-Rezeptor.

Die Ergebnisse sind in Tabelle 1 aufgeführt. Die Nummern der Verbindungen beziehen sich auf die Synthesebeispiele.

**Tabelle 1**

| Beispiel | Kᵢ(h-V1b)* [nM] | Kᵢ(h-V1a)/Kᵢ(h-V1b)* | Kᵢ(h-OT)/Kᵢ(h-V1b)* |
|---|---|---|---|
| 2 | ++ | +++ | ++ |
| 3 | ++ | +++ | + |
| 4 | ++ | +++ | ++ |
| 5 | ++ | +++ | +++ |
| 6 | ++ | +++ | ++ |
| 7 | ++ | +++ | ++ |
| 13 | + | + | ++ |
| 14 | ++ | + | ++ |
| 15 | ++ | ++ | +++ |
| 16 | ++ | +++ | +++ |
| 17 | ++ | + | + |
| 24 | ++ | +++ | + |
| 25 | + | ++ | + |
| 26 | + | ++ | +++ |
| 27 | + | + | +++ |
| 28 | + | + | +++ |
| 29 | ++ | ++ | + |
| 30 | ++ | +++ | +++ |
| 31 | +++ | +++ | +++ |
| 33 | +++ | +++ | +++ |
| 34 | ++ | + | +++ |
| 35 | ++ | + | +++ |
| 37 | ++ | ++ | +++ |
| 38 | ++ | + | + |
| 40 | ++ | ++ | ++ |
| 41 | ++ | ++ | ++ |
| 43 | ++ | +++ | + |
| 50 | ++ | + | +++ |
| 57 | + | ++ | +++ |
| 59 | ++ | ++ | +++ |
| 60 | ++ | ++ | ++ |
| 68 | ++ | + | ++ |
| 70 | ++ | ++ | + |
| 73 | + | +++ | ++ |
| 74 | ++ | +++ | +++ |
| 75 | + | ++ | +++ |
| 77 | ++ | ++ | ++ |
| 79 | + | ++ | +++ |

| | | | |
|---|---|---|---|
| * h = human | | | |

Schlüssel:

| | Kᵢ(V1b) | Kᵢ(h-V1a)/Kᵢ(h-V1b) | Kᵢ(h-V1a)/Kᵢ(h-OT) |
|---|---|---|---|
| + | > 10 - 100nm | 10 - < 25 | 10 - < 25 |
| ++ | 1 - 10nm | 25 - 75 | 25 - 75 |
| +++ | < 1 nm | > 75 | > 75 |

## Patentansprüche

1. Verbindungen der Formel I 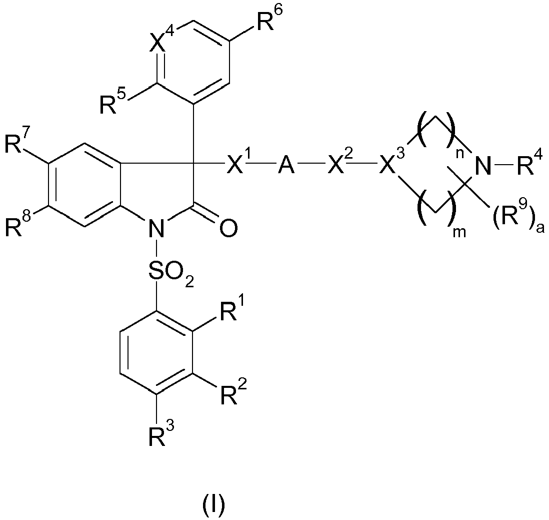 worin
X¹ für -O-, -O-CH₂-, -O-C(=O)-, -NR¹¹-, -NR¹¹-CH₂- oder -NR¹¹-C(=O)- steht;
X² für eine Einfachbindung, CO oder CH₂ steht;
X³ für N oder CH steht;
X⁴ für N oder CH steht;
A für Phenylen oder 6-gliedriges Hetarylen mit 1 oder 2 Stickstoffatomen als Ringgliedern steht, wobei Phenylen oder Hetarylen durch 1 oder 2 Reste R¹⁰ substituiert sein kann;
R¹ und R³ unabhängig voneinander für Wasserstoff, C₁-C₃-Alkyl, C₁-C₃-Fluoralkyl, C₁-C₃-Alkoxy, C₁-C₃-Fluoralkoxy, Halogen oder CN stehen;
R² für Wasserstoff oder Methoxy steht;
wobei wenigstens einer der Reste R¹, R² und R³ für Wasserstoff steht;
R⁴ für Wasserstoff oder C₁-C₄-Alkyl steht;
R⁵ für Ethoxy, fluoriertes Ethoxy, Fluormethoxy, Difluormethoxy, Trifluormethoxy oder Isopropoxy steht;
R⁶ für Wasserstoff oder Methyl steht;
R⁷ für Wasserstoff, I, Br, Cl, F oder CN steht;
R⁸ für Wasserstoff, I, Br, Cl, F oder CN steht;
R⁹ für C₁-C₃-Alkyl oder C₁-C₃-Fluoralkyl steht;
R¹⁰ für C₁-C₃-Alkyl, C₁-C₃-Fluoralkyl, C₁-C₃-Alkoxy oder C₁-C₃-Fluoralkoxy steht;
R¹¹ für Wasserstoff, C₁-C₄-Alkyl, C₁-C₄-Fluoralkyl, C₁-C₄-Alkoxy oder C₁-C₄-Fluoralkoxy steht;
a für 0, 1 oder 2 steht; und
m und n unabhängig voneinander für 1 oder 2 stehen;
sowie deren pharmazeutisch verträglichen Salze.

2. Verbindungen nach Anspruch 1, worin R¹ für Wasserstoff, Methoxy, Ethoxy, Fluormethoxy, Difluormethoxy oder Trifluormethoxy, vorzugsweise für Wasserstoff, Methoxy oder Trifluormethoxy und insbesondere für Wasserstoff oder Methoxy steht.

3. Verbindungen nach einem der vorhergehenden Ansprüche, worin R² für Wasserstoff steht.

4. Verbindungen nach einem der vorhergehenden Ansprüche, worin R³ für Wasserstoff oder Methoxy steht.

5. Verbindungen nach einem der vorhergehenden Ansprüche, worin R⁴ für Wasserstoff, Methyl oder Ethyl steht.

6. Verbindungen nach einem der vorhergehenden Ansprüche, worin R⁵ für Ethoxy oder fluoriertes Ethoxy, vorzugsweise für Ethoxy, 2,2-Difluorethoxy oder 2,2,2-Trifluorethoxy und insbesondere für Ethoxy steht.

7. Verbindungen nach einem der vorhergehenden Ansprüche, worin R⁶ für Wasserstoff steht.

8. Verbindungen nach einem der vorhergehenden Ansprüche, worin R⁷ für CN steht und R⁸ für H oder F steht.

9. Verbindungen nach einem der Ansprüche 1 bis 7, worin R⁷ für I steht und R⁸ für H oder F steht.

10. Verbindungen nach einem der Ansprüche 1 bis 7, worin R⁷ für Cl steht und R⁸ für H oder F steht.

11. Verbindungen nach einem der vorhergehenden Ansprüche, worin R¹⁰ für Methyl oder Methoxy steht.

12. Verbindungen nach einem der vorhergehenden Ansprüche, worin R¹¹ für H steht.

13. Verbindungen nach einem der vorhergehenden Ansprüche, worin a für 0 steht.

14. Verbindungen nach einem der vorhergehenden Ansprüche, worin X¹ für -O-C(=O)-, -NH-, -NH-CH₂- oder -NH-C(=O)- und vorzugsweise für -NH-CH₂-steht.

15. Verbindungen nach einem der vorhergehenden Ansprüche, worin X² für eine Einfachbindung steht.

16. Verbindungen nach einem der vorhergehenden Ansprüche, worin X³ für N steht und m und n beide für 2 stehen.

17. Verbindungen nach einem der Ansprüche1 bis 15, worin X³ für CH steht und m und n beide für 2 stehen oder beide für 1 stehen oder m für 1 und n für 2 steht.

18. Verbindungen nach einem der vorhergehenden Ansprüche, worin X⁴ für N steht.

19. Verbindungen nach einem der vorhergehenden Ansprüche, worin A für Phenylen, vorzugsweise für 1,4- oder 1,3-Phenylen steht.

20. Verbindungen nach einem der Ansprüche 1 bis 18, worin A für Pyridylen oder Pyrimidylen, vorzugsweise für 3,5- oder 3,6-Pyridylen steht.

21. Verbindungen nach einem der Ansprüche 1 bis 19, worin
R¹ für H oder Methoxy steht;
R² für H steht;
R³ für Methoxy steht;
R⁴ für H, Methyl oder Ethyl steht;
R⁵ für Ethoxy steht;
R⁶ für H steht;
R⁷ für CN steht;
R⁸ für H oder F steht;
X¹ für-NH-oder-NHCH₂-steht;
X² für eine Einfachbindung steht;
X³ für N steht;
X⁴ für N steht;
A für 1,4-Phenylen steht;
a für 0 steht; und
m und n für 2 stehen;
und worin vorzugsweise
R¹ für H oder Methoxy steht;
R² für H steht;
R³ für Methoxy steht;
R⁴ für H oder Methyl steht;
R⁵ für Ethoxy steht;
R⁶ für H steht;
R⁷ für CN steht;
R⁸ für H oder F steht;
X¹ für-NHCH₂-steht;
X² für eine Einfachbindung steht;
X³ für N steht;
X⁴ für N steht;
A für 1,4-Phenylen steht;
a für 0 steht; und
m und n für 2 stehen.

22. Verbindungen nach Anspruch 1, ausgewählt unter
4-(4-Methyl-piperazin-1-yl)-benzoesäure-1-(2,4-di methoxy-phenylsulfonyl)-3-(2-ethoxy-pyridin-3-yl)-5-iod-2-oxo-2,3-dihydro-1H-indol-3ylester;
4-(4-Methyl-piperazin-1-yl)-benzoesäure-5-cyano-3-(2-ethoxy-pyridin-3-yl)-1-(4-methoxy-phenylsulfonyl)-2-oxo-2,3-dihydro-1H-indol-3ylester;
4-(4-Methyl-piperazin-1-yl)-benzoesäure-5-cyano-1-(2,4-d imethoxy-phenylsulfonyl)-3-(2-ethoxy-pyridin-3-yl)-2-oxo-2,3-dihydro-1H-indol-3yl-ester;
N-[5-Cyano-3-(2-ethoxy-pyridin-3-yl)-1-(4-methoxy-phenylsulfonyl)-2-oxo-2,3-dihydro-1H-indol-3-yl]-4-(4-methyl-piperazin-1-yl)-benzamid;
N-[5-Cyano-3-(2-ethoxy-pyridin-3-yl)-1-(4-methoxy-phenylsulfonyl)-2-oxo-2,3-dihydro-1H-indol-3-yl]-4-(4-ethyl-piperazin-1-yl)-benzamid;
N-[5-Cyano-1-(2,4-dimethoxy-phenylsulfonyl)-3-(2-ethoxy-pyridin-3-yl)-2-oxo-2,3-dihydro-1H-indol-3-yl]-4-(4-methyl-piperazin-1-yl)-benzamid;
N-[5-Cyano-1-(2,4-dimethoxy-phenylsulfonyl)-3-(2-ethoxy-pyridin-3-yl)-2-oxo-2,3-dihydro-1H-indol-3-yl]-4-(4-ethyl-piperazin-1-yl)-benzamid;
N-[1-[4-(2,2-Difluor-ethoxy)-phenylsulfonyl]-3-(2-ethoxy-pyridin-3-yl)-5-iod-2-oxo-2,3-dihydro-1H-indol-3-yl]-4-(4-methyl-piperazin-1-yl)-benzamid;
1-(2,4-Dimethoxy-phenylsulfonyl)-3-(2-ethoxy-pyridin-3-yl)-5-iod-3-[4-(4-methylpiperazin-1-yl)-benzylamino]-1,3-dihydro-indol-2-on;
1-(2,4-Dimethoxy-phenylsulfonyl)-3-(2-ethoxy-pyridin-3-yl)-5-iod-3-(4-piperazin-1-yl-benzylamino)-1,3-dihydro-indol-2-on;
5-Chlor-3-(2-ethoxy-pyridin-3-yl)-1-(4-methoxy-phenylsulfonyl)-3-[4-(4-methyl-piperazin-1-yl)-benzylamino]-1,3-dihydro-indol-2-on;
5-Chlor-3-(2-ethoxy-pyridin-3-yl)-6-fluor-1-(4-methoxy-phenylsulfonyl)-3-[4-(4-methyl-piperazin-1-yl)-benzylamino]-1,3-dihydro-indol-2-on;
6-Chlor-3-(2-ethoxy-pyridin-3-yl)-5-fluor-1-(4-methoxy-phenylsulfonyl)-3-[4-(4-methyl-piperazin-1-yl)-benzylamino]-1,3-dihydro-indol-2-on;
5,6-Difluor-3-(2-ethoxy-pyridin-3-yl)-1-(4-methoxy-phenylsulfonyl)-3-[4-(4-methyl-piperazin-1-yl)-benzylamino]-1,3-dihydro-indol-2-on;
3-(2-Ethoxy-pyridin-3-yl)-5-fluor-1-(4-methoxy-phenylsulfonyl)-3-[4-(4-methyl-piperazin-1-yl)-benzylamino]-1,3-dihydro-indol-2-on;
3-(2-Ethoxy-pyridin-3-yl)-1-(4-methoxy-phenylsulfonyl)-3-[4-(4-methyl-piperazin-1-yl)-benzylamino]-2-oxo-2,3-dihydro-1H-indol-5-carbonitril;
1-(2,4-Dimethoxy-phenylsulfonyl)-3-(2-ethoxy-pyridin-3-yl)-3-[4-(4-methyl-piperazin-1-yl)-benzylamino]-2-oxo-2,3-dihydro-1H-indol-5-carbonitril;
3-(2-Ethoxy-pyridin-3-yl)-1-(4-methoxy-2-trifluormethoxy-phenylsulfonyl)-3-[4-(4-methyl-piperazin-1-yl)-benzylamino]-2-oxo-2,3-dihydro-1H-indol-5-carbonitril;
3-(2-Ethoxy-pyridin-3-yl)-3-[4-(4-methyl-piperazin-1-yl)-benzylamino]-2-oxo-1-(4-trifluormethoxy-phenylsulfonyl)-2,3-dihydro-1H-indol-5-carbonitril;
3-(2-Ethoxy-pyridin-3-yl)-3-[4-(4-methyl-piperazin-1-yl)-benzylamino]-2-oxo-1-[4-(2,2,2-trifluor-ethoxy)-phenylsulfonyl]-2,3-dihydro-1H-indol-5-carbonitril;
1-[4-(2,2-Difluor-ethoxy)-phenylsulfonyl]-3-(2-ethoxy-pyridin-3-yl)-3-[4-(4-methyl-piperazin-1-yl)-benzylamino]-2-oxo-2,3-dihydro-1H-indol-5-carbonitril;
3-(2-Ethoxy-pyridin-3-yl)-1-[4-(2-fluor-ethoxy)-phenylsulfonyl]-3-[4-(4-methyl-piperazin-1-yl)-benzylamino]-2-oxo-2,3-dihydro-1H-indol-5-carbonitril;
1-(4-Difluormethoxy-phenylsulfonyl)-3-(2-ethoxy-pyridin-3-yl)- 3-[4-(4-methylpiperazin-1-yl)-benzylamino]-2-oxo-2,3-dihydro-1H-indol-5-carbonitril;
3-(2-Ethoxy-pyridin-3-yl)-1-(2-methoxy-phenylsulfonyl)-3-[4-(4-methyl-piperazin-1-yl)-benzylamino]-2-oxo-2,3-dihydro-1H-indol-5-carbonitril;
3-(2-Ethoxy-pyridin-3-yl)-1-(3-methoxy-phenylsulfonyl)-3-[4-(4-methyl-piperazin-1-yl)-benzylamino]-2-oxo-2,3-dihydro-1H-indol-5-carbonitril;
1-(4-Chlor-phenylsulfonyl)-3-(2-ethoxy-pyridin-3-yl)-3-[4-(4-methyl-piperazin-1-yl)-benzylamino]-2-oxo-2,3-dihydro-1H-indol-5-carbonitril;
1-Phenylsulfonyl-3-(2-ethoxy-pyridin-3-yl)-3-[4-(4-methyl-piperazin-1-yl)-benzylamino]-2-oxo-2,3-dihydro-1H-indol-5-carbonitril;
1-(4-Cyano-phenylsulfonyl)-3-(2-ethoxy-pyridin-3-yl)-3-[4-(4-methyl-piperazin-1-yl)-benzylamino]-2-oxo-2,3-dihydro-1H-indol-5-carbonitril;
1-(2,4-Dimethoxy-phenylsulfonyl)-3-(2-ethoxy-pyridin-3-yl)-2-oxo-3-(4-piperazin-1-yl-benzylamino)-2,3-dihydro-1H-indol-5-carbonitril;
3-(2-Ethoxy-pyridin-3-yl)-1-(4-methoxy-phenylsulfonyl)-2-oxo-3-(4-piperazin-1-yl-benzylamino)-2,3-dihydro-1H-indol-5-carbonitril;
3-(2-Ethoxy-pyridin-3-yl)-3-[4-(4-ethyl-piperazin-1-yl)-benzylamino]-1-(4-methoxy-phenylsulfonyl)-2-oxo-2,3-dihydro-1H-indol-5-carbonitril;
1-(2,4-Dimethoxy-phenylsulfonyl)-3-(2-ethoxy-pyridin-3-yl)-3-[4-(4-ethyl-piperazin-1-yl)-benzylamino]-2-oxo-2,3-dihydro-1H-indol-5-carbonitril;
3-(2-Ethoxy-pyridin-3-yl)-6-fluor-1-(4-methoxy-phenylsulfonyl)-3-[4-(4-methyl-piperazin-1-yl)-benzylamino]-2-oxo-2,3-dihydro-1H-indol-5-carbonitril;
3-(2-Ethoxy-pyridin-3-yl)-6-fluor-1-(4-methoxy-phenylsulfonyl)-2-oxo-3-(4-piperazin-1-yl-benzylamino)-2,3-dihydro-1H-indol-5-carbonitril;
3-(2-Ethoxy-pyridin-3-yl)-3-[4-(4-ethyl-piperazin-1-yl)-benzylamino]-6-fluor-1-(4-methoxy-phenylsulfonyl)-2-oxo-2,3-dihydro-1H-indol-5-carbonitril;
1-[4-(2,2-Difluor-ethoxy)-phenylsulfonyl]-3-(2-ethoxy-pyridin-3-yl)-6-fluor-3-[4-(4-methyl-piperazin-1-yl)-benzylamino]-2-oxo-2,3-dihydro-1H-indol-5-carbonitril;
3-(2-Ethoxy-phenyl)-1-(4-methoxy-phenylsulfonyl)-3-[4-(4-methyl-piperazin-1-yl)-benzylamino]-2-oxo-2,3-dihydro-1H-indol-5-carbonitril;
1-(2,4-Dimethoxy-phenylsulfonyl)-3-(2-ethoxy-pyridin-3-yl)-5-iod-3-[3-(4-methyl-piperazin-1-yl)-benzylamino]-1,3-dihydro-indol-2-on;
3-(2-Ethoxy-pyridin-3-yl)-5-iod-1-(4-methoxy-2-trifluormethoxy-phenylsulfonyl)-3-[3-(4-methyl-piperazin-1-yl)-benzylamino]-1,3-dihydro-indol-2-on;
3-(2-Ethoxy-pyridin-3-yl)-5-iod-1-(4-methoxy-phenylsulfonyl)-3-[3-(4-methyl-piperazin-1-yl)-benzylamino]-1,3-dihydro-indol-2-on;
3-(2-Ethoxy-pyridin-3-yl)-5-iod-1-(2-methoxy-phenylsulfonyl)-3-[3-(4-methyl-piperazin-1-yl)-benzylamino]-1,3-dihydro-indol-2-on;
1-(2,4-Dimethoxy-phenylsulfonyl)-3-(2-ethoxy-pyridin-3-yl)-3-[3-(4-methyl-piperazin-1-yl)-benzylamino]-2-oxo-2,3-dihydro-1H-indol-5-carbonitril;
3-(2-Ethoxy-pyridin-3-yl)-1-(4-methoxy-phenylsulfonyl)-3-[3-(4-methyl-piperazin-1-yl)-benzylamino]-2-oxo-2,3-dihydro-1H-indol-5-carbonitril;
3-(2-Ethoxy-pyridin-3-yl)-1-(2-methoxy-phenylsulfonyl)-3-[3-(4-methyl-piperazin-1-yl)-benzylamino]-2-oxo-2,3-dihydro-1H-indol-5-carbonitril;
1-(2,4-Dimethoxy-phenylsulfonyl)-3-(2-ethoxy-pyridin-3-yl)-3-[2-(4-methyl-piperazin-1-yl)-benzylamino]-2-oxo-2,3-dihydro-1H-indol-5-carbonitril;
3-(2-Ethoxy-pyridin-3-yl)-1-(4-methoxy-phenylsulfonyl)-3-[2-(4-methyl-piperazin-1-yl)-benzylamino]-2-oxo-2,3-dihydro-1H-indol-5-carbonitril;
3-(2-Ethoxy-pyridin-3-yl)-1-(2-methoxy-phenylsulfonyl)-3-[2-(4-methyl-piperazin-1-yl)-benzylamino]-2-oxo-2,3-dihydro-1H-indol-5-carbonitril;
1-(2,4-Dimethoxy-phenylsulfonyl)-3-(2-ethoxy-pyridin-3-yl)-3-[4-(4-ethyl-piperazin-1-yl)-phenylamino]-5-iod-1,3-dihydro-indol-2-on;
1-(2,4-Dimethoxy-phenylsulfonyl)-3-(2-ethoxy-pyridin-3-yl)-5-iod-3-[4-(4-methyl-piperazin-1-yl)-phenylamino]-1,3-dihydro-indol-2-on;
3-(2-Ethoxy-pyridin-3-yl)-5-iod-1-(4-methoxy-phenylsulfonyl)-3-[4-(4-methyl-piperazin-1-yl)-phenylamino]-1,3-dihydro-indol-2-on;
5-Chlor-3-(2-ethoxy-pyridin-3-yl)-1-(4-methoxy-phenylsulfonyl)-3-[4-(4-methyl-piperazin-1-yl)-phenylamino]-1,3-dihydro-indol-2-on;
5-Chlor-3-(2-ethoxy-pyridin-3-yl)-6-fluor-1-(4-methoxy-phenylsulfonyl)-3-[4-(4-methyl-piperazin-1-yl)-phenylamino]-1,3-dihydro-indol-2-on;
6-Chlor-3-(2-ethoxy-pyridin-3-yl)-5-fluor-1-(4-methoxy-phenylsulfonyl)-3-[4-(4-methyl-piperazin-1-yl)-phenylamino]-1,3-dihydro-indol-2-on;
3-(2-Ethoxy-pyridin-3-yl)-5,6-difluor-1-(4-methoxy-phenylsulfonyl)-3-[4-(4-methyl-piperazin-1-yl)-phenylamino]-1,3-dihydro-indol-2-on;
1-(2,4-Dimethoxy-phenylsulfonyl)-3-(2-ethoxy-pyridin-3-yl)-3-[4-(4-methyl-piperazin-1-yl)-phenylamino]-2-oxo-2,3-dihydro-1H-indol-5-carbonitril;
1-(2,4-Dimethoxy-phenylsulfonyl)-3-(2-ethoxy-pyridin-3-yl)-3-[4-(4-ethyl-piperazin-1-yl)-phenylamino]-2-oxo-2,3-dihydro-1H-indol-5-carbonitril;
3-(2-Ethoxy-pyridin-3-yl)-3-[4-(4-ethyl-piperazin-1-yl)-phenylamino]-1-(4-methoxy-phenylsulfonyl)-2-oxo-2,3-dihydro-1H-indol-5-carbonitril;
3-(2-Ethoxy-pyridin-3-yl)-3-[4-(4-ethyl-piperazin-1-yl)-phenylamino]-1-(2-methoxy-phenylsulfonyl)-2-oxo-2,3-dihydro-1H-indol-5-carbonitril;
3-(2-Ethoxy-pyridin-3-yl)-6-fluor-1-(4-methoxy-phenylsulfonyl)-3-[4-(4-methylpiperazin-1-yl)-phenylamino]-2-oxo-2,3-dihydro-1H-indol-5-carbonitril;
1-(2,4-Dimethoxy-phenylsulfonyl)-3-(2-ethoxy-pyridin-3-yl)-5-iod-3-[3-(4-methylpiperazin-1-yl)-phenylamino]-1,3-dihydro-indol-2-on;
3-(2-Ethoxy-pyridin-3-yl)-1-(4-methoxy-phenylsulfonyl)-3-[3-(4-methyl-piperazin-1-yl)-phenylamino]-2-oxo-2,3-dihydro-1H-indol-5-carbonitril;
1-(2,4-Dimethoxy-phenylsulfonyl)-3-(2-ethoxy-pyridin-3-yl)-3-[3-(4-methylpiperazin-1-yl)-phenylamino]-2-oxo-2,3-dihydro-1H-indol-5-carbonitril;
3-(4-Azetidin-3-yl-phenylamino)-1-(2,4-dimethoxy-phenylsulfonyl)-3-(2-ethoxy-pyridin-3-yl)-5-iod-1,3-dihydro-indol-2-on;
1-(2,4-Dimethoxy-phenylsulfonyl)-3-(2-ethoxy-pyridin-3-yl)-3-[4-(1-ethyl-azetidin-3-yl)-phenylamino]-5-iod-1,3-dihydro-indol-2-on;
3-(4-Azetidin-3-yl-phenylamino)-3-(2-ethoxy-pyridin-3-yl)-5-iod-1-(4-methoxy-phenylsulfonyl)-1,3-dihydro-indol-2-on;
3-(2-Ethoxy-pyridin-3-yl)-3-[4-(1-ethyl-azetidin-3-yl)-phenylamino]-5-iod-1-(4-methoxy-phenylsulfonyl)-1,3-dihydro-indol-2-on;
1-(2,4-Dimethoxy-phenylsulfonyl)-3-(2-ethoxy-pyridin-3-yl)-5-iod-3-[6-(4-methyl-piperazin-1-yl)-pyridin-3-ylamino]-1,3-dihydro-indol-2-on;
3-(2-Ethoxy-pyridin-3-yl)-5-iod-1-(4-methoxy-phenylsulfonyl)-3-[6-(4-methylpiperazin-1-yl)-pyridin-3-ylamino]-1,3-dihydro-indol-2-on;
1-Phenylsulfonyl-3-(2-ethoxy-pyridin-3-yl)-5-iod-3-[6-(4-methyl-piperazin-1-yl)-pyridin-3-ylamino]-1,3-dihydro-indol-2-on;
3-(2-Ethoxy-pyridin-3-yl)-5-iod-1-(4-methoxy-phenylsulfonyl)-3-[4-(4-methylpiperazin-1-carbonyl)-phenylamino]-1,3-dihydro-indol-2-on;
1-(2,4-Dimethoxy-phenylsulfonyl)-3-(2-ethoxy-pyridin-3-yl)-5-iod-3-[4-(4-methylpiperazin-1-carbonyl)-phenylamino]-1,3-dihydro-indol-2-on;
3-(2-Ethoxy-pyridin-3-yl)-1-(4-methoxy-phenylsulfonyl)-3-[4-(4-methyl-piperazin-1-carbonyl)-phenylamino]-2-oxo-2,3-dihydro-1H-indol-5-carbonitril;
1-(2,4-Dimethoxy-phenylsulfonyl)-3-(2-ethoxy-pyridin-3-yl)-3-[4-(4-methylpiperazin-1-carbonyl)-phenylamino]-2-oxo-2,3-dihydro-1H-indol-5-carbonitril;
1-(2,4-Dimethoxy-phenylsulfonyl)-3-(2-ethoxy-pyridin-3-yl)-3-[4-(4-methylpiperazin-1-ylmethyl)-phenylamino]-2-oxo-2,3-dihydro-1H-indol-5-carbonitril;
3-(2-Ethoxy-pyridin-3-yl)-1-(4-methoxy-phenylsulfonyl)-3-[4-(4-methyl-piperazin-1-ylmethyl)-phenylamino]-2-oxo-2,3-dihydro-1H-indol-5-carbonitril;
3-(2-(2,2-Difluorethoxy)-pyridin-3-yl)-1-(4-methoxy-phenylsulfonyl)-3-[4-(4-methylpiperazin-1-yl)-benzylamino]-2-oxo-2,3-dihydro-1H-indol-5-carbonitril;
1-(4-Methoxy-phenylsulfonyl)-3-(2-(2,2,2-trifluorethoxy)-pyridin-3-yl)-3-[4-(4-methyl-piperazin-1-yl)-benzylamino]-2-oxo-2,3-dihydro-1H-indol-5-carbonitril;
3-(2-(2,2-Difluorethoxy)-phenyl)-1-(4-methoxy-phenylsulfonyl)-3-[4-(4-methylpiperazin-1-yl)-benzylamino]-2-oxo-2,3-dihydro-1H-indol-5-carbonitril;
3-[4-(4-Ethyl-piperazin-1-yl)-phenylamino]-1-(4-methoxy-phenylsulfonyl)-3-(2-(2,2-difluorethoxy)-pyridin-3-yl)-2-oxo-2,3-dihydro-1H-indol-5-carbonitril;
3-[4-(4-Ethyl-piperazin-1-yl)-phenylamino]-1-(4-methoxy-phenylsulfonyl)-3-(2-(2,2,2-trifluorethoxy)-pyridin-3-yl)-2-oxo-2,3-dihydro-1H-indol-5-carbonitril;
3-(2-(2,2-Difluorethoxy)-phenyl)-3-[4-(4-ethyl-piperazin-1-yl)-phenylamino]-1-(4-methoxy-phenylsulfonyl)-2-oxo-2,3-dihydro-1H-indol-5-carbonitril;
1-(2,4-Dimethoxy-phenylsulfonyl)-3-[4-(4-methyl-piperazin-1-yl)-phenylamino]-3-(2-(trifluormethoxy)-phenyl)-2-oxo-2,3-dihydro-1H-indol-5-carbonitril;
und deren pharmazeutisch verträglichen Salzen.

23. Verbindungen der Formel XVIII 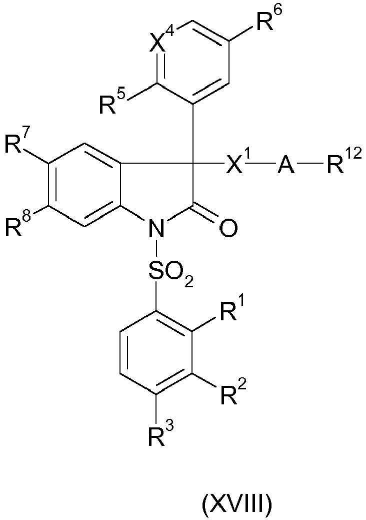 worin R¹, R², R³, R⁵, R⁶, R⁷, R⁸, X¹, X⁴ und A wie in einem der vorhergehenden Ansprüche definiert sind und
R¹² für Halogen, CN, OR^{a} oder NR^{b}R^{c} und vorzugsweise für Halogen steht, worin
R^{a} für H, C₁-C₄-Alkyl, Phenyl oder Benzyl steht; und
R^{b} und R^{c} unabhängig voneinander für H, C₁-C₄-Alkyl, C₁-C₄-Alkoxy, Phenyl oder Benzyl stehen.

24. Pharmazeutisches Mittel, enthaltend wenigstens eine Verbindung der Formel I gemäß der Definition in einem der Ansprüche 1 bis 22 und/oder wenigstens ein pharmazeutisch verträgliches Salz davon und wenigstens einen pharmazeutisch verträglichen Träger.

25. Verwendung von Verbindungen der Formel I gemäß der Definition in einem der Ansprüche 1 bis 22 oder von pharmazeutisch verträglichen Salzen davon zur Herstellung eines Medikaments zur Behandlung und/oder Prophylaxe von Vasopressin-abhängigen Krankheiten.

26. Verwendung von Verbindungen der Formel I gemäß der Definition in einem der Ansprüche 1 bis 22 oder von pharmazeutisch verträglichen Salzen davon zur Herstellung eines Medikaments zur Behandlung und/oder Prophylaxe von Krankheiten, die ausgewählt sind unter
Diabetes, Insulin-Resistenz, Enuresis nocturna, Inkontinenz, Krankheiten, bei denen Blutgerinnungsstörungen auftreten,
Hypertonie, pulmonaler Hypertonie, Herzinsuffizienz, Myocardinfarkt, koronarem Spasmus, instabiler Angina, PTCA (percutaneous transluminal coronary angioplastie), Ischämien des Herzens, Störungen des renalen Systems, Ödemen,
renalem Vasospasmus, Nekrose des renalen Cortex, Hyponatriämie, Hypokaliämie, Schwartz-Bartter Syndrom, Störungen des Gastrointestinaltraktes, gastritischem Vasospasmus, Hepatozirrhose, Magen- und Darmulkus, Emesis, auftretender Emesis bei der Chemotherapie, Reisekrankheit,
affektiven Störungen,
Angststörungen, stressabhängigen Angststörungen,
Gedächtnisleistungsstörungen, Morbus Alzheimer,
Psychosen, psychotischen Störungen,
Cushing-Syndrom, sonstigen stress-abhängigen Krankheiten, Schlafstörungen,
depressiven Erkrankungen, vorzugsweise childhood onset mood disorders,
vasomotorischen Symptomen, thermoregulatorischen Fehlfunktionen, Drogen-, Arzneimittel- und/oder durch sonstige Faktoren vermittelten Abhängigkeiten; von Stress bedingt durch den Entzug von einem oder mehreren die Abhängigkeit vermittelnden Faktoren; und/oder von Stress-induzierten Rückfällen in die Drogen-, Arzneimittel- und/oder durch sonstige Faktoren vermittelte Abhängigkeiten,
Schizophrenie und Psychose,
und/oder zur Verzögerung der Miktion.

## Claims

1. Compounds of the formula I 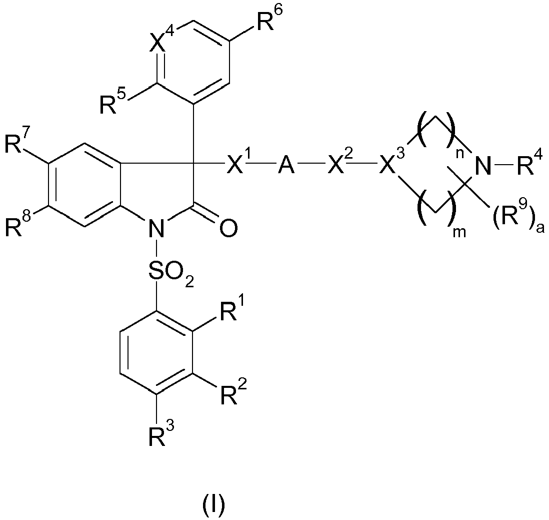 in which
X¹ is -O-, -O-CH₂-, -O-C (=O) -, -NR¹¹-, -NR¹¹-CH₂- or -NR¹¹-C (=O) -;
X² is a single bond, CO or CH₂;
X³ is N or CH;
X⁴ is N or CH;
A is phenylene or 6-membered hetarylene with 1 or 2 nitrogen atoms as ring members, where phenylene or hetarylene may be substituted by 1 or 2 radicals R¹⁰;
R¹ and R³ are independently of one another hydrogen, C₁-C₃-alkyl, C₁-C₃-fluoroalkyl, C₁-C₃-alkoxy, C₁-C₃-fluoroalkoxy, halogen or CN;
R² is hydrogen or methoxy;
where at least one of the radicals R¹, R² and R³ is hydrogen;
R⁴ is hydrogen or C₁-C₄-alkyl;
R⁵ is ethoxy, fluorinated ethoxy, fluoromethoxy, difluoromethoxy, trifluoromethoxy or iso-propoxy;
R⁶ is hydrogen or methyl;
R⁷ is hydrogen, I, Br, Cl, F or CN;
R⁸ is hydrogen, I, Br, Cl, F or CN;
R⁹ is C₁-C₃-alkyl or C₁-C₃-fluoroalkyl;
R¹⁰ is C₁-C₃-alkyl, C₁-C₃-fluoroalkyl, C₁-C₃-alkoxy or C₁-C₃-fluoroalkoxy;
R¹¹ is hydrogen, C₁-C₄-alkyl, C₁-C₄-fluoroalkyl, C₁-C₄-alkoxy or C₁-C₄-fluoroalkoxy;
a is 0, 1 or 2; and
m and n are independently of one another 1 or 2;
and their pharmaceutically acceptable salts.

2. Compounds according to Claim 1, in which R¹ is hydrogen, methoxy, ethoxy, fluoromethoxy, difluoromethoxy or trifluoromethoxy, preferably is hydrogen, methoxy or trifluoromethoxy and in particular is hydrogen or methoxy.

3. Compounds according to either of the preceding claims, in which R² is hydrogen.

4. Compounds according to any of the preceding claims, in which R³ is hydrogen or methoxy.

5. Compounds according to any of the preceding claims, in which R⁴ is hydrogen, methyl or ethyl.

6. Compounds according to any of the preceding claims, in which R⁵ is ethoxy or fluorinated ethoxy, preferably is ethoxy, 2,2-difluoroethoxy or 2,2,2-trifluoroethoxy and in particular is ethoxy.

7. Compounds according to any of the preceding claims, in which R⁶ is hydrogen.

8. Compounds according to any of the preceding claims, in which R⁷ is CN and R⁸ is H or F.

9. Compounds according to any of Claims 1 to 7, in which R⁷ is I and R⁸ is H or F.

10. Compounds according to any of Claims 1 to 7, in which R⁷ is Cl and R⁸ is H or F.

11. Compounds according to any of the preceding claims, in which R¹⁰ is methyl or methoxy.

12. Compounds according to any of the preceding claims, in which R¹¹ is H.

13. Compounds according to any of the preceding claims, in which a is 0.

14. Compounds according to any of the preceding claims, in which X¹ is -O-C(=O)-, -NH-, -NH-CH₂- or -NH-C(=O)- and preferably is -NH-CH₂-.

15. Compounds according to any of the preceding claims, in which X² is a single bond.

16. Compounds according to any of the preceding claims, in which X³ is N, and m and n are both 2.

17. Compounds according to any of Claims 1 to 15, in which X³ is CH, and m and n are both 2, or are both 1, or m is 1 and n is 2.

18. Compounds according to any of the preceding claims, in which X⁴ is N.

19. Compounds according to any of the preceding claims, in which A is phenylene, preferably is 1,4- or 1,3-phenylene.

20. Compounds according to any of Claims 1 to 18, in which A is pyridylene or pyrimidylene, preferably is 3,5- or 3,6-pyridylene.

21. Compounds according to any of Claims 1 to 19, in which
R¹ is H or methoxy;
R² is H;
R³ is methoxy;
R⁴ is H, methyl or ethyl;
R⁵ is ethoxy;
R⁶ is H;
R⁷ is CN;
R⁸ is H or F;
X¹ is -NH- or -NHCH₂-;
X² is a single bond;
X³ is N;
X⁴ is N;
A is 1,4-phenylene;
a is 0; and
m and n are 2;
and in which preferably
R¹ is H or methoxy;
R² is H;
R³ is methoxy;
R⁴ is H or methyl;
R⁵ is ethoxy;
R⁶ is H;
R⁷ is CN;
R⁸ is H or F;
X¹ is -NHCH₂-;
X² is a single bond;
X³ is N;
X⁴ is N;
A is 1,4-phenylene;
a is 0, and
m and n are 2.

22. Compounds according to Claim 1, selected from the group consisting of
1-(2,4-dimethoxyphenylsulphonyl)-3-(2-ethoxy-pyridin-3-yl)-5-iodo-2-oxo-2,3-dihydro-1H-indol-3-yl 4-(4-methylpiperazin-1-yl)benzoate;
5-cyano-3-(2-ethoxypyridin-3-yl)-1-(4-methoxy-phenylsulphonyl)-2-oxo-2,3-dihydro-1H-indol-3-yl 4-(4-methylpiperazin-1-yl)benzoate;
5-cyano-1-(2,4-dimethoxyphenylsulphonyl)-3-(2-ethoxypyridin-3-yl)-2-oxo-2,3-dihydro-1H-indol-3-yl 4-(4-methylpiperazin-1-yl)benzoate;
N-[5-cyano-3-(2-ethoxypyridin-3-yl)-1-(4-methoxy-phenylsulphonyl)-2-oxo-2,3-dihydro-1H-indol-3-yl]-4-(4-methylpiperazin-1-yl)benzamide;
N-[5-cyano-3-(2-ethoxypyridin-3-yl)-1-(4-methoxy-phenylsulphonyl)-2-oxo-2,3-dihydro-1H-indol-3-yl]-4-(4-ethylpiperazin-1-yl)benzamide;
N-[5-cyano-1-(2,4-dimethoxyphenylsulphonyl)-3-(2-ethoxypyridin-3-yl)-2-oxo-2,3-dihydro-1H-indol-3-yl]-4-(4-methylpiperazin-1-yl)benzamide;
N-[5-cyano-1-(2,4-dimethoxyphenylsulphonyl)-3-(2-ethoxypyridin-3-yl)-2-oxo-2,3-dihydro-1H-indol-3-yl]-4-(4-ethylpiperazin-1-yl)benzamide;
N-[1-[4-(2,2,-difluoroethoxy)phenylsulphonyl]-3-(2-ethoxypyridin-3-yl)-5-iodo-2-oxo-2,3-dihydro-1H-indol-3-yl]-4-(4-methylpiperazin-1-yl)benzamide;
1-(2,4-dimethoxyphenylsulphonyl)-3-(2-ethoxy-pyridin-3-yl)-5-iodo-3-[4-(4-methylpiperazin-1-yl)benzylamino]-1,3-dihydro-indol-2-one;
1-(2,4-dimethoxyphenylsulphonyl)-3-(2-ethoxy-pyridin-3-yl)-5-iodo-3-(4-piperazin-1-yl-benzylamino)-1,3-dihydroindol-2-one;
5-chloro-3-(2-ethoxypyridin-3-yl)-1-(4-methoxy-phenylsulphonyl)-3-[4-(4-methylpiperazin-1-yl)benzylamino]-1,3-dihydroindol-2-one;
5-chloro-3-(2-ethoxypyridin-3-yl)-6-fluoro-1-(4-methoxyphenylsulphonyl)-3-[4-(4-methylpiperazin-1-yl)benzylamino]-1,3-dihydroindol-2-one;
6-chloro-3-(2-ethoxypyridin-3-yl)-5-fluoro-1-(4-methoxyphenylsulphonyl)-3-[4-(4-methylpiperazin-1-yl)benzylamino]-1,3-dihydroindol-2-one;
5,6-difluoro-3-(2-ethoxypyridin-3-yl)-1-(4-methoxyphenylsulphonyl)-3-[4-(4-methylpiperazin-1-yl)benzylamino]-1,3-dihydroindol-2-one;
3-(2-ethoxypyridin-3-yl)-5-fluoro-1-(4-methoxy-phenylsulphonyl)-3-[4-(4-methylpiperazin-1-yl)-benzylamino]-1,3-dihydroindol-2-one;
3-(2-ethoxypyridin-3-yl)-1-(4-methoxyphenyl-sulphonyl)-3-[4-(4-methylpiperazin-1-yl)benzyl-amino]-2-oxo-2,3-dihydro-1H-indole-5-carbonitrile;
1-(2,4-dimethoxyphenylsulphonyl)-3-(2-ethoxy-pyridin-3-yl)-3-[4-(4-methylpiperazin-1-yl)benzyl-amino]-2-oxo-2,3-dihydro-1H-indole-5-carbonitrile;
3-(2-ethoxypyridin-3-yl)-1-(4-methoxy-2-trifluoro-methoxyphenylsulphonyl)-3-[4-(4-methylpiperazin-1-yl)benzylamino]-2-oxo-2,3-dihydro-1H-indole-5-carbonitrile;
3-(2-ethoxypyridin-3-yl)-3-[4-(4-methylpiperzin-1-yl)benzylamino]-2-oxo-1-(4-trifluoromethoxyphenyl-sulphonyl)-2,3-dihydro-1H-indole-5-carbonitrile;
3-(2-ethoxypyridin-3-yl)-3-[4-(4-methylpiperzin-1-yl)benzylamino]-2-oxo-1-[4-(2,2,2-trifluoroethoxy)phenylsulphonyl]-2,3-dihydro-1H-indole-5-carbonitrile;
1-[4-(2,2-difluoroethoxy)phenylsulphonyl]-3-(2-ethoxypyridin-3-yl)-3-[4-(4-methylpiperazin-1-yl)-benzylamino]-2-oxo-2,3-dihydro-1H-indole-5-carbonitrile;
3-(2-ethoxypyridin-3-yl)-1-[4-(2-fluoroethoxy)-phenylsulphonyl]-3-[4-(4-methylpiperazin-1-yl)-benzylamino]-2-oxo-2,3-dihydro-1H-indole-5-carbonitrile;
1-(4-difluoromethoxyphenylsulphonyl)-3-(2-ethoxy-pyridin-3-yl)-3-[4-(4-methylpiperazin-1-yl)-benzylamino]-2-oxo-2,3-dihydro-1H-indole-5-carbonitrile;
3-(2-ethoxypyridin-3-yl)-1-(2-methoxyphenyl-sulphonyl)-3-[4-(4-methylpiperazin-1-yl)benzyl-amino]-2-oxo-2,3-dihydro-1H-indole-5-carbonitrile;
3-(2-ethoxypyridin-3-yl)-1-(3-methoxyphenyl-sulphonyl)-3-[4-(4-methylpiperazin-1-yl)benzyl-amino]-2-oxo-2,3-dihydro-1H-indole-5-carbonitrile;
1-(4-chlorophenylsulphonyl)-3-(2-ethoxypyridin-3-yl)-3-[4-(4-methylpiperazin-1-yl)benzylamino]-2-oxo-2,3-dihydro-1H-indole-5-carbonitrile;
1-phenylsulphonyl-3-(2-ethoxypyridin-3-yl)-3-[4-(4-methylpiperazin-1-yl)benzylamino]-2-oxo-2,3-dihydro-1H-indole-5-carbonitrile;
1-(4-cyanophenylsulphonyl)-3-(2-ethoxypyridin-3-yl)-3-[4-(4-methylpiperazin-1-yl)benzylamino]-2-oxo-2,3-dihydro-1H-indole-5-carbonitrile;
1-(2,4-dimethoxyphenylsulphonyl)-3-(2-ethoxy-pyridin-3-yl)-2-oxo-3-(4-piperazin-1-yl-benzylamino)-2,3-dihydro-1H-indole-5-carbonitrile;
3-(2-ethoxypyridin-3-yl)-1-(4-methoxyphenyl-sulphonyl)-2-oxo-3-(4-piperazin-1-yl-benzylamino)-2,3-dihydro-1H-indole-5-carbonitrile;
3-(2-ethoxypyridin-3-yl)-3-[4-(4-ethylpiperazin-1-yl)benzylamino]-1-(4-methoxyphenylsulphonyl)-2-oxo-2,3-dihydro-1H-indole-5-carbonitrile;
1-(2,4-dimethoxyphenylsulphonyl)-3-(2-ethoxy-pyridin-3-yl)-3-[4-(4-ethylpiperazin-1-yl)benzyl-amino]-2-oxo-2,3-dihydro-1H-indole-5-carbonitrile;
3-(2-ethoxypyridin-3-yl)-6-fluoro-1-(4-methoxy-phenylsulphonyl)-3-[4-(4-methylpiperazin-1-yl)-benzylamino]-2-oxo-2,3-dihydro-1H-indole-5-carbonitrile;
3-(2-ethoxypyridin-3-yl)-6-fluoro-1-(4-methoxy-phenylsulphonyl)-2-oxo-3-(4-piperazin-1-yl-benzylamino)-2,3-dihydro-1H-indole-5-carbonitrile;
3-(2-ethoxypyridin-3-yl)-3-[4-(4-ethylpiperazin-1-yl)benzylamino]-6-fluoro-1-(4-methoxyphenyl-sulphonyl)-2-oxo-2,3-dihydro-1H-indole-5-carbonitrile;
1-[4-(2,2-difluoroethoxy)phenylsulphonyl]-3-(2-ethoxypyridin-3-yl)-6-fluoro-3-[4-(4-methyl-piperazin-1-yl)benzylamino]-2-oxo-2,3-dihydro-1H-indole-5-carbonitrile;
3-(2-ethoxyphenyl)-1-(4-methoxyphenylsulphonyl)-3-[4-(4-methylpiperazin-1-yl)benzylamino]-2-oxo-2,3-dihydro-1H-indole-5-carbonitrile;
1-(2,4-dimethoxyphenylsulphonyl)-3-(2-ethoxy-pyridin-3-yl)-5-iodo-3-[3-(4-methylpiperazin-1-yl)benzylamino]-1,3-dihydroindo-2-one;
3-(2-ethoxypyridin-3-yl)-5-iodo-1-(4-methoxy-2-trifluoromethoxyphenylsulphonyl)-3-[3-(4-methyl-piperazin-1-yl)benzylamino]-1,3-dihydroindol-2-one;
3-(2-ethoxypyridin-3-yl)-5-iodo-1-(4-methoxy-phenylsulphonyl)-3-[3-(4-methylpiperazin-1-yl)-benzylamino]-1,3-dihydroindol-2-one;
3-(2-ethoxypyridin-3-yl)-5-iodo-1-(2-methylphenyl-sulphonyl)-3-[3-(4-methylpiperazin-1-yl)benzyl-amino]-1,3-dihydroindol-2-one;
1-(2,4-dimethoxyphenylsulphonyl)-3-(2-ethoxy-pyridin-3-yl)-3-[3-(4-methylpiperazin-1-yl)benzyl-amino]-2-oxo-2,3-dihydro-1H-indole-5-carbonitrile;
3-(2-ethoxypyridin-3-yl)-1-(4-methoxyphenyl-sulphonyl)-3-[3-(4-methylpiperazin-1-yl)benzyl-amino]-2-oxo-2,3-dihydro-1H-indole-5-carbonitrile;
3-(2-ethoxypyridin-3-yl)-1-(2-methoxyphenyl-sulphonyl)-3-[3-(4-methylpiperazin-1-yl)benzyl-amino]-2-oxo-2,3-dihydro-1H-indole-5-carbonitrile;
1-(2,4-dimethoxyphenylsulphonyl)-3-(2-ethoxy-pyridin-3-yl)-3-[2-(4-methylpiperazin-1-yl)benzylamino]-2-oxo-2,3-dihydro-1H-indole-5-carbonitrile;
3-(2-ethoxypyridin-3-yl)-1-(4-methoxyphenyl-sulphonyl)-3-[2-(4-methylpiperazin-1-yl)benzylamino]-2-oxo-2,3-dihydro-1H-indole-5-carbonitrile;
3-(2-ethoxypyridin-3-yl)-1-(2-methoxyphenyl-sulphonyl)-3-[2-(4-methylpiperazin-1-yl)benzylamino]-2-oxo-2,3-dihydro-1H-indole-5-carbonitrile;
1-(2,4-dimethoxyphenylsulphonyl)-3-(2-ethoxy-pyridin-3-yl)-3-[4-(4-ethylpiperazin-1-yl)phenyl-amino]-5-iodo-1,3-dihydroindol-2-one;
1-(2,4-dimethoxyphenylsulphonyl)-3-(2-ethoxy-pyridin-3-yl)-5-iodo-3-[4-(4-methylpiperazin-1-yl)phenylamino]-1,3-dihydroindol-2-one;
3-(2-ethoxypyridin-3-yl)-5-iodo-1-(4-methoxy-phenylsulphonyl)-3-[4-(4-methylpiperazin-1-yl)phenylamino]-1,3-dihydroindol-2-one;
5-chloro-3-(2-ethoxypyridin-3-yl)-1-(4-methoxy-phenylsulphonyl)-3-[4-(4-methylpiperazin-1-yl)phenylamino]-1,3-dihydroindol-2-one;
5-chloro-3-(2-ethoxypyridin-3-yl)-6-fluoro-1-(4-methoxyphenylsulphonyl)-3-[4-(4-methylpiperazin-1-yl)phenylamino]-1,3-dihydroindol-2-one;
6-chloro-3-(2-ethoxypyridin-3-yl)-5-fluoro-1-(4-methoxyphenylsulphonyl)-3-[4-(4-methylpiperazin-1-yl)phenylamino]-1,3-dihydroindol-2-one;
3-(2-ethoxypyridin-3-yl)-5,6-difluoro-1-(4-methoxyphenylsulphonyl)-3-[4-(4-methylpiperazin-1-yl)phenylamino]-1,3-dihydroindol-2-one;
1-(2,4-dimethoxyphenylsulphonyl)-3-(2-ethoxy-pyridin-3-yl)-3-[4-(4-methylpiperazin-1-yl)phenyl-amino]-2-oxo-2,3-dihydro-1H-indole-5-carbonitrile;
1-(2,4-dimethoxyphenylsulphonyl)-3-(2-ethoxy-pyridin-3-yl)-3-[4-(4-ethylpiperazin-1-yl)phenyl-amino]-2-oxo-2,3-dihydro-1H-indole-5-carbonitrile;
3-(2-ethoxypyridin-3-yl)-3-[4-(4-ethylpiperazin-1-yl)phenylamino]-1-(4-methoxyphenylsulphonyl)-2-oxo-2,3-dihydro-1H-indole-5-carbonitrile;
3-(2-ethoxypyridin-3-yl)-3-[4-(4-ethylpiperazin-1-yl)phenylamino]-1-(2-methoxyphenylsulphonyl)-2-oxo-2,3-dihydro-1H-indole-5-carbonitrile;
3-(2-ethoxypyridin-3-yl)-6-fluoro-1-(4-methoxy-phenylsulphonyl)-3-[4-(4-methylpiperazin-1-yl)-phenylamino]-2-oxo-2,3-dihydro-1H-indole-5-carbonitrile;
1-(2,4-dimethoxyphenylsulphonyl)-3-(2-ethoxy-pyridin-3-yl)-5-iodo-3-[3-(4-methylpiperazin-1-yl)phenylamino]-1,3-dihydro-2-one;
3-(2-ethoxypyridin-3-yl)-1-(4-methoxyphenyl-sulphonyl)-3-[3-(4-methylpiperazin-1-yl)phenyl-amino]-2-oxo-2,3-dihydro-1H-indole-5-carbonitrile;
1-(2,4-dimethoxyphenylsulphonyl)-3-(2-ethoxy-pyridin-3-yl)-3-[3-(4-methylpiperazin-1-yl)phenyl-amino]-2-oxo-2,3-dihydro-1H-indole-5-carbonitrile;
3-(4-azetidin-3-yl-phenylamino)-1-(2,4-dimethoxy-phenylsulphonyl)-3-(2-ethoxypyridin-3-yl)-5-iodo-1,3-dihydroindol-2-one;
1-(2,4-dimethoxyphenylsulphonyl)-3-(2-ethoxy-pyridin-3-yl)-3-[4-(1-ethylazetidin-3-yl)phenyl-amino]-5-iodo-1,3-dihydroindol-2-one;
3-(4-azetidin-3-yl-phenylamino)-3-(2-ethoxypyridin-3-yl)-5-iodo-1-(4-methoxyphenylsulphonyl)-1,3-dihydroindol-2-one;
3-(2-ethoxypyridin-3-yl)-3-[4-(1-ethylazetidin-3-yl)phenylamino]-5-iodo-1-(4-methoxyphenyl-sulphonyl)-1,3-dihydroindol-2-one;
1-(2,4-dimethoxyphenylsulphonyl)-3-(2-ethoxy-pyridin-3-yl)-5-iodo-3-[6-(4-methylpiperazin-1-yl)pyridin-3-ylamino]-1,3-dihydroindol-2-one;
3-(2-ethoxypyridin-3-yl)-5-iodo-1-(4-methoxy-phenylsulphonyl)-3-[6-(4-methylpiperazin-1-yl)pyridin-3-ylamino]-1,3-dihydroindol-2-one;
1-phenylsulphonyl-3-(2-ethoxypyridin-3-yl)-5-iodo-3-[6-(4-methylpiperazin-1-yl)pyridin-3-ylamino]-1,3-dihydroindol-2-one;
3-(2-ethoxypyridin-3-yl)-5-iodo-1-(4-methoxy-phenylsulphonyl)-3-[4-(4-methylpiperazin-1-carbonyl)phenylamino]-1,3-dihydroindol-2-one;
1-(2,4-dimethoxyphenylsulphonyl)-3-(2-ethoxy-pyridin-3-yl)-5-iodo-3-[4-(4-methylpiperazin-1-carbonyl)phenylamino]-1,3-dihydroindol-2-one;
3-(2-ethoxypyridin-3-yl)-1-(4-methoxyphenyl-sulphonyl)-3-[4-(4-methylpiperazin-1-carbonyl)-phenylamino]-2-oxo-2,3-dihydro-1H-indole-5-carbonitrile;
1-(2,4-dimethoxyphenylsulphonyl)-3-(2-ethoxy-pyridin-3-yl)-3-[4-(4-methylpiperazin-1-carbonyl)-phenylamino]-2-oxo-2,3-dihydro-1H-indole-5-carbonitrile;
1-(2,4-dimethoxyphenylsulphonyl)-3-(2-ethoxy-pyridin-3-yl)-3-[4-(4-methylpiperazin-1-ylmethyl)-phenylamino]-2-oxo-2,3-dihydro-1H-indole-5-carbonitrile;
3-(2-ethoxypyridin-3-yl)-1-(4-methoxyphenyl-sulphonyl)-3-[4-(4-methylpiperazin-1-ylmethyl)-.phenylamino]-2-oxo-2,3-dihydro-1H-indole-5-carbonitrile;
3-(2-(2,2-difluoroethoxy)pyridin-3-yl)-1-(4-methoxyphenylsulphonyl)-3-[4-(4-methylpiperazin-1-yl)benzylamino]-2-oxo-2,3-dihydro-1H-indole-5-carbonitrile;
1-(4-methoxyphenylsulphonyl)-3-(2-(2,2,2-trifluoroethoxy)pyridin-3-yl)-3-[4-(4-methyl-piperazin-1-yl)benzylamino]-2-oxo-2,3-dihydro-1H-indole-5-carbonitrile;
3-(2-(2,2-difluoroethoxy)phenyl)-1-(4-methoxy-phenylsulphonyl)-3-[4-(4-methylpiperazin-1-yl)-benzylamino]-2-oxo-2,3-dihydro-1H-indole-5-carbonitrile;
3-[4-(4-ethylpiperazin-1-yl)phenylamino]-1-(4-methoxyphenylsulphonyl)-3-(2-(2,2-difluoroethoxy)-pyridin-3-yl)-2-oxo-2,3-dihydro-1H-indole-5-carbonitrile;
3-[4-(4-ethylpiperazin-1-yl)phenylamino]-1-(4-methoxyphenylsulphonyl)-3-(2-(2,2,2-trifluoroethoxy)pyridin-3-yl)-2-oxo-2,3-dihydro-1H-indole-5-carbonitrile;
3-(2-(2,2-difluoroethoxy)phenyl)-3-[4-(4-ethyl-piperazin-1-yl)phenylamino]-1-(4-methoxyphenyl-sulphonyl)-2-oxo-2,3-dihydro-1H-indole-5-carbonitrile;
1-(2,4-dimethoxyphenylsulphonyl)-3-[4-(4-methyl-piperazin-1-yl)phenylamino]-3-(2-(trifluoromethoxy)phenyl-2-oxo-2,3-dihydro-1H-indole-5-carbonitrile;
and their pharmaceutically acceptable salts.

23. Compounds of the formula XVIII 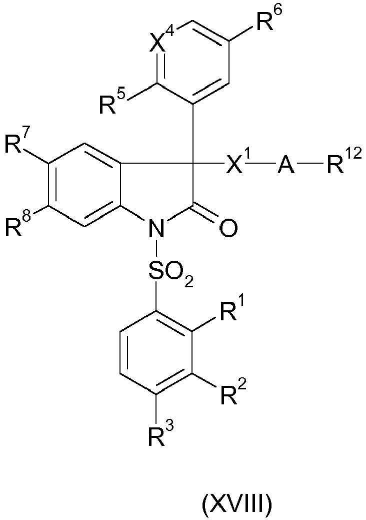 in which R¹, R², R³, R⁵, R⁶, R⁷, R⁸, X¹, X⁴ and A are as defined in any of the preceding claims, and
R¹² is halogen, CN, OR^{a} or NR^{b}R^{c} and preferably is halogen, in which
R^{a} is H, C₁-C₄-alkyl, phenyl or benzyl; and
R^{b} and R^{c} are independently of one another H, C₁-C₄-alkyl, C₁-C₄-alkoxy, phenyl or benzyl.

24. Pharmaceutical composition comprising at least one compound of the formula I as defined in any of Claims 1 to 22 and/or at least one pharmaceutically acceptable salt thereof and at least one pharmaceutically acceptable carrier.

25. Use of compounds of the formula I as defined in any of Claims 1 to 22 or of pharmaceutically acceptable salts thereof for the manufacture of a medicament for the treatment and/or prophylaxis of vasopressin-dependent diseases.

26. Use of compounds of the formula I as defined in any of Claims 1 to 22 or of pharmaceutically acceptable salts thereof for the manufacture of a medicament for the treatment and/or prophylaxis of diseases selected from
diabetes, insulin resistance, nocturnal enuresis, incontinence, diseases in which impairments of blood clotting occur,
hypertension, pulmonary hypertension, heart failure, myocardial infarction, coronary spasm, unstable angina, PTCA (percutaneous transluminal coronary angioplasty), ischemias of the heart, impairments of the renal system, edemas, renal vasospasm, necrosis of the renal cortex, hyponatremia, hypokalemia, Schwartz-Bartter syndrome, impairments of the gastrointestinal tract, gastritic vasospasm, hepatocirrhosis, gastric and intestinal ulcers, emesis, emesis occurring during chemotherapy, travel sickness, affective disorders. anxiety disorders, stress-dependent anxiety disorders, memory impairments, Alzheimer's disease, psychoses, psychotic disorders, Cushing's syndrome, other stress-dependent diseases, sleep isorders, depressive disorders, preferably childhood onset mood disorders, vasomotor symptoms, thermoregulatory dysfunctions, drug or pharmaceutical dependencies and/or dependencies mediated by other factors; of stress caused by withdrawal of one or more factors mediating the dependence; and/or of stress-induced relapses into drug or pharmaceutical dependencies and/or dependencies mediated by other factors, schizophrenia and psychosis and/or for delaying micturition.

## Revendications

1. Composés de formule I 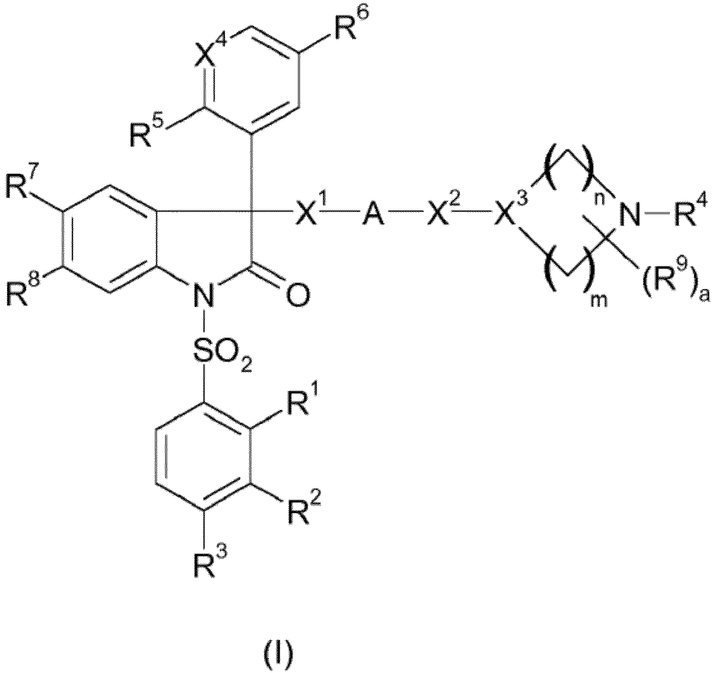 dans laquelle
X¹ représente -O-, -O-CH₂-, -O-C (=O) -, -NR¹¹-, -NR¹¹-CH₂- ou -NR¹¹-C (=O) - ;
X² représente une simple liaison, CO ou CH₂ ;
X³ représente N ou CH ;
X⁴ représente N ou CH ;
A représente phénylène ou hétarylène à 6 éléments contenant 1 ou 2 atomes d'azote en tant qu'éléments de cycle, le phénylène ou l'hétarylène pouvant être substitué par 1 ou 2 radicaux R¹⁰ ;
R¹ et R³ représentent indépendamment l'un de l'autre hydrogène, alkyle en C₁-C₃, fluoroalkyle en C₁-C₃, alcoxy en C₁-C₃, fluoroalcoxy en C₁-C₃, halogène ou CN ;
R² représente hydrogène ou méthoxy ;
au moins un des radicaux R¹, R² et R³ représentant hydrogène ;
R⁴ représente hydrogène ou alkyle en C₁-C₄ ;
R⁵ représente éthoxy, éthoxy fluoré, fluorométhoxy, difluorométhoxy, trifluorométhoxy ou isopropoxy ;
R⁶ représente hydrogène ou méthyle ;
R⁷ représente hydrogène, I, Br, Cl, F ou CN ;
R⁸ représente hydrogène, I, Br, Cl, F ou CN ;
R⁹ représente alkyle en C₁-C₃ ou fluoroalkyle en C₁-C₃ ;
R¹⁰ représente alkyle en C₁-C₃, fluoroalkyle en C₁-C₃, alcoxy en C₁-C₃ ou fluoroalcoxy en C₁-C₃ ;
R¹¹ représente hydrogène, alkyle en C₁-C₄, fluoroalkyle en C₁-C₄, alcoxy en C₁-C₄ ou fluoroalcoxy en C₁-C₄ ;
a représente 0, 1 ou 2 ; et
m et n représentent indépendamment l'un de l'autre 1 ou 2 ;
ainsi que leurs sels pharmaceutiquement compatibles.

2. Composés selon la revendication 1, dans lesquels R¹ représente hydrogène, méthoxy, éthoxy, fluorométhoxy, difluorométhoxy ou trifluorométhoxy, de préférence hydrogène, méthoxy ou trifluorométhoxy, et notamment hydrogène ou méthoxy.

3. Composés selon l'une quelconque des revendications précédentes, dans lesquels R² représente hydrogène.

4. Composés selon l'une quelconque des revendications précédentes, dans lesquels R³ représente hydrogène ou méthoxy.

5. Composés selon l'une quelconque des revendications précédentes, dans lesquels R⁴ représente hydrogène, méthyle ou éthyle.

6. Composés selon l'une quelconque des revendications précédentes, dans lesquels R⁵ représente éthoxy ou éthoxy fluoré, de préférence éthoxy, 2,2-difluoroéthoxy ou 2,2,2-trifluoroéthoxy, et notamment éthoxy.

7. Composés selon l'une quelconque des revendications précédentes, dans lesquels R⁶ représente hydrogène.

8. Composés selon l'une quelconque des revendications précédentes, dans lesquels R⁷ représente CN et R⁸ représente H ou F.

9. Composés selon l'une quelconque des revendications 1 à 7, dans lesquels R⁷ représente I et R⁸ représente H ou F.

10. Composés selon l'une quelconque des revendications 1 à 7, dans lesquels R⁷ représente Cl et R⁸ représente H ou F.

11. Composés selon l'une quelconque des revendications précédentes, dans lesquels R¹⁰ représente méthyle ou méthoxy.

12. Composés selon l'une quelconque des revendications précédentes, dans lesquels R¹¹ représente H.

13. Composés selon l'une quelconque des revendications précédentes, dans lesquels a représente 0.

14. Composés selon l'une quelconque des revendications précédentes, dans lesquels X¹ représente -O-C(=O)-, -NH-, -NH-CH₂- ou -NH-C(=O)-, et de préférence -NH-CH₂-.

15. Composés selon l'une quelconque des revendications précédentes, dans lesquels X² représente une simple liaison.

16. Composés selon l'une quelconque des revendications précédentes, dans lesquels X³ représente N, et m et n représentent tous les deux 2.

17. Composés selon l'une quelconque des revendications 1 à 15, dans lesquels X³ représente CH, et m et n représentent tous les deux 2, ou tous les deux 1, ou m représente 1 et n représente 2.

18. Composés selon l'une quelconque des revendications précédentes, dans lesquels X⁴ représente N.

19. Composés selon l'une quelconque des revendications précédentes, dans lesquels A représente phénylène, de préférence 1,4- ou 1,3-phénylène.

20. Composés selon l'une quelconque des revendications 1 à 18, dans lesquels A représente pyridylène ou pyrimidylène, de préférence 3,5- ou 3,6-pyridylène.

21. Composés selon l'une quelconque des revendications 1 à 19, dans lesquels
R¹ représente H ou méthoxy ;
R² représente H ;
R³ représente méthoxy ;
R⁴ représente H, méthyle ou éthyle ;
R⁵ représente éthoxy ;
R⁶ représente H ;
R⁷ représente CN ;
R⁸ représente H ou F ;
X¹ représente -NH- ou -NHCH₂- ;
X² représente une simple liaison ;
X³ représente N ;
X⁴ représente N ;
A représente 1,4-phénylène ;
a représente 0 ; et
m et n représentent 2 ;
et de préférence
R¹ représente H ou méthoxy ;
R² représente H ;
R³ représente méthoxy ;
R⁴ représente H ou méthyle ;
R⁵ représente éthoxy ;
R⁶ représente H ;
R⁷ représente CN ;
R⁸ représente H ou F ;
X¹ représente -NHCH₂- ;
X² représente une simple liaison ;
X³ représente N ;
X⁴ représente N ;
A représente 1,4-phénylène ;
a représente 0 ; et
m et n représentent 2.

22. Composés selon la revendication 1, choisis parmi :
l'ester 1-(2,4-diméthoxy-phénylsulfonyl)-3-(2-éthoxy-pyridin-3-yl)-5-iodo-2-oxo-2,3-dihydro-1H-indol-3ylique de l'acide 4-(4-méthyl-pipérazin-1-yl)-benzoïque ;
l'ester 5-cyano-3-(2-éthoxy-pyridin-3-yl)-1-(4-méthoxy-phénylsulfonyl)-2-oxo-2,3-dihydro-1H-indol-3ylique de l'acide 4-(4-méthyl-pipérazin-1-yl)-benzoïque ;
l'ester 5-cyano-1-(2,4-diméthoxy-phénylsulfonyl)-3-(2-éthoxy-pyridin-3-yl)-2-oxo-2,3-dihydro-1H-indol-3ylique de l'acide 4-(4-méthyl-pipérazin-1-yl)-benzoïque ;
le N-[5-cyano-3-(2-éthoxy-pyridin-3-yl)-1-(4-méthoxy-phénylsulfonyl)-2-oxo-2,3-dihydro-1H-indol-3-yl]-4-(4-méthyl-pipérazin-1-yl)-benzamide ;
le N-[5-cyano-3-(2-éthoxy-pyridin-3-yl)-1-(4-méthoxy-phénylsulfonyl)-2-oxo-2,3-dihydro-1H-indol-3-yl]-4-(4-éthyl-pipérazin-1-yl)-benzamide ;
le N-[5-cyano-1-(2,4-diméthoxy-phénylsulfonyl)-3-(2-éthoxy-pyridin-3-yl)-2-oxo-2,3-dihydro-1H-indol-3-yl]-4-(4-méthyl-pipérazin-1-yl)-benzamide ;
le N-[5-cyano-1-(2,4-diméthoxy-phénylsulfonyl)-3-(2-éthoxy-pyridin-3-yl)-2-oxo-2,3-dihydro-1H-indol-3-yl]-4-(4-éthyl-pipérazin-1-yl)-benzamide ;
le N-[1-[4-(2,2-difluoro-éthoxy)-phénylsulfonyl]-3-(2-éthoxy-pyridin-3-yl)-5-iodo-2-oxo-2,3-dihydro-1H-indol-3-yl]-4-(4-méthyl-pipérazin-1-yl)-benzamide ;
la 1-(2,4-diméthoxy-phénylsulfonyl)-3-(2-éthoxy-pyridin-3-yl)-5-iodo-3-[4-(4-méthyl-pipérazin-1-yl)-benzylamino]-1,3-dihydro-indol-2-one ;
la 1-(2,4-diméthoxy-phénylsulfonyl)-3-(2-éthoxy-pyridin-3-yl)-5-iodo-3-(4-pipérazin-1-yl-benzylamino)-1,3-dihydro-indol-2-one ;
la 5-chloro-3-(2-éthoxy-pyridin-3-yl)-1-(4-méthoxy-phénylsulfonyl)-3-[4-(4-méthyl-pipérazin-1-yl)-benzylamino]-1,3-dihydro-indol-2-one ;
la 5-chloro-3-(2-éthoxy-pyridin-3-yl)-6-fluoro-1-(4-méthoxy-phénylsulfonyl)-3-[4-(4-méthyl-pipérazin-1-yl)-benzylamino]-1,3-dihydro-indol-2-one ;
la 6-chloro-3-(2-éthoxy-pyridin-3-yl)-5-fluoro-1-(4-méthoxy-phénylsulfonyl)-3-[4-(4-méthyl-pipérazin-1-yl)-benzylamino]-1,3-dihydro-indol-2-one ;
la 5,6-difluoro-3-(2-éthoxy-pyridin-3-yl)-1-(4-méthoxy-phénylsulfonyl)-3-[4-(4-méthyl-pipérazin-1-yl)-benzylamino]-1,3-dihydro-indol-2-one ;
la 3-(2-éthoxy-pyridin-3-yl)-5-fluoro-1-(4-méthoxy-phénylsulfonyl)-3-[4-(4-méthyl-pipérazin-1-yl)-benzylamino]-1,3-dihydro-indol-2-one ;
le 3-(2-éthoxy-pyridin-3-yl)-1-(4-méthoxy-phénylsulfonyl)-3-[4-(4-méthyl-pipérazin-1-yl)-benzylamino]-2-oxo-2,3-dihydro-1H-indol-5-carbonitrile ;
le 1-(2,4-diméthoxy-phénylsulfonyl)-3-(2-éthoxy-pyridin-3-yl)-3-[4-(4-méthyl-pipérazin-1-yl)-benzylamino]-2-oxo-2,3-dihydro-1H-indol-5-carbonitrile ;
le 3-(2-éthoxy-pyridin-3-yl)-1-(4-méthoxy-2-trifluorométhoxy-phénylsulfonyl)-3-[4-(4-méthyl-pipérazin-1-yl)-benzylamino]-2-oxo-2,3-dihydro-1H-indol-5-carbonitrile ;
le 3-(2-éthoxy-pyridin-3-yl)-3-[4-(4-méthyl-pipérazin-1-yl)-benzylamino]-2-oxo-1-(4-trifluorométhoxy-phénylsulfonyl)-2,3-dihydro-1H-indol-5-carbonitrile ;
le 3-(2-éthoxy-pyridin-3-yl)-3-[4-(4-méthyl-pipérazin-1-yl)-benzylamino]-2-oxo-1-[4-(2,2,2-trifluoro-éthoxy)-phénylsulfonyl]-2,3-dihydro-1H-indol-5-carbonitrile ;
le 1-[4-(2,2-difluoro-éthoxy)-phénylsulfonyl]-3-(2-éthoxy-pyridin-3-yl)-3-[4-(4-méthyl-pipérazin-1-yl)-benzylamino]-2-oxo-2,3-dihydro-1H-indol-5-carbonitrile ;
le 3-(2-éthoxy-pyridin-3-yl)-1-[4-(2-fluoro-éthoxy)-phénylsulfonyl]-3-[4-(4-méthyl-pipérazin-1-yl)-benzylamino]-2-oxo-2,3-dihydro-1H-indol-5-carbonitrile ;
le 1-(4-difluorométhoxy-phénylsulfonyl)-3-(2-éthoxy-pyridin-3-yl)-3-[4-(4-méthyl-pipérazin-1-yl)-benzylamino]-2-oxo-2,3-dihydro-1H-indol-5-carbonitrile ;
le 3-(2-éthoxy-pyridin-3-yl)-1-(2-méthoxy-phénylsulfonyl)-3-[4-(4-méthyl-pipérazin-1-yl)-benzylamino]-2-oxo-2,3-dihydro-1H-indol-5-carbonitrile ;
le 3-(2-éthoxy-pyridin-3-yl)-1-(3-méthoxy-phénylsulfonyl)-3-[4-(4-méthyl-pipérazin-1-yl)-benzylamino]-2-oxo-2,3-dihydro-1H-indol-5-carbonitrile ;
le 1-(4-chloro-phénylsulfonyl)-3-(2-éthoxy-pyridin-3-yl)-3-[4-(4-méthyl-pipérazin-1-yl)-benzylamino]-2-oxo-2,3-dihydro-1H-indol-5-carbonitrile ;
le 1-phénylsulfonyl-3-(2-éthoxy-pyridin-3-yl)-3-[4-(4-méthyl-pipérazin-1-yl)-benzylamino]-2-oxo-2,3-dihydro-1H-indol-5-carbonitrile ;
le 1-(4-cyano-phénylsulfonyl)-3-(2-éthoxy-pyridin-3-yl)-3-[4-(4-méthyl-pipérazin-1-yl)-benzylamino]-2-oxo-2,3-dihydro-1H-indol-5-carbonitrile ;
le 1-(2,4-diméthoxy-phénylsulfonyl)-3-(2-éthoxy-pyridin-3-yl)-2-oxo-3-(4-pipérazin-1-yl-benzylamino)-2,3-dihydro-1H-indol-5-carbonitrile ;
le 3-(2-éthoxy-pyridin-3-yl)-1-(4-méthoxy-phénylsulfonyl)-2-oxo-3-(4-pipérazin-1-yl-benzylamino)-2,3-dihydro-1H-indol-5-carbonitrile ;
le 3-(2-éthoxy-pyridin-3-yl)-3-[4-(4-éthyl-pipérazin-1-yl)-benzylamino]-1-(4-méthoxy-phénylsulfonyl)-2-oxo-2,3-dihydro-1H-indol-5-carbonitrile ;
le 1-(2,4-diméthoxy-phénylsulfonyl)-3-(2-éthoxy-pyridin-3-yl)-3-[4-(4-éthyl-pipérazin-1-yl)-benzylamino]-2-oxo-2,3-dihydro-1H-indol-5-carbonitrile ;
le 3-(2-éthoxy-pyridin-3-yl)-6-fluoro-1-(4-méthoxy-phénylsulfonyl)-3-[4-(4-méthyl-pipérazin-1-yl)-benzylamino]-2-oxo-2,3-dihydro-1H-indol-5-carbonitrile ;
le 3-(2-éthoxy-pyridin-3-yl)-6-fluoro-1-(4-méthoxy-phénylsulfonyl)-2-oxo-3-(4-pipérazin-1-yl-benzylamino)-2,3-dihydro-1H-indol-5-carbonitrile ;
le 3-(2-éthoxy-pyridin-3-yl)-3-[4-(4-éthyl-pipérazin-1-yl)-benzylamino]-6-fluoro-1-(4-méthoxy-phénylsulfonyl)-2-oxo-2,3-dihydro-1H-indol-5-carbonitrile ;
le 1-[4-(2,2-difluoro-éthoxy)-phénylsulfonyl]-3-(2-éthoxy-pyridin-3-yl)-6-fluoro-3-[4-(4-méthyl-pipérazin-1-yl)-benzylamino]-2-oxo-2,3-dihydro-1H-indol-5-carbonitrile ;
le 3-(2-éthoxy-phényl)-1-(4-méthoxy-phénylsulfonyl)-3-[4-(4-méthyl-pipérazin-1-yl)-benzylamino]-2-oxo-2,3-dihydro-1H-indol-5-carbonitrile ;
la 1-(2,4-diméthoxy-phénylsulfonyl)-3-(2-éthoxy-pyridin-3-yl)-5-iodo-3-[3-(4-méthyl-pipérazin-1-yl)-benzylamino]-1,3-dihydro-indol-2-one ;
la 3-(2-éthoxy-pyridin-3-yl)-5-iodo-1-(4-méthoxy-2-trifluorométhoxy-phénylsulfonyl)-3-[3-(4-méthyl-pipérazin-1-yl)-benzylamino]-1,3-dihydro-indol-2-one ;
la 3-(2-éthoxy-pyridin-3-yl)-5-iodo-1-(4-méthoxy-phénylsulfonyl)-3-[3-(4-méthyl-pipérazin-1-yl)-benzylamino]-1,3-dihydro-indol-2-one ;
la 3-(2-éthoxy-pyridin-3-yl)-5-iodo-1-(2-méthoxy-phénylsulfonyl)-3-[3-(4-méthyl-pipérazin-1-yl)-benzylamino]-1,3-dihydro-indol-2-one ;
le 1-(2,4-diméthoxy-phénylsulfonyl)-3-(2-éthoxy-pyridin-3-yl)-3-[3-(4-méthyl-pipérazin-1-yl)-benzylamino]-2-oxo-2,3-dihydro-1H-indol-5-carbonitrile ;
le 3-(2-éthoxy-pyridin-3-yl)-1-(4-méthoxy-phénylsulfonyl)-3-[3-(4-méthyl-pipérazin-1-yl)-benzylamino]-2-oxo-2,3-dihydro-1H-indol-5-carbonitrile ;
le 3-(2-éthoxy-pyridin-3-yl)-1-(2-méthoxy-phénylsulfonyl)-3-[3-(4-méthyl-pipérazin-1-yl)-benzylamino]-2-oxo-2,3-dihydro-1H-indol-5-carbonitrile ;
le 1-(2,4-diméthoxy-phénylsulfonyl)-3-(2-éthoxy-pyridin-3-yl)-3-[2-(4-méthyl-pipérazin-1-yl)-benzylamino]-2-oxo-2,3-dihydro-1H-indol-5-carbonitrile ;
le 3-(2-éthoxy-pyridin-3-yl)-1-(4-méthoxy-phénylsulfonyl)-3-[2-(4-méthyl-pipérazin-1-yl)-benzylamino]-2-oxo-2,3-dihydro-1H-indol-5-carbonitrile ;
le 3-(2-éthoxy-pyridin-3-yl)-1-(2-méthoxy-phénylsulfonyl)-3-[2-(4-méthyl-pipérazin-1-yl)-benzylamino]-2-oxo-2,3-dihydro-1H-indol-5-carbonitrile ;
la 1-(2,4-diméthoxy-phénylsulfonyl)-3-(2-éthoxy-pyridin-3-yl)-3-[4-(4-éthyl-pipérazin-1-yl)-phénylamino]-5-iodo-1,3-dihydro-indol-2-one ;
la 1-(2,4-diméthoxy-phénylsulfonyl)-3-(2-éthoxy-pyridin-3-yl)-5-iodo-3-[4-(4-méthyl-pipérazin-1-yl)-phénylamino]-1,3-dihydro-indol-2-one ;
la 3-(2-éthoxy-pyridin-3-yl)-5-iodo-1-(4-méthoxy-phénylsulfonyl)-3-[4-(4-méthyl-pipérazin-1-yl)-phénylamino]-1,3-dihydro-indol-2-one ;
la 5-chloro-3-(2-éthoxy-pyridin-3-yl)-1-(4-méthoxy-phénylsulfonyl)-3-[4-(4-méthyl-pipérazin-1-yl)-phénylamino]-1,3-dihydro-indol-2-one ;
la 5-chloro-3-(2-éthoxy-pyridin-3-yl)-6-fluoro-1-(4-méthoxy-phénylsulfonyl)-3-[4-(4-méthyl-pipérazin-1-yl)-phénylamino]-1,3-dihydro-indol-2-one ;
la 6-chloro-3-(2-éthoxy-pyridin-3-yl)-5-fluoro-1-(4-méthoxy-phénylsulfonyl)-3-[4-(4-méthyl-pipérazin-1-yl)-phénylamino]-1,3-dihydro-indol-2-one ;
la 3-(2-éthoxy-pyridin-3-yl)-5,6-difluoro-1-(4-méthoxy-phénylsulfonyl)-3-[4-(4-méthyl-pipérazin-1-yl)-phénylamino]-1,3-dihydro-indol-2-one ;
le 1-(2,4-diméthoxy-phénylsulfonyl)-3-(2-éthoxy-pyridin-3-yl)-3-[4-(4-méthyl-pipérazin-1-yl)-phénylamino]-2-oxo-2,3-dihydro-1H-indol-5-carbonitrile ;
le 1-(2,4-diméthoxy-phénylsulfonyl)-3-(2-éthoxy-pyridin-3-yl)-3-[4-(4-éthyl-pipérazin-1-yl)-phénylamino]-2-oxo-2,3-dihydro-1H-indol-5-carbonitrile ;
le 3-(2-éthoxy-pyridin-3-yl)-3-[4-(4-éthyl-pipérazin-1-yl)-phénylamino]-1-(4-méthoxy-phénylsulfonyl)-2-oxo-2,3-dihydro-1H-indol-5-carbonitrile ;
le 3-(2-éthoxy-pyridin-3-yl)-3-[4-(4-éthyl-pipérazin-1-yl)-phénylamino]-1-(2-méthoxy-phénylsulfonyl)-2-oxo-2,3-dihydro-1H-indol-5-carbonitrile ;
le 3-(2-éthoxy-pyridin-3-yl)-6-fluoro-1-(4-méthoxy-phénylsulfonyl)-3-[4-(4-méthyl-pipérazin-1-yl)-phénylamino]-2-oxo-2,3-dihydro-1H-indol-5-carbonitrile ;
la 1-(2,4-diméthoxy-phénylsulfonyl)-3-(2-éthoxy-pyridin-3-yl)-5-iodo-3-[3-(4-méthyl-pipérazin-1-yl)-phénylamino]-1,3-dihydro-indol-2-one ;
le 3-(2-éthoxy-pyridin-3-yl)-1-(4-méthoxy-phénylsulfonyl)-3-[3-(4-méthyl-pipérazin-1-yl)-phénylamino]-2-oxo-2,3-dihydro-1H-indol-5-carbonitrile ;
le 1-(2,4-diméthoxy-phénylsulfonyl)-3-(2-éthoxy-pyridin-3-yl)-3-[3-(4-méthyl-pipérazin-1-yl)-phénylamino]-2-oxo-2,3-dihydro-1H-indol-5-carbonitrile ;
la 3-(4-azétidin-3-yl-phénylamino)-1-(2,4-diméthoxy-phénylsulfonyl)-3-(2-éthoxy-pyridin-3-yl)-5-iodo-1,3-dihydro-indol-2-one ;
la 1-(2,4-diméthoxy-phénylsulfonyl)-3-(2-éthoxy-pyridin-3-yl)-3-[4-(1-éthyl-azétidin-3-yl)-phénylamino]-5-iodo-1,3-dihydro-indol-2-one ;
la 3-(4-azétidin-3-yl-phénylamino)-3-(2-éthoxy-pyridin-3-yl)-5-iodo-1-(4-méthoxy-phénylsulfonyl)-1,3-dihydro-indol-2-one ;
la 3-(2-éthoxy-pyridin-3-yl)-3-[4-(1-éthyl-azétidin-3-yl)-phénylamino]-5-iodo-1-(4-méthoxy-phénylsulfonyl)-1,3-dihydro-indol-2-one ;
la 1-(2,4-diméthoxy-phénylsulfonyl)-3-(2-éthoxy-pyridin-3-yl)-5-iodo-3-[6-(4-méthyl-pipérazin-1-yl)-pyridin-3-ylamino]-1,3-dihydro-indol-2-one ;
la 3-(2-éthoxy-pyridin-3-yl)-5-iodo-1-(4-méthoxy-phénylsulfonyl)-3-[6-(4-méthyl-pipérazin-1-yl)-pyridin-3-ylamino]-1,3-dihydro-indol-2-one ;
la 1-phénylsulfonyl-3-(2-éthoxy-pyridin-3-yl)-5-iodo-3-[6-(4-méthyl-pipérazin-1-yl)-pyridin-3-ylamino]-1,3-dihydro-indol-2-one ;
la 3-(2-éthoxy-pyridin-3-yl)-5-iodo-1-(4-méthoxy-phénylsulfonyl)-3-[4-(4-méthyl-pipérazin-1-carbonyl)-phénylamino]-1,3-dihydro-indol-2-one ;
la 1-(2,4-diméthoxy-phénylsulfonyl)-3-(2-éthoxy-pyridin-3-yl)-5-iodo-3-[4-(4-méthyl-pipérazin-1-carbonyl)-phénylamino]-1,3-dihydro-indol-2-one ;
le 3-(2-éthoxy-pyridin-3-yl)-1-(4-méthoxy-phénylsulfonyl)-3-[4-(4-méthyl-pipérazin-1-carbonyl)-phénylamino]-2-oxo-2,3-dihydro-1H-indol-5-carbonitrile ;
le 1-(2,4-diméthoxy-phénylsulfonyl)-3-(2-éthoxy-pyridin-3-yl)-3-[4-(4-méthyl-pipérazin-1-carbonyl)-phénylamino]-2-oxo-2,3-dihydro-1H-indol-5-carbonitrile ;
le 1-(2,4-diméthoxy-phénylsulfonyl)-3-(2-éthoxy-pyridin-3-yl)-3-[4-(4-méthyl-pipérazin-1-ylméthyl)-phénylamino]-2-oxo-2,3-dihydro-1H-indol-5-carbonitrile ;
le 3-(2-éthoxy-pyridin-3-yl)-1-(4-méthoxy-phénylsulfonyl)-3-[4-(4-méthyl-pipérazin-1-ylméthyl)-phénylamino]-2-oxo-2,3-dihydro-1H-indol-5-carbonitrile ;
le 3-(2-(2,2-difluoroéthoxy)-pyridin-3-yl)-1-(4-méthoxy-phénylsulfonyl)-3-[4-(4-méthyl-pipérazin-1-yl)-benzylamino]-2-oxo-2,3-dihydro-1H-indol-5-carbonitrile ;
le 1-(4-méthoxy-phénylsulfonyl)-3-(2-(2,2,2-trifluoroéthoxy)-pyridin-3-yl)-3-[4-(4-méthyl-pipérazin-1-yl)-benzylamino]-2-oxo-2,3-dihydro-1H-indol-5-carbonitrile ;
le 3-(2-(2,2-difluoroéthoxy)-phényl)-1-(4-méthoxy-phénylsulfonyl)-3-[4-(4-méthyl-pipérazin-1-yl)-benzylamino]-2-oxo-2,3-dihydro-1H-indol-5-carbonitrile ;
le 3-[4-(4-éthyl-pipérazin-1-yl)-phénylamino]-1-(4-méthoxy-phénylsulfonyl)-3-(2-(2,2-difluoroéthoxy)-pyridin-3-yl)-2-oxo-2,3-dihydro-1H-indol-5-carbonitrile ;
le 3-[4-(4-éthyl-pipérazin-1-yl)-phénylamino]-1-(4-méthoxy-phénylsulfonyl)-3-(2-(2,2,2-trifluoroéthoxy)-pyridin-3-yl)-2-oxo-2,3-dihydro-1H-indol-5-carbonitrile ;
le 3-(2-(2,2-difluoroéthoxy)-phényl)-3-[4-(4-éthyl-pipérazin-1-yl)-phénylamino]-1-(4-méthoxy-phénylsulfonyl)-2-oxo-2,3-dihydro-1H-indol-5-carbonitrile ;
le 1-(2,4-diméthoxy-phénylsulfonyl)-3-[4-(4-méthyl-pipérazin-1-yl)-phénylamino]-3-(2-(trifluorométhoxy)-phényl)-2-oxo-2,3-dihydro-1H-indol-5-carbonitrile ;
et leurs sels pharmaceutiquement compatibles.

23. Composés de formule XVIII 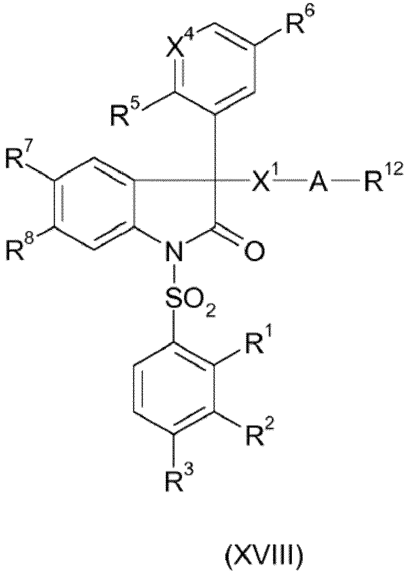 dans laquelle R¹, R², R³, R⁵, R⁶, R⁷, R⁸, X¹, X⁴ et A sont tels que définis dans l'une quelconque des revendications précédentes, et
R¹² représente halogène, CN, OR^{a} ou NR^{b}R^{c}, et de préférence halogène,
R^{a} représentant H, alkyle en C₁-C₄, phényle ou benzyle ; et
R^{b} et R^{c} représentant indépendamment l'un de l'autre H, alkyle en C₁-C₄, alcoxy en C₁-C₄, phényle ou benzyle.

24. Agent pharmaceutique, contenant au moins un composé de formule I selon la définition dans l'une quelconque des revendications 1 à 22 et/ou au moins un sel pharmaceutiquement compatible de celui-ci, et au moins un véhicule pharmaceutiquement compatible.

25. Utilisation de composés de formule I selon la définition dans l'une quelconque des revendications 1 à 22 ou de sels pharmaceutiquement compatibles de ceux-ci pour la fabrication d'un médicament pour le traitement et/ou la prophylaxie de maladies dépendantes de la vasopressine.

26. Utilisation de composés de formule I selon la définition dans l'une quelconque des revendications 1 à 22 ou de sels pharmaceutiquement compatibles de ceux-ci pour la fabrication d'un médicament pour le traitement et/ou la prophylaxie de maladies choisies parmi
le diabète, la résistance à l'insuline, l'énurésie nocturne, l'incontinence, les maladies comprenant des troubles de la coagulation,
l'hypertension, l'hypertension pulmonaire, l'insuffisance cardiaque, l'infarctus du myocarde, le spasme coronarien, l'angine instable, l'ACTP (angioplastie coronarienne transluminale percutanée), les ischémies du coeur, les perturbations du système rénal, les oedèmes, le vasospasme rénal, la nécrose du cortex rénal, l'hyponatrémie, l'hypokaliémie, le syndrome de Schwartz-Bartter, les perturbations des voies gastro-intestinales, le vasospasme gastrique, l'hépatocirrhose, l'ulcère de l'estomac et de l'intestin, les vomissements, les vomissements qui se produisent lors de la chimiothérapie, le mal de la route,
les troubles affectifs,
les troubles de l'anxiété, les troubles de l'anxiété liés au stress,
les troubles de la mémoire, la maladie d'Alzheimer,
les psychoses, les troubles psychotiques,
le syndrome de Cushing, les autres maladies liées au stress,
les troubles du sommeil,
les maladies dépressives, de préférence les troubles de l'humeur apparaissant pendant l'enfance,
les symptômes vasomoteurs, les dysfonctionnements thermorégulateurs,
les dépendances à des drogues, des médicaments et/ou médiées par d'autres facteurs ; le stress causé par le sevrage d'un ou de plusieurs facteurs médiant la dépendance ; et/ou les rechutes induites par le stress dans les dépendances à des drogues, des médicaments et/ou médiées par d'autres facteurs ;
la schizophrénie et la psychose,
et/ou le retard de la miction.
